# EUROPEAN PATENT SPECIFICATION

(11) **EP 1 523 489 B1**
(45) Date of publication and mention of the grant of the patent: **12.03.2014**
(21) Application number: 03761744.6
(22) Date of filing: 27.06.2003
(51) Int. Cl.: C07H 19/06, C07H 19/10, C07H 19/16, C07H 19/20, C07H 19/04, C07H 19/044, C07H 19/048, C07H 19/052, C07H 19/056, C07H 19/12, C07H 19/14, C07H 19/22, C07H 19/23, A61K 31/7056, A61K 31/706, A61K 31/7064, A61P 31/14

(54) **MODIFIED 2' AND 3' -NUCLEOSIDE PRODUGS FOR TREATING FLAVIRIDAE INFECTIONS**
MODIFIZIERTE 2'- UND 3'-NUKLEOSIDPRODRUGS ZUR BEHANDLUNG VON FLAVIVIRIDAE INFEKTIONEN
PROMEDICAMENTS A NUCLEOSIDES 2' ET 3' DESTINES A TRAITER LES INFECTIONS AUX FLAVIVIRUS

(30) Priority: 28.06.2002 US 392350 P; 28.06.2002 US 392351 P; 28.04.2003 US 466194 P; 14.05.2003 US 470949 P
(43) Date of publication of application: 20.04.2005
(60) Divisional application: 10183144.4
(73) Proprietor: IDENIX Pharmaceuticals, Inc., Cambridge, MA 02141 (US); CENTRE NATIONAL DE LA RECHERCHE SCIENTIFIQUE (CNRS), 75794 Paris Cedex 16 (FR); UNIVERSITA DEGLI STUDI DI CAGLIARI, 09042 Monserrato (IT); L'Université Montpellier II, 34095 Montpellier Cedex 5 (FR)
(72) Inventor: SOMMADOSSI, Jean-Pierre, Cambridge, MA 02138 (US); LA COLLA, Poalo, I-09012 Capoterra Cagliari (IT); STORER, Richard, Folkestone Kent CT20 2JE (GB); GOSSELIN, Gilles, 34070 Montpellier (FR)
(74) Representative: Savic, Bojan
(86) International application number: PCT/IB2003/003246
(87) International publication number: WO 2004/002999

(56) References cited:
- WO-A-01/60315
- WO-A-01/90121
- WO-A-01/92282
- WO-A-03/105770
- FR-A- 1 521 076
- JP-A- 06 228 186
- MAHMOUDIAN M ET AL: "A Versatile Procedure for the Generation of Nucleoside 5'-Carboxylic Acids Using Nucleoside Oxidase" TETRAHEDRON, ELSEVIER SCIENCE PUBLISHERS, AMSTERDAM, NL, vol. 54, no. 28, 9 July 1998 (1998-07-09), pages 8171-8182, XP004124009 ISSN: 0040-4020
- S. VELAZQUEZ ET AL.: "Synthesis of [1-[3',5'-bis-O-(tert-butyldimethylsilyl)- beta-D-arabino- and beta-D-ribofuranosyl]cytosine]-2'-spiro-5' '-(4''-amino-1'',2''-oxathiole-2'',2''-dio xide). Analogues of the highly specific anti-HIV-1 agent TSAO-T" TETRAHEDRON, vol. 50, 1994, pages 11013-11022, XP002277147
- A.E.A. HASSAN ET AL.: "Nucleosides and nucleotides. 156." J. ORG. CHEM., vol. 62, 1997, pages 11-17, XP002277148
- A.E.A. HASSAN ET AL.: "Nucleosides and Nucleotides. 151." J. ORG. CHEM., vol. 61, 1996, pages 6261-6267, XP002277149
- U. CHIACCHIO ET AL.: "Stereoselective synthesis of 2'-amino-2',3'-dideoxynucleosides by nitrone 1,3-dipolar cycloaddition: A new efficient entry toward d4T and its 2-methyl analogue" J. ORG. CHEM., vol. 64, 1999, pages 28-36, XP002277150
- S. CZERNECKI ET AL.: "Synthesis of 2'-deoxy-2'-spirocyclopropyl cytidine as potential inhibitor of ribonucleotide diphosphate reductase" CAN. J. CHEM., vol. 71, 1993, pages 413-416, XP002277151
- HOSSAIN N ET AL: "SYNTHESIS OF 2'- AND 3'-SPIRO-ISOXAZOLIDINE DERIVATIVES OF THYMIDINE & THEIR CONVERSIOS TO 2',3'-DIDEOXY-2',3'-DIDEHYDRO-3'-C-SUBSTIT UTED NUCLEOSIDES BY RADICAL PROMOTED FRAGMENTATION" TETRAHEDRON, ELSEVIER SCIENCE PUBLISHERS, AMSTERDAM, NL, vol. 49, no. 44, 1993, pages 10133-10156, XP001041896 ISSN: 0040-4020
- PATENT ABSTRACTS OF JAPAN vol. 018, no. 575 (C-1268), 4 November 1994 (1994-11-04) & JP 06 211890 A (YAMASA SHOYU CO LTD;OTHERS: 01), 2 August 1994 (1994-08-02)
- BIANCO A ET AL: "Synthesis of a New Carbocyclic Nucleoside Analog" TETRAHEDRON LETTERS, ELSEVIER SCIENCE PUBLISHERS, AMSTERDAM, NL, vol. 38, no. 36, 8 September 1997 (1997-09-08), pages 6433-6436, XP004087957 ISSN: 0040-4039

## Description

This invention is in the area of pharmaceutical chemistry, and is in particular, a 2'-branched pyrimidine nucleoside or 2'-branched purine nucleoside as defined in the claims. The invention is also a pharmaceutical composition comprising the nucleoside, and the nucleoside for use in a method for the treatment of a *Flaviviridae* infection, such as a hepatitis C virus infection.

### Flaviviridae Viruses

The Flaviviridae family of viruses comprises at least three distinct genera: *pestiviruses,* which cause disease in cattle and pigs; *flaviviruses,* which are the primary cause of diseases such as dengue fever and yellow fever; and *hepaciviruses,* whose sole member is HCV. The flavivirus genus includes more than 68 members separated into groups on the basis of serological relatedness (Calisher et al., J. Gen. Virol, 1993, 70, 37-43). Clinical symptoms vary and include fever, encephalitis and hemorrhagic fever (Fields Virology, Editors: Fields, B. N., Knipe, D. M., and Howley, P. M., Lippincott-Raven Publishers, Philadelphia, PA, 1996, Chapter 31, 931-959). Flaviviruses of global concern that are associated with human disease include the dengue hemorrhagic fever viruses (DHF), yellow fever virus, shock syndrome and Japanese encephalitis virus (Halstead, S. B., Rev. Infect. Dis., 1984, 6, 251-264; Halstead, S. B., Science, 239:476-481, 1988; Monath, T. P., New Eng. J. Med., 1988, 319, 641-643).

The pestivirus genus includes bovine viral diarrhea virus (BVDV), classical swine fever virus (CSFV, also called hog cholera virus) and border disease virus (BDV) of sheep (Moennig, V. et al. Adv. Vir. Res. 1992, 41, 53-98). Pestivirus infections of domesticated livestock (cattle, pigs and sheep) cause significant economic losses worldwide. BVDV causes mucosal disease in cattle and is of significant economic importance to the livestock industry (Meyers, G. and Thiel, H.-J., Advances in Virus Research, 1996, 47, 53-118; Moennig V., et al, Adv. Vir. Res. 1992, 41, 53-98). Human pestiviruses have not been as extensively characterized as the animal pestiviruses. However, serological surveys indicate considerable pestivirus exposure in humans.

*Pestiviruses* and *hepaciviruses* are closely related virus groups within the Flaviviridae family. Other closely related viruses in this family include the GB virus A, GB virus A-like agents, GB virus-B and GB virus-C (also called hepatitis G virus, HGV). The hepacivirus group (hepatitis C virus; HCV) consists of a number of closely related but genotypically distinguishable viruses that infect humans. There are approximately 6 HCV genotypes and more than 50 subtypes. Due to the similarities between pestiviruses and hepaciviruses, combined with the poor ability of hepaciviruses to grow efficiently in cell culture, bovine viral diarrhea virus (BVDV) is often used as a surrogate to study the HCV virus.

The genetic organization of pestiviruses and hepaciviruses is very similar. These positive stranded RNA viruses possess a single large open reading frame (ORF) encoding all the viral proteins necessary for virus replication. These proteins are expressed as a polyprotein that is co- and post-translationally processed by both cellular and virus-encoded proteinases to yield the mature viral proteins. The viral proteins responsible for the replication of the viral genome RNA are located within approximately the carboxy-terminal. Two-thirds of the ORF are termed nonstructural (NS) proteins. The genetic organization and polyprotein processing of the nonstructural protein portion of the ORF for pestiviruses and hepaciviruses is very similar. For both the pestiviruses and hepaciviruses, the mature nonstructural (NS) proteins, in sequential order from the amino-terminus of the nonstructural protein coding region to the carboxy-terminus of the ORF, consist of p7, NS2, NS3, NS4A, NS4B, NS5A, and NS5B.

The NS proteins of pestiviruses and hepaciviruses share sequence domains that are characteristic of specific protein functions. For example, the NS3 proteins of viruses in both groups possess amino acid sequence motifs characteristic of serine proteinases and of helicases (Gorbalenya et al. (1988) Nature 333:22; Bazan and Fletterick (1989) Virology 171:637-639; Gorbalenya et al. (1989) Nucleic Acid Res. 17.3889-3897). Similarly, the NS5B proteins of pestiviruses and hepaciviruses have the motifs characteristic of RNA-directed RNA polymerases (Koonin, E.V. and Dolja, V.V. (1993) Crit. Rev. Biochem. Molec. Biol. 28:375-430).

The actual roles and functions of the NS proteins of pestiviruses and hepaciviruses in the lifecycle of the viruses are directly analogous. In both cases, the NS3 serine proteinase is responsible for all proteolytic processing of polyprotein precursors downstream of its position in the ORF (Wiskerchen and Collett (1991) Virology 184:341-350; Bartenschlager et al. (1993) J. Virol. 67:3835-3844; Eckart et al. (1993) Biochem. Biophys. Res. Comm. 192:399-406; Grakoui et al. (1993) J. Virol. 67:2832-2843; Grakoui et al. (1993) Proc. Natl. Acad. Sci. USA 90:10583-10587; Hijikata et al. (1993) J. Virol. 67:4665-4675; Tome et al. (1993) J. Virol. 67:4017-4026). The NS4A protein, in both cases, acts as a cofactor with the NS3 serine protease (Bartenschlager et al. (1994) J. Virol. 68:5045-5055; Failla et al. (1994) J. Virol. 68: 3753-3760; Lin et al. (1994) 68:8147-8157; Xu et al. (1997) J. Virol. 71:5312-5322). The NS3 protein of both viruses also functions as a helicase (Kim et al. (1995) Biochem. Biophys. Res. Comm. 215: 160-166; Jin and Peterson (1995) Arch. Biochem. Biophys., 323:47-53; Warrener and Collett (1995) J. Virol. 69:1720-1726). Finally, the NS5B proteins of pestiviruses and hepaciviruses have the predicted RNA-directed RNA polymerases activity (Behrens et al.(1996) EMBO J. 15:12-22; Lchmannet al.(1997) J. Virol. 71:8416-8428; Yuan et al.(1997) Biochem. Biophys. Res. Comm. 232:231-235; Hagedorn, PCT WO 97/12033; Zhong et al.(1998) J. Virol. 72.9365-9369).

### Hepatitis C Virus

The hepatitis C virus (HCV) is the leading cause of chronic liver disease worldwide. (Boyer, N. et al. J. Hepatol. 32:98-112, 2000). HCV causes a slow growing viral infection and is the major cause of cirrhosis and hepatocellular carcinoma (Di Besceglie, A. M. and Bacon, B. R., Scientific American, Oct.: 80-85, (1999); Boyer, N. et al. J. Hepatol. 32:98-112, 2000). An estimated 170 million persons are infected with HCV worldwide. (Boyer, N. et al. J. Hepatol. 32:98-112, 2000). Cirrhosis caused by chronic hepatitis C infection accounts for 8,000-12,000 deaths per year in the United States, and HCV infection is the leading indication for liver transplantation.

HCV is known to cause at least 80% of posttransfusion hepatitis and a substantial proportion of sporadic acute hepatitis. Preliminary evidence also implicates HCV in many cases of "idiopathic" chronic hepatitis, "cryptogenic" cirrhosis, and probably hepatocellular carcinoma unrelated to other hepatitis viruses, such as Hepatitis B Virus (HBV). A small proportion of healthy persons appear to be chronic HCV carriers, varying with geography and other epidemiological factors. The numbers may substantially exceed those for HBV, though information is still preliminary; how many of these persons have subclinical chronic liver disease is unclear. (The Merck Manual, ch. 69, p. 901, 16th ed., (1992)).

HCV is an enveloped virus containing a positive-sense single-stranded RNA genome of approximately 9.4kb. The viral genome consists of a 5' untranslated region (UTR), a long open reading frame encoding a polyprotein precursor of approximately 3011 amino acids, and a short 3' UTR. The 5' UTR is the most highly conserved part of the HCV genome and is important for the initiation and control of polyprotein translation. Translation of the HCV genome is initiated by a cap-independent mechanism known as internal ribosome entry. This mechanism involves the binding of ribosomes to an RNA sequence known as the internal ribosome entry site (IRES). An RNA pseudoknot structure has recently been determined to be an essential structural element of the HCV IRES. Viral structural proteins include a nucleocapsid core protein (C) and two envelope glycoproteins, E1 and E2. HCV also encodes two proteinases, a zinc-dependent metalloproteinase encoded by the NS2-NS3 region and a serine proteinase encoded in the NS3 region. These proteinases are required for cleavage of specific regions of the precursor polyprotein into mature peptides. The carboxyl half of nonstructural protein 5, NS5B, contains the RNA-dependent RNA polymerase. The function of the remaining nonstructural proteins, NS4A and NS4B, and that of NS5A (the amino-terminal half of nonstructural protein 5) remain unknown.

A significant focus of current antiviral research is directed to the development of improved methods of treatment of chronic HCV infections in humans (Di Besceglie, A. M. and Bacon, B. R., Scientific American, Oct.: 80-85, (1999)).

### Treatment of HCV Infection with Interferon

Interferons (IFNs) have been commercially available for the treatment of chronic hepatitis for nearly a decade. IFNs are glycoproteins produced by immune cells in response to viral infection. IFNs inhibit replication of a number of viruses, including HCV, and when used as the sole treatment for hepatitis C infection, IFN can in certain cases suppress serum HCV-RNA to undetectable levels. Additionally, IFN can normalize serum amino transferase levels. Unfortunately, the effect of IFN is temporary and a sustained response occurs in only 8%-9% of patients chronically infected with HCV (Gary L. Davis. Gastroenterolgy 118:S104-S114, 2000). Most patients, however, have difficulty tolerating interferon treatment, which causes severe flu-like symptoms, weight loss, and lack of energy and stamina.

A number of patents disclose Flaviviridae, including HCV, treatments, using interferon-based therapies. For example, U.S. Patent No. 5,980,884 to Blatt et al. discloses methods for retreatment of patients afflicted with HCV using consensus interferon. U.S. Patent No. 5,942,223 to Bazer et al. discloses an anti-HCV therapy using ovine or bovine interferon-tau. U.S. Patent No. 5,928,636 to Alber et al. discloses the combination therapy of interleukin-12 and interferon alpha for the treatment of infectious diseases including HCV. U.S. Patent No. 5,849,696 to Chretien et al. discloses the use of thymosins, alone or in combination with interferon, for treating HCV. U.S. Patent No. 5,830,455 to Valtuena et al. discloses a combination HCV therapy employing interferon and a free radical scavenger. U.S. Patent No. 5,738,845 to Imakawa discloses the use of human interferon tau proteins for treating HCV. Other interferon-based treatments for HCV are disclosed in U.S. Patent No. 5,676,942 to Testa et al.*,* U.S. Patent No. 5,372,808 to Blatt et al.*,* and U.S. Patent No. 5,849,696. A number of patents also disclose pegylated forms of interferon, such as, U.S. Patent Nos. 5,747,646, 5,792,834 and 5,834,594 to Hoffmann-La Roche Inc; PCT Publication No. WO 99/32139 and WO 99/32140 to Enzon; WO 95/13090 and US Patent Nos. 5,738,846 and 5,711,944 to Schering; and U.S. Patent No. 5,908,621 to Glue et al.*.*

Interferon alpha-2a and interferon alpha-2b are currently approved as monotherapy for the treatment of HCV. ROFERON®-A (Roche) is the recombinant form of interferon alpha-2a. PEGASYS® (Roche) is the pegylated (i.e. polyethylene glycol modified) form of interferon alpha-2a. INTRON®A (Schering Corporation) is the recombinant form of Interferon alpha-2b, and PEG-INTRON® (Schering Corporation) is the pegylated form of interferon alpha-2b.

Other forms of interferon alpha, as well as interferon beta, gamma, tau and omega are currently in clinical development for the treatment of HCV. For example, INFERGEN (interferon alphacon-1) by InterMune, OMNIFERON (natural interferon) by Viragen, ALBUFERON by Human Genome Sciences, REBIF (interferon beta-1a) by Ares-Serono, Omega Interferon by BioMedicine, Oral Interferon Alpha by Amarillo Biosciences, and interferon gamma, interferon tau, and interferon gamma- 1b by InterMune are in development.

### Ribivarin

Ribavirin (1-β-D-ribofuranosyl-1-1,2,4-triazole-3-carboxamide) is a synthetic, non-interferon-inducing, broad spectrum antiviral nucleoside analog sold under the trade name, Virazole (The Merck Index, 11th edition, Editor: Budavari, S., Merck & Co., Inc., Rahway, NJ, p1304, 1989). United States Patent No. 3,798,209 and RE29,835 disclose and claim ribavirin. Ribavirin is structurally similar to guanosine, and has in vitro activity against several DNA and RNA viruses including *Flaviviridae* (Gary L. Davis. Gastroenterology 118:S104-S114,2000).

Ribavirin reduces serum amino transferase levels to normal in 40% of patients, but it does not lower serum levels of HCV-RNA (Gary L. Davis. Gastroenterology 118:S104-S114, 2000). Thus, ribavirin alone is not effective in reducing viral RNA levels. Additionally, ribavirin has significant toxicity and is known to induce anemia.

Ribavirin is not approved fro monotherapy against HCV. It has been approved in combination with interferon alpha-2a or interferon alpha-2b for the treatment of HCV.

### Combination of Interferon and Ribavirin

The current standard of care for chronic hepatitis C is combination therapy with an alpha interferon and ribavirin. The combination of interferon and ribavirin for the treatment of HCV infection has been reported to be effective in the treatment of interferon naïve patients (Battaglia, A.M. et al., Ann. Pharmacother. 34:487-494, 2000), as well as for treatment of patients when histological disease is present (Berenguer, M. et al. Antivir. Ther. 3(Suppl. 3):125-136, 1998). Studies have show that more patients with hepatitis C respond to pegylated interferon-alpha/ribavirin combination therapy than to combination therapy with unpegylated interferon alpha. However, as with monotherapy, significant side effects develop during combination therapy, including hemolysis, flu-like symptoms, anemia, and fatigue. (Gary L. Davis. Gastroenterology 118:S104-S114, 2000).

Combination therapy with PEG-INTRON® (peginterferon alpha-2b) and REBETOL® (Ribavirin, USP) Capsules is available from Schering Corporation. REBETOL® (Schering Corporation) has also been approved in combination with INTRON® A (Interferon alpha-2b, recombinant, Schering Corporation). Roche's PEGASYS® (pegylated interferon alpha-2a) and COPEGUS® (ribavirin) are also approved for the treatment of HCV.

PCT Publication Nos. WO 99/59621, WO 00/37110, WO 01/81359, WO 02/32414 and WO 03/024461 by Schering Corporation disclose the use of pegylated interferon alpha and ribavirin combination therapy for the treatment of HCV. PCT Publication Nos. WO 99/15194, WO 99/64016, and WO 00/24355 by Hoffmann-La Roche Inc also disclose the use of pegylated interferon alpha and ribavirin combination therapy for the treatment of HCV.

### Additional Methods to Treat Flaviviridae Infections

The development of new antiviral agents for flaviviridae infections, especially hepatitis C, is currently underway. Specific inhibitors of HCV-derived enzymes such as protease, helicase, and polymerase inhibitors are being developed. Drugs that inhibit other steps in HCV replication are also in development, for example, drugs that block production of HCV antigens from the RNA (IRES inhibitors), drugs that prevent the normal processing of HCV proteins (inhibitors of glycosylation), drugs that block entry of HCV into cells (by blocking its receptor) and nonspecific cytoprotective agents that block cell injury caused by the virus infection. Further, molecular approaches are also being developed to treat hepatitis C, for example, ribozymes, which are enzymes that break down specific viral RNA molecules, and antisense oligonucleotides, which are small complementary segments of DNA that bind to viral RNA and inhibit viral replication, are under investigation. A number of HCV treatments are reviewed by Bymock et al. in Antiviral Chemistry & Chemotherapy, 11:2; 79-95 (2000) and De Francesco et al. in Antiviral Research, 58: 1-16 (2003).

Examples of classes of drugs that are being developed to treat Flaviviridae infections include:
(1) Protease inhibitors
   Substrate-based NS3, protease inhibitors (Attwood *et al., Antiviral peptide derivatives,* PCT WO 98/22496, 1998**;** Attwood et al., Antiviral Chemistry and Chemotherapy 1999, 10, 259-273; Attwood *et al., Preparation and use of amino acid derivatives as anti-viral agents,* German Patent Pub. DE 19914474; Tung *et al. Inhibitors of serine proteases, particularly hepatitis C virus NS3 protease,* PCT WO 98/17679), including alphaketoamides and hydrazinoureas, and inhibitors that terminate in an electrophile such as a boronic acid or phosphonate (Llinas-Brunet et al, *Hepatitis C inhibitor peptide analogues,* PCT WO 99/07734) are being investigated.
   Non-substrate-based NS3 protease inhibitors such as 2,4,6-trihydroxy-3-nitro-benzamide derivatives (Sudo K. et al., Biochemical and Biophysical Research Communications, 1997, 238, 643-647; Sudo K. et al. Antiviral Chemistry and Chemotherapy, 1998, 9, 186), including RD3-4082 and RD3-4078, the former substituted on the amide with a 14 carbon chain and the latter processing a para-phenoxyphenyl group are also being investigated.
   Sch 68631, a phenanthrenequinone, is an HCV protease inhibitor (Chu M. et al., Tetrahedron Letters 37:7229-7232, 1996). In another example by the same authors, Sch 351633, isolated from the fungus *Penicillium griseofulvum,* was identified as a protease inhibitor (Chu M. et al., Bioorganic and Medicinal Chemistry Letters 9:1949-1952). Nanomolar potency against the HCV NS3 protease enzyme has been achieved by the design of selective inhibitors based on the macromolecule eglin c. Eglin c, isolated from leech, is a potent inhibitor of several serine proteases such as S. griseus proteases A and B, α-chymotrypsin, chymase and subtilisin. Qasim M.A. et al., Biochemistry 36:1598-1607, 1997.
   Several U.S. patents disclose protease inhibitors for the treatment of HCV. For example, U.S. Patent No. 6,004,933 to Spruce et al. discloses a class of cysteine protease inhibitors for inhibiting HCV endopeptidase 2. U.S. Patent No. 5,990,276 to Zhang et al. discloses synthetic inhibitors of hepatitis C virus NS3 protease. The inhibitor is a subsequence of a substrate of the NS3 protease or a substrate of the NS4A cofactor. The use of restriction enzymes to treat HCV is disclosed in U.S. Patent No. 5,538,865 to Reyes et al. Peptides as NS3 serine protease inhibitors of HCV are disclosed in WO 02/008251 to Corvas International, Inc, and WO 02/08187 and WO 02/008256 to Schering Corporation. HCV inhibitor tripeptides are disclosed in US Patent Nos. 6,534,523, 6,410,531, and 6,420,380 to Boehringer Ingelheim and WO 02/060926 to Bristol Myers Squibb. Diaryl peptides as NS3 serine protease inhibitors of HCV are disclosed in WO 02/48172 to Schering Corporation. Imidazoleidinones as NS3 serine protease inhibitors of HCV are disclosed in WO 02/08198 to Schering Corporation and WO 02/48157 to Bristol Myers Squibb. WO 98/17679 to Vertex Pharmaceuticals and WO 02/48116 to Bristol Myers Squibb also disclose HCV protease inhibitors.
(2) Thiazolidine derivatives which show relevant inhibition in a reverse-phase HPLC assay with an NS3/4A fusion protein and NS5A/5B substrate (Sudo K. et al., Antiviral Research, 1996, 32, 9-18), especially compound RD-1-6250, possessing a fused cinnamoyl moiety substituted with a long alkyl chain, RD4 6205 and RD4 6193;
(3) Thiazolidines and benzanilides identified in Kakiuchi N. et al. J. EBS Letters 421, 217-220; Takeshita N. et al. Analytical Biochemistry, 1997, 247, 242-246;
(4) A phenan-threnequinone possessing activity against protease in a SDS-PAGE and autoradiography assay isolated from the fermentation culture broth of *Streptomyces* sp., Sch 68631 (Chu M. et al., Tetrahedron Letters, 1996, 37, 7229-7232), and Sch 351633, isolated from the fungus *Penicillium griseofulvum,* which demonstrates activity in a scintillation proximity assay (Chu M. et al., Bioorganic and Medicinal Chemistry Letters 9, 1949-1952);
(5) Helicase inhibitors (for example Diana *G.D. et al., Compounds, compositions and methods for treatment of hepatitis C*, U.S. Pat. No. 5,633,358; Diana G.D. *et al., Piperidine derivatives, pharmaceutical compositions thereof and their use in the treatment of hepatitis C,* PCT WO 97/36554);
(6) Nucleotide polymerase inhibitors and gliotoxin (Ferrari R. et al. Journal of Virology, 1999, 73, 1649-1654), and the natural product cerulenin (Lohmann V. et al., Virology, 1998, 249, 108-118);
(7) Antisense phosphorothioate oligodeoxynucleotides (S-ODN) complementary to sequence stretches in the 5' non-coding region (NCR) of the virus (Alt M. et al., Hepatology, 1995, 22, 707-717), or nucleotides 326-348 comprising the 3' end of the NCR and nucleotides 371-388 located in the core coding region of the HCV RNA (Alt M. et al., Archives of Virology, 1997, 142, 589-599; Galderisi U. et al., Journal of Cellular Physiology, 1999, 181, 251-257);
(8) Inhibitors of IRES-dependent translation (Ikeda N *et al., Agent for the prevention and treatment of hepatitis C,* Japanese Patent Pub. JP-08268890; Kai Y. *et al. Prevention and treatment of viral diseases,* Japanese Patent Pub. JP-10101591);
(9) Ribozymes, such as nuclease-resistant ribozymes (Maccjak, D. J. et al., Hepatology 1999, 30, abstract 995) and those disclosed in U.S. Patent No. 6,043,077 to Barber et al.*,* and U.S. Patent Nos. 5,869,253 and 5,610,054 to Draper et al.*;* and
(10) Nucleoside analogs have also been developed for the treatment of Flaviviridae infections.
   Idenix Pharmaceuticals discloses the use of branched nucleosides in the treatment of flaviviruses (including HCV) and pestiviruses in International Publication Nos. WO 01/90121 and WO 01/92282. Specifically, a method for the treatment of hepatitis C infection (and flaviviruses and pestiviruses) in humans and other host animals is disclosed in the Idenix publications that includes administering an effective amount of a biologically active 1', 2', 3' or 4'-branched β-D or β-L nucleosides or a pharmaceutically acceptable salt or derivative thereof, administered either alone or in combination with another antiviral agent, optionally in a pharmaceutically acceptable carrier.
   Other patent applications disclosing the use of certain nucleoside analogs to treat hepatitis C virus include: PCT/CA00/01316 (WO 01/32153; filed November 3, 2000) and PCT/CA01/00197 (WO 01/60315; filed February 19, 2001) filed by BioChem Pharma, Inc. (now Shire Biochem, Inc.); PCT/US02/01531 (WO 02/057425; filed January 18, 2002) and PCT/US02/03086 (WO 02/057287; filed January 18, 2002) filed by Merck & Co., Inc., PCT/EP01/09633 (WO 02/18404; published August 21, 2001) filed by Roche, and PCT Publication Nos. WO 01/79246 (filed April 13, 2001), WO 02/32920 (filed October 18, 2001) and WO 02/48165 by Pharmasset, Ltd.
   PCT Publication No. WO 99/43691 to Emory University, entitled "2'-Fluoronucleosides" discloses the use of certain 2'-fluoronucleosides to treat HCV.
   Eldrup et al. (Oral Session V, Hepatitis C Virus, Flaviviridae; 16th International Conference on Antiviral Research (April 27, 2003, Savannah, Ga.)) described the structure activity relationship of 2'-modified nucleosides for inhibition of HCV.
   Bhat et al. (Oral Session V, Hepatitis C Virus, Flaviviridae, 2003 (Oral Session V, Hepatitis C Virus, Flaviviridae; 16th International Conference on Antiviral Research (April 27, 2003, Savannah, Ga.); p A75) describe the synthesis and pharmacokinetic properties of nucleoside analogues as possible inhibitors of HCV RNA replication. The authors report that 2'-modified nucleosides demonstrate potent inhibitory activity in cell-based replicon assays.
   Olsen et al. (Oral Session V, Hepatitis C Virus, Flaviviridae; 16th International Conference on Antiviral Research (April 27, 2003, Savannah, Ga.) p A76) also described the effects of the 2'-modified nucleosides on HCV RNA replication.
(11) Other miscellaneous compounds including 1-amino-alkylcyclohexanes (U.S. Patent No. 6,034,134 to Gold et al.), alkyl lipids (U.S. Pat. No. 5,922,757 to Chojkier et al.), vitamin E and other antioxidants (U.S. Pat. No. 5,922,757 to Chojkier et al.), squalene, amantadine, bile acids (U.S. Pat. No. 5,846,964 to Ozeki et al.), N-(phosphonoacetyl)-L-aspartic acid, (U.S. Pat. No. 5,830,905 to Diana et al.), benzenedicarboxamides (U.S. Pat. No. 5,633,388 to Diana et al.), polyadenylic acid derivatives (U.S. Pat. No. 5,496,546 to Wang *et al*.), 2',3'-dideoxyinosine (U.S. Pat. No. 5,026,687 to Yarchoan et al.), benzimidazoles (U.S. Pat. No. 5,891,874 to Colacino et al.), plant extracts (U.S. Patent No. 5,837,257 to Tsai et al.*,* U.S. Patent No. 5,725,859 to Omer et al.*,* and U.S. Patent No. 6,056,961), and piperidenes (U.S. Patent No. 5,830,905 to Diana et al.*).*
(12) Other compounds currently in preclinical or clinical development for treatment of hepatitis C virus include: Interleukin-10 by Schering-Plough, IP-501 by Interneuron, Merimebodib (VX-497) by Vertex, AMANTADINE® (Symmetrel) by Endo Labs Solvay, HEPTAZYME® by RPI, IDN-6556 by Idun Pharma., XTL-002 by XTL., HCV/MF59 by Chiron, CIVACIR® (Hepatitis C Immune Globulin) by NABI, LEVOVIRIN® by ICN/Ribapharm, VIRAMIDINE® by ICN/Ribapharm, ZADAXIN® (thymosin alpha-1) by Sci Clone, thymosin plus pegylated interferon by Sci Clone, CEPLENE® (histamine dihydrochloride) by Maxim, VX 950 / LY 570310 by Vertex/Eli Lilly, ISIS 14803 by Isis Pharmaceutical/Elan, IDN-6556 by Idun Pharmaceuticals, Inc., JTK 003 by AKROS Pharma, BILN-2061 by Boehringer Ingelheim, CellCept (mycophenolate mofetil) by Roche, T67, a β-tubulin inhibitor, by Tularik, a therapeutic vaccine directed to E2 by Innogenetics, FK788 by Fujisawa Healthcare, Inc., IdB 1016 (Siliphos, oral silybin-phosphatdylcholine phytosome), RNA replication inhibitors (VP50406) by ViroPharma/Wyeth, therapeutic vaccine by Intercell, therapeutic vaccine by Epimmune/Genencor, IRES inhibitor by Anadys, ANA 245 and ANA 246 by Anadys, immunotherapy (Therapore) by Avant, protease inhibitor by Corvas/SChering, helicase inhibitor by Vertex, fusion inhibitor by Trimeris, T cell therapy by CellExSys, polymerase inhibitor by Biocryst, targeted RNA chemistry by PTC Therapeutics, Dication by Immtech, Int., protease inhibitor by Agouron, protease inhibitor by Chiron/Medivir, antisense therapy by AVI BioPharma, antisense therapy by Hybridon, hemopurifier by Aethlon Medical, therapeutic vaccine by Merix, protease inhibitor by Bristol-Myers Squibb/Axys, Chron-VacC, a therapeutic vaccine, by Tripep, UT 231B by United Therapeutics, protease, helicase and polymerase inhibitors by Genelabs Technologies, IRES inhibitors by Immusol, R803 by Rigel Pharmaceuticals, INFERGEN® (interferon alphacon-1) by InterMune, OMNIFERON® (natural interferon) by Viragen, ALBUFERON® by Human Genome Sciences, REBIF® (interferon beta-1a) by Ares-Serono, Omega Interferon by BioMedicine, Oral Interferon Alpha by Amarillo Biosciences, interferon gamma, interferon tau, and Interferon gamma- 1b by InterMune.

Nucleoside prodrugs have been previously described for the treatment of other forms of hepatitis. WO 00/09531 (filed August 10, 1999) and WO 01/96353 (filed June 15, 2001) to Idenix Pharmaceuticals discloses 2'-deoxy-β-L-nucleosides and their 3'-prodrugs for the treatment of HBV. U.S. Patent No. 4,957,924 to Beauchamp discloses various therapeutic esters of acyclovir.

In light of the fact that HCV infection has reached epidemic levels worldwide, and has tragic effects on the infected patient, there remains a strong need to provide new effective pharmaceutical agents to treat hepatitis C that have low toxicity to the host.

Further, given the rising threat of other flaviviridae infections, there remains a strong need to provide new effective pharmaceutical agents that have low toxicity to the host.

Therefore, it is an object of the present invention to provide a compound, a composition and their use in a method for the treatment of a host infected with hepatitis C virus.

It is another object of the present invention to provide a compound, a composition and their use in a method for the treatment of patients infected with *pestiviruses, flaviviruses,* or *hepaciviruses.*

This invention relates to 2'-branched nucleosides, compositions thereof, and the nucleosides for use in methods as defined in the claims.

3'-prodrugs of 2'-branched β-D or β-L nucleosides, or their pharmaceutically acceptable salts or pharmaceutically acceptable formulations containing these compounds are useful in the prevention and treatment of *Flaviviridae* infections and other related conditions such as anti- *Flaviviridae* antibody positive and *Flaviviridae-*positive conditions, chronic liver inflammation caused by HCV, cirrhosis, acute hepatitis, fulminant hepatitis, chronic persistent hepatitis, and fatigue. These compounds or formulations can also be used prophylactically to prevent or retard the progression of clinical illness in individuals who are anti*-Flaviviridae* antibody or *Flaviviridae*-antigen positive or who have been exposed to a *Flaviviridae.*

A method for the treatment of a *Flaviviridae* viral infection in a host, including a human, is also disclosed that includes administering an effective amount of a 3'-prodrug of a biologically active 2'-branched β-D or β-L nucleoside or a pharmaceutically acceptable salt thereof, administered either alone or in combination or alternation with another *anti-Flaviviridae* agent, optionally in a pharmaceutically acceptable carrier. The term 3'-prodrug, as used herein, refers to a 1', 2', 3' or 4'-branched β-D or β-L nucleoside that has a biologically cleavable moiety at the 3'-position, including, but not limited to acyl, and in one embodiment, a natural or synthetic D or L amino acid, preferably an L-amino acid.

Pharmaceutically acceptable salts include tosylate, methanesulfonate, acetate, citrate, malonate, tartarate, succinate, benzoate, ascorbate, α-ketoglutarate, and α-glycerophosphate, formate, fumarate, propionate, glycolate, lactate, pyruvate, oxalate, maleate, salicyate, sulfate, sulfonate, nitrate, bicarbonate, hydrobromate, hydrobromide, hydroiodide, carbonate, and phosphoric acid salts. A particularly preferred embodiment is the mono or dihydrochloride salt.

The 2'-branched β-D or β-L nucleoside can have biologically cleavable moieties at the 5' positions. Preferred moieties are natural or synthetic D or L amino acid esters, including D or L-valyl, though preferably L-amino acid esters, such as L-valyl, and alkyl esters including acetyl.

The 2'-branched β-D or β-L nucleoside can have biologically cleavable moieties at the 3' and/or 5' positions. Preferred moieties are natural or synthetic D or L amino acid esters, such as valyl, though preferably L-amino acids, such as L-valyl, and alkyl esters including acetyl. Therefore, this invention specifically includes 3'-L-amino acid ester and 3',5'-L-diamino acid ester of 2'-branched β-D or β-L nucleosides with any desired purine or pyrimidine base, wherein the parent drug optionally has an EC₅₀ of less than 15 micromolar, and even more preferably less than 10 micromolar; 3'-(alkyl or aryl) ester or 3',5'-L-di(alkyl or aryl) ester of 2' -branched β-D or β-L nucleosides with any desired purine or pyrimidine base, wherein the parent drug optionally has an EC₅₀ of less than 10 or 15 micromolar; and prodrugs of 3',5'-diesters of 2'-branched β-D or β-L nucleosides wherein (i) the 3' ester is a natural or synthetic D or L amino acid ester and the 5'-ester is an alkyl or aryl ester; (ii) both esters are natural or synthetic D or L-amino acid esters; (iii) both esters are independently alkyl or aryl esters; and (iv) the 3' ester is independently an alkyl or aryl ester and the 5'-ester is a natural or synthetic D or L-amino acid ester, wherein the parent drug optionally has an EC₅₀ of less than 10 or 15 micromolar.

Examples of prodrugs are 3'-L-valine ester of β-D-2',6-dimethyl-cytidine; 3'-L-valine ester of β-D-2',6-dimethyl-thymidine; 3'-L-valine ester of β-D-2',8-dimethyl-adenosine; 3'-L-valine ester of β-D-2',8-dimethyl-guanosine; 3'-L-valine ester of β-D-2',6-dimethyl-5-fluorocytidine; 3'-L-valine ester of β-D-2',6-dimethyl-uridine; 3'-acetyl ester of β-D-2',6-dimethyl-cytidine; 3'-acetyl ester of β-D-2',6-dimethyl-thymidine; 3'-acetyl ester of β-D-2',8-dimethyl-adenosine; 3'-acetyl ester of β-D-2',8-dimethyl-guanosine; 3'-acetyl ester of β-D-2',6-dimethyl-5-fluoro-cytidine; and 3'-esters of β-D-2',6-dimethyl-(cytidine, 5-fluorocytidine, uridine or thymidine) or 3'-esters of β-D-2',8-dimethyl-(guanosine, adenosine or inosine) wherein (i) the 3' ester is an amino acid ester; or (ii) the 3' ester is an alkyl or aryl ester.

Additional examples of prodrugs are 3',5'-L-divaline ester of β-D-2',6-dimethyl-cytidine (dival-2',6-diMe-L-dC); 3',5'-L-divaline ester of β-D-2',6-dimethyl-thymidine; 3',5'-L-divaline ester of β-D-2',8-dimethyl-adenosine; 3',5'-L-divaline ester of β-D-2',8-dimethyl-guanosine; 3',5'-L-divaline ester of β-D-2',6-dimethyl-5-fluoro-cytidine; 3',5'-L-divaline ester of β-D-2',6-dimethyl-uridine; 3',5'-diacetyl ester of β-D-2',6-dimethyl-cytidine; 3',5'-diacetyl ester of β-D-2',6-dimethyl-thymidine; 3',5'-diacetyl ester of β-D-2',8-dimethyl-adenosine; 3',5'-diacetyl ester of β-D-2',8-dimethyl-guanosine; 3',5'-diacetyl ester of β-D-2',6-dimethyl-5-fluoro-cytidine; and 3',5'-diesters of β-D-2',6-dimethyl-(cytidine, 5-fluorocytidine, uridine or thymidine) or 3',5'-diesters of β-D-2',8-dimethyl-(guanosine, adenosine or inosine) wherein (i) the 3' ester is an amino acid ester and the 5'-ester is an alkyl or aryl ester; (ii) both esters are amino acid esters; (iii) both esters are independently alkyl or aryl esters; or (iv) the 3' ester is an alkyl or aryl ester and the 5'-ester is an amino acid ester.

Amino acid ester and the 3'-ester is an alkyl or aryl ester; (ii) both esters are amino acid esters; (iii) both esters are independently alkyl or aryl esters; and (iv) the 2' ester is independently an alkyl or aryl ester and the 3'-ester is an amino acid ester, wherein the parent drug optionally has an EC₅₀ of less than 10 or 15 micromolar. Further, 2',3',5'-triesters of 1', 2', 3' or 4'-branched β-D or β-L nucleosides wherein (i) all three esters are amino acid esters; (ii) all three esters are independently alkyl or aryl esters; (iii) the 2' ester is an amino acid ester, the 3' ester is an amino acid ester and the 5'-ester is an alkyl or aryl ester; (iv) the 2' ester is an amino acid ester, the 3' ester is an alkyl or aryl ester and the 5'-ester is an alkyl or aryl ester; (v) the 2' ester is an alkyl or aryl ester, the 3' ester is an alkyl or aryl ester and the 5'-ester is an amino acid ester; (vi) the 2' ester is an alkyl or aryl ester, the 3' ester is an amino acid ester and the 5'-ester is an amino acid ester; (vii) the 2' ester is an alkyl or aryl ester, the 3' ester is an amino acid ester and the 5'-ester is an alkyl or aryl ester; and (viii) the 2' ester is an amino acid ester, the 3' ester is an alkyl or aryl ester and the 5'-ester is an amino acid ester; wherein the parent drug optionally has an EC₅₀ of less than 10 or 15 micromolar.

Examples of prodrugs falling within the disclosure include 2',3'-L-divaline ester of β-D-2',6-dimethyl-cytidine (dival-2',6-diMe-L-dC); 2',3'-L-divaline ester of β-D-2',6-dimethyl-thymidine; 2',3'-L-divaline ester of β-D-2',8-dimethyl-adenosine; 2',3'-L-divaline ester of β-D-2',8-dimethyl-guanosine; 2',3'-L-divaline ester of β-D-2',6-dimethyl-5-fluorocytidine; 2',3'-L-divaline ester of β-D-2',6-dimethyl-uridine; 2',3'-diacetyl ester of β-D-2',6-dimethyl-cytidine; 2',3'-diacetyl ester of β-D-2',6-dimethyl-thymidine; 2',3'-diacetyl ester of β-D-2',8-dimethyl-adenosine; 2',3'-diacetyl ester of β-D-2',8-dimethyl-guanosine; 2',3'-diacetyl ester of β-D-2',6-dimethyl-5-fluoro-cytidine; and 2',3'-diesters of β-D-2',6-dimethyl-(cytidine, 5-fluorocytidine, uridine or thymidine) or 2',3'-diesters of β-D-2',8-dimethyl-(guanosine, adenosine or inosine) wherein (i) the 2' ester is an amino acid ester and the 3'-ester is an alkyl or aryl ester; (ii) both esters are amino acid esters; (iii) both esters are independently alkyl or aryl esters; or (iv) the 2' ester is an alkyl or aryl ester and the 3'-ester is an amino acid ester.

Additional examples of prodrugs falling within the disclosure include 2',3',5'-L-trivaline ester of β-D-2',6-dimethyl-cytidine (trival-2',6-diMe-L-dC); 2',3',5'-L-trivaline ester of β-D-2',6-dimethyl-thymidine; 2',3',5'-L-trivaline ester of β-D-2',8-dimethyl-adenosine; 2',3',5'-L-trivaline ester of β-D-2',8-dimethyl-guanosine; 2',3',5'-L-trivaline ester of β-D-2',6-dimethyl-5-fluoro-cytidine; 2',3',5'-L-trivaline ester of β-D-2',6-dimethyl-uridine; 2',3',5'-triacetyl ester of β-D-2',6-dimethyl-cytidine; 2',3',5'-triacetyl ester of β-D-2',6-dimethyl-thymidine; 2',3',5'-triacetyl ester of β-D-2',8-dimethyl-adenosine; 2',3',5'-triacetyl ester of β-D-2',8-dimethyl-guanosine; 2',3',5'-triacetyl ester of β-D-2',6-dimethyl-5-fluoro-cytidine; and 2',3',5'-triesters of β-D-2',6-dimethyl-(cytidine, 5-fluorocytidine, uridine or thymidine) and 2',3',5'-triesters of β-D-2',8-dimethyl-(guanosine, adenosine or inosine) wherein (i) all three esters are amino acid esters; (ii) all three esters are independently alkyl or aryl esters; (iii) the 2' ester is an amino acid ester, the 3' ester is an amino acid ester and the 5'-ester is an alkyl or aryl ester; (iv) the 2' ester is an amino acid ester, the 3' ester is an alkyl or aryl ester and the 5'-ester is an alkyl or aryl ester; (v) the 2' ester is an alkyl or aryl ester, the 3' ester is an alkyl or aryl ester and the 5'-ester is an amino acid ester; (vi) the 2' ester is an alkyl or aryl ester, the 3' ester is an amino acid ester and the 5'-ester is an amino acid ester; (vii) the 2' ester is an alkyl or aryl ester, the 3' ester is an amino acid ester and the 5'-ester is an alkyl or aryl ester; and (viii) the 2' ester is an amino acid ester, the 3' ester is an alkyl or aryl ester and the 5'-ester is an amino acid ester.

In a principal embodiment, a compound of Formula (IX), or a pharmaceutically acceptable salt, or a tautomeric or polymorphic form thereof, is provided, as well as the compound for use in a method for the treatment of a host infected with a *Flaviviridae* comprising administering an effective treatment amount of the compound of Formula (IX): 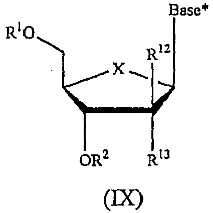 or a pharmaceutically acceptable salt, or a tautomeric or polymorphic form thereof, wherein:
R¹ and R² are independently H; phosphate; straight chained, branched or cyclic alkyl; acyl; CO-alkyl; CO-aryl; CO-alkoxyalkyl; CO-aryloxyalkyl; CO-substituted aryl; sulfonate ester; benzyl, wherein the phenyl group is optionally substituted with one or more substituents; alkylsulfonyl; arylsulfonyl; aralkylsulfonyl; a lipid; an amino acid; a carbohydrate; a peptide; or a cholesterol;
X is O;
Base* is a purine or pyrimidine base;
R¹² is C(Y³)₃;
Y³ is H; and
R¹³ is fluoro.

In another preferred embodiment, R² is an amino acid residue, and is preferably L-valinyl.

The β-D- and β-L-nucleosides of this invention may inhibit *Flaviviridae* polymerase activity. Nucleosides can be screened for their ability to inhibit *Flaviviridae* polymerase activity *in vitro* according to screening methods set forth more particularly herein. One can readily determine the spectrum of activity by evaluating the compound in the assays described herein or with another confirmatory assay.

In one embodiment the efficacy of the *anti-Flaviviridae* compound is measured according to the concentration of compound necessary to reduce the plaque number of the virus *in vitro,* according to methods set forth more particularly herein, by 50% (i.e. the compound's EC₅₀). In preferred embodiments the parent of the prodrug compound exhibits an EC₅₀ of less than 25, 15, 10, 5, or 1 micromolar. In preferred embodiments the compound exhibits an EC₅₀ of less than 15 or 10 micromolar, when measured according to the polymerase assay described in Ferrari et al., Jnl. of Vir., 73:1649-1654, 1999; Ishii et al., Hepatology, 29:1227-1235,1999; Lohmann et al., Jnl, of Bio. Chem., 274:10807-10815, 1999; or Yamashita et al, Jnl. of Bio. Chem., 273:15479-15486, 1998.

In another embodiment, combination and/or alternation therapy are provided. In combination therapy, an effective dosage of two or more agents are administered together, whereas during alternation therapy an effective dosage of each agent is administered serially. The dosages will depend on absorption, inactivation, and excretion rates of the drug as well as other factors known to those of skill in the art. It is to be noted that dosage values will also vary with the severity of the condition to be alleviated. It is to be further understood that for any particular subject, specific dosage regimens and schedules should be adjusted over time according to the individual need and the professional judgment of the person administering or supervising the administration of the compositions.

The invention provides combinations of at least two of the herein described prodrugs. The invention further provides at least one of the described 3'-prodrugs in combination or alternation with a second nucleoside that exhibits activity against a *Flaviviridae,* including but not limited to a parent drug of any of the prodrugs defined herein, i.e. β-D-2',6-dimethyl-cytidine, β-D-2',6-dimethyl-thymidine, β-D-2',8-dimethyl-adenosine, β-D-2',8-dimethyl-guanosine, β-D-2',6-dimethyl-5-fluorocytidine and/or β-D-2',6-dimethyl-uridine. Alternatively, the 3'-prodrugs can be administered in combination or alternation with other *anti-Flaviviridae* agent exhibits an EC₅₀ of less than 10 or 15 micromolar, or their prodrugs or pharmaceutically acceptable salts.

Nonlimiting examples of antiviral agents that can be used in combination with the compounds disclosed herein include: 1) an interferon and/or ribavirin; (2) Substrate-based NS3 protease inhibitors;. (3) Non-substrate-based inhibitors; (4) Thiazolidine derivatives; (5) Thiazolidines and benzanilides; (6) A phenan-threnequinone; (7) NS3 inhibitors; (8) HCV helicase inhibitors; (9) polymerase inhibitors, including RNA-dependent RNA-polymerase inhibitors; (10) Antisense oligodeoxynucleotides; (11) Inhibitors of IRES-dependent translation; (12) Nuclease-resistant ribozymes; and (13) other compounds that exhibit activity against a flaviviridae. The invention further includes administering the prodrug in combination or alternation with an immune modulator or other pharmaceutically active modifer of viral replication, including a biological material such as a protein, peptide, oligonucleotide, or gamma globulin, including but not limited to interfereon, interleukin, or an antisense oligonucleotides to genes which express or regulate *Flaviviridae* replication.

The prodrug can be administered in combination or alternation with an immune modulator or other pharmaceutically active modifer of viral replication, including a biological material such as a protein, peptide, oligonucleotide, or gamma globulin, including but not limited to interfereon, interleukin, or an antisense oligonucleotides to genes which express or regulate *Flaviviridae* replication.

In particular, the present invention provides the following:
(a) pharmaceutical compositions for the treatment of a *Flaviviridae* infection in a host comprising a compound of Formula (IX), or its pharmaceutically acceptable salt thereof, together with a pharmaceutically acceptable carrier or dileuent;
(b) pharmaceutical compositions for the treatment of a *Flaviviridae* infection in a host comprising a compound of Formula (IX), or its pharmaceutically acceptable salt thereof, with one or more other effective antiviral agent, optionally with a pharmaceutically acceptable carrier or dileuent;
(c) a compound of Formula (IX), or its pharmaceutically acceptable salt thereof, optionally with a pharmaceutically acceptable carrier or dileuent for use in methods for the treatment of a *Flaviviridae* infection in a host.
(d) a compound of Formula (IX), or its pharmaceutically acceptable salt thereof, with one or more other effective antiviral agent, optionally with a pharmaceutically acceptable carrier or dileuent for use in methods for the treatment of a *Flaviviridae* infection in a host.

In an alternative embodiment, the parent nucleoside compound of any of the 3'-prodrugs (i.e.., the nucleosides without the 3'-cleavable moieties) are provided for use in methods for the treatment of a *Flaviviridae* infection and in particular a hepatitis C infection.

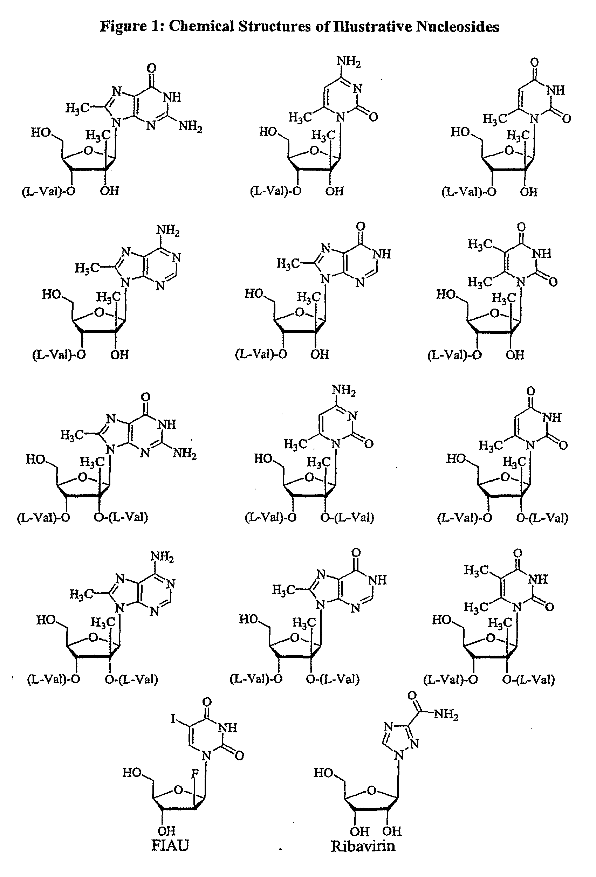

**Figure 1** provides the structure of various nucleosides, in particular FIAU and ribavirin.
**Figure 2** provides a non-limiting example of the steps involved in esterification of the 1', 2', 3' or 4'-branched β-D or β-L nucleoside to obtain a 2'-prodrug (not according to the invention). The same general procedure can be used to obtain the 3'-prodrug by selectively protecting the 2' and 5'-hydroxyl groups or protecting the 2', 3' and 5'-hydroxyl groups and selectively deprotecting the 3'-hydroxyl.
**Figure 3** provides a non-limiting example of the steps involved in esterification of the 1',2',3' or 4'-branched β-D or β-L nucleoside to obtain a 2'-prodrug (not according to the invention).
**Figure 4** provides a non-limiting example of the steps involved in esterification of the 1', 2', 3' or 4'-branched β-D or β-L nucleoside to obtain a 2',3'-prodrug (not according to the invention).

The invention as disclosed herein is a compound, a composition, and the compound for use in a method for the treatment of a *Flaviviridae* infection in humans and other host animals. The method includes the administration of an effective *anti-Flaviviridae* treatment amount of a compound as defined in the claims or a pharmaceutically acceptable salt thereof, optionally in a pharmaceutically acceptable carrier. The compounds of this invention either possesses antiviral (i.e., anti-HCV) activity, or are metabolized to a compound that exhibits such activity. HCV is a member of the *Flaviviridae* family. HCV has been placed in a new monotypic genus, hepacivirus. Therefore, in one embodiment, the *Flaviviridae* is HCV. In an alternate embodiment, the *Flaviviridae* is a flavivirus or pestivirus.

The 3'-prodrugs of a 2'-branched β-D or β-L nucleoside are acyl derivates of a secondary or tertiary alcohol alpha to a secondary or tertiary carbon. Due to the steric hindrance of these prodrugs over the 5'-prodrugs, an acyl derivative of a primary alcohol, these prodrugs differentially modulate the biological properties of the molecule *in vivo.* It has been discovered that the 3'-prodrugs of a 2'-branched β-D or β-L nucleoside can provide a drug with increased half-life and improved pharmacokinetic profile.

The 3'-prodrug in a preferred embodiment is a cleavable acyl group, and most particularly, an amino acid moiety, prepared from any naturally occurring and synthetic α, β γ or δ amino acid, including but is not limited to, glycine, alanine, valine, leucine, isoleucine, methionine, phenylalanine, tryptophan, proline, serine, threonine, cysteine, tyrosine, asparagine, glutamine, aspartate, glutamate, lysine, arginine and histidine. In a preferred embodiment, the amino acid is in the L-configuration. Alternatively, the amino acid can be a derivative of alanyl, valinyl, leucinyl, isoleuccinyl, prolinyl, phenylalaninyl, tryptophanyl, methioninyl, glycinyl, serinyl, threoninyl, cysteinyl, tyrosinyl, asparaginyl, glutaminyl, aspartoyl, glutaroyl, lysinyl, argininyl, histidinyl, β-alanyl, β-valinyl, β-leucinyl, β-isoleuccinyl, β-prolinyl, β-phenylalaninyl, β-tryptophanyl, β-methioninyl, β-glycinyl, β-serinyl, β-threoninyl, β-cysteinyl, β-tyrosinyl, β-asparaginyl, β-glutaminyl, β-aspartoyl, β-glutaroyl, β-lysinyl, β-argininyl or β-histidinyl. In one particular, embodiment, the moiety is a valine ester. One particularly preferred compound is the 3'-valine ester of 2',6-dimethyl-ribo-cytidine.

The oral bio-availability of 2'-branched β-D or β-L nucleoside as the neutral base and the HCl salt is low in rodents and non-human primates. It has been discovered that there is significant competition of 2'-branched β-D or β-L nucleoside with other nucleosides or nucleoside analogs for absorption, or transport, from the gastrointestinal tract and competition of other nucleosides or nucleoside analogs for the absorption with 2'-branched β-D or β-L nucleoside. In order to improve oral bioavailability and reduce the potential for drug-drug interaction, 3'-prodrugs of 2'-branched β-D or β-L nucleoside were obtained with higher oral bioavailability than the parent molecule and a reduced effect on the bioavailability of other nucleosides or nucleoside analogs used in combination.

The 3' and/or 5'-mono or divaline ester of a 2'-branched β-D or β-L nucleoside have higher oral bioavailability than the parent 2'-branched β-D or β-L nucleoside and reduced interaction with other nucleosides or nucleoside analogs when used in combination as compared to 2'-branched β-D or β-L nucleoside.

The 3' and/or 5'-mono or divaline ester of a 2'-branched β-D or β-L nucleoside can be converted to the parent 2'-branched β-D or β-L nucleoside through de-esterification in the gastrointestinal mucosa, blood or liver. The 3' and/or 5'-mono or divaline ester of a 2'-branched β-D or β-L nucleoside can be actively transported from the gastrointestinal lumen after oral delivery into the bloodstream by an amino acid transporter function in the mucosa of the gastrointestinal tract. This accounts for the increase in oral bioavailability compared to the parent 2' -branched β-D or β-L nucleoside that is transported primarily by a nucleoside transporter function. There is also reduced competition for uptake of the 3' and/or 5'-mono or divaline ester of 2'-branched β-D or β-L nucleoside with other nucleosides or nucleoside analogs that are transported by the nucleoside transporter function and not the amino acid transporter function. As partial de-esterification of the divaline ester of 2'-branched β-D or β-L nucleoside occurs prior to complete absorption, the mono ester continues to be absorbed using the amino acid transporter function. Therefore, the desired outcome of better absorption, or bioavailability, and reduced competition with other nucleosides or nucleoside analogs for uptake into the bloodstream can be maintained.

In summary, the present invention includes the following features:
(a) a 3'-prodrug of a 2'-branched β-D or β-L nucleoside, as described herein, and pharmaceutically acceptable salts and compositions thereof;
(b) a 3'-prodrug of a 2'-branched β-D or β-L nucleoside as described herein, and pharmaceutically acceptable salts and compositions thereof for use in the treatment and/or prophylaxis of a *Flaviviridae* infection, especially in individuals diagnosed as having a *Flaviviridae* infection or being at risk of becoming infected by hepatitis C;
(c) a 3'-prodrug of a 2'-branched β-D or β-L nucleoside, or their pharmaceutically acceptable salts and compositions as described herein substantially in the absence of the opposite enantiomers of the described nucleoside, or substantially isolated from other chemical entities;
(d) processes for the preparation of a 3'-prodrug of a 2'-branched β-D or β-L nucleoside, as described in more detail below;
(e) pharmaceutical formulations comprising a 3'-prodrug of a 2'-branched β-D or β-L nucleoside or a pharmaceutically acceptable salt thereof together with a pharmaceutically acceptable carrier or diluent;
(f) pharmaceutical formulations comprising a 3'-prodrug of a 2'-branched β-D or β-L nucleoside or a pharmaceutically acceptable salt thereof together with one or more other effective anti-HCV agents, optionally in a pharmaceutically acceptable carrier or diluent;
(g) pharmaceutical formulations comprising a 3'-prodrug of a 2'-branched β-D or β-L nucleoside or a pharmaceutically acceptable salt thereof together with the parent of a different a 1', 2', 3' or 4'-branched β-D or β-L nucleoside, optionally in a pharmaceutically acceptable carrier or diluent;
(h) a 3'-prodrug of a 2'-branched β-D or β-L nucleoside, its pharmaceutically acceptable salt or composition for use in a method for the treatment and/or prophylaxis of a host infected with *Flaviviridae*;
(i) a 3'-prodrug of a 2'-branched β-D or β-L nucleoside, its pharmaceutically acceptable salt or composition in combination and/or alternation with one or more effective anti-HCV agent for use in a method for the treatment and/or prophylaxis of a host infected with *Flaviviridae*;
(j) a 3'-prodrug of a 2'-branched β-D or β-L nucleoside, or its pharmaceutically acceptable salt or composition with the parent of a different a 1', 2', 3' or 4'-branched β-D or β-L nucleoside for use in a method for the treatment and/or prophylaxis of a host infected with *Flaviviridae*;
(k) a 3'-prodrug of a β-D-2'-methyl-cytidine, or its pharmaceutically acceptable salt or composition thereof for use in a method for the treatment and/or prophylaxis of a host infected with *Flaviviridae*;
(l) a 3'-valyl or acetyl ester, of β-D-2'-methyl-cytidine, or its pharmaceutically acceptable salt or composition thereof for use in a method for the treatment and/or prophylaxis of a *Flaviviridae* infection in a host;

*Flaviviridae* included within the scope of this invention are discussed generally in Fields Virology, Editors: Fields, B. N., Knipe, D. M., and Howley, P. M., Lippincott-Raven Publishers, Philadelphia, PA, Chapter 31, 1996. In a particular embodiment of the invention, the *Flaviviridae* is HCV. In an alternate embodiment of the invention, the *Flaviviridae* is a flavivirus or pestivirus. Specific flaviviruses include, without limitation: Absettarov, Alfuy, Apoi, Aroa, Bagaza, Banzi, Bouboui, Bussuquara, Cacipacore, Carey Island, Dakar bat, Dengue 1, Dengue 2, Dengue 3, Dengue 4, Edge Hill, Entebbe bat, Gadgets Gully, Hanzalova, Hypr, Ilheus, Israel turkey meningoencephalitis, Japanese encephalitis, Jugra, Jutiapa, Kadam, Karshi, Kedougou, Kokobera, Koutango, Kumlinge, Kunjin, Kyasanur Forest disease, Langat, Louping ill, Meaban, Modoc, Montana myotis leukoencephalitis, Murray valley encephalitis, Naranjal, Negishi, Ntaya, Omsk hemorrhagic fever, Phnom-Penh bat, Powassan, Rio Bravo, Rocio, Royal Farm, Russian spring-summer encephalitis, Saboya, St. Louis encephalitis, Sal Vieja, San Perlita, Saumarez Reef, Sepik, Sokuluk, Spondweni, Stratford, Tembusu, Tyuleniy, Uganda S, Usutu, Wesselsbron, West Nile, Yaounde, Yellow fever, and Zika.

Pestiviruses included within the scope of this invention are discussed generally in Fields Virology, Editors: Fields, B. N., Knipe, D. M., and Howley, P. M., Lippincott-Raven Publishers, Philadelphia, PA, Chapter 33, 1996. Specific pestiviruses include, without limitation: bovine viral diarrhea virus ("BVDV"), classical swine fever virus ("CSFV," also called hog cholera virus), and border disease virus ("BDV").

### I. Active Compounds

In a principal embodiment, a compound of Formula (IX) or a pharmaceutically acceptable salt, or a tautomeric, or polymorphic form thereof, is provided, as well as the compound for use in a method for the treatment of a host infected with a *Flaviviridae* comprising administering an effective treatment amount of compound of Formula (IX): 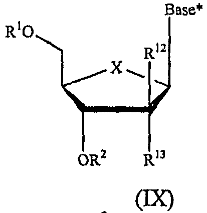 or a pharmaceutically acceptable salt or a tautomeric or polymorphic form thereof, wherein:
R¹,R², Y³ and X are as defined in the claims;
Base* is a purine or pyrimidine base;
R¹² is C(Y³)₃ and
R¹³ is fluoro;
X is O.

In an embodiment, a compound of Formula (IX), or a pharmaceutically acceptable salt, or a tautomeric or polymorphic form thereof, is provided, as well as the compound for use in a method for the treatment of a nost infected with a *Flaviviridae* comprising administering an effective treatment amount of compound of Formula (IX): 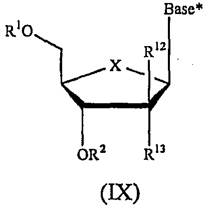 or a pharmaceutically acceptable salt thereof, or a tautomeric or polymorphic form thereof, wherein:
R¹ and R² are independently H; phosphate; straight chained, branched or cyclic alkyl; acyl; CO-alkyl; CO-aryl; CO-alkoxyalkyl; CO-aryloxyalkyl; CO-substituted aryl; sulfonate ester; benzyl, wherein the phenyl group is optionally substituted with one or more substituents; alkylsulfonyl; arylsulfonyl; aralkylsulfonyl; a lipid; an amino acid; a carbohydrate; a peptide; or cholesterol;
X is O;
Base* is a purine or pyrimidine base;
R¹² is C(Y³)₃;
Y³ is H; and
R¹³ is fluoro.

In one subembodiment, R¹ and R² are H.

### II. Stereochemistry

It is appreciated that nucleosides of the present invention have several chiral centers and may exist in and be isolated in optically active and racemic forms. Some compounds may exhibit polymorphism. It is to be understood that the present invention encompasses any racemic, optically-active, diastereomeric, polymorphic, or stereoisomeric form, or mixtures thereof, of a compound of the invention, which possess the useful properties described herein. It being well known in the art how to prepare optically active forms (for example, by resolution of the racemic form by recrystallization techniques, by synthesis from optically-active starting materials, by chiral synthesis, or by chromatographic separation using a chiral stationary phase).

In particular, since the 1' and 4' carbons of the nucleoside are chiral, their nonhydrogen substituents (the base and the CHOR groups, respectively) can be either cis (on the same side) or trans (on opposite sides) with respect to the sugar ring system. The four optical isomers therefore are represented by the following configurations (when orienting the sugar moiety in a horizontal plane such that the oxygen atom is in the back): cis (with both groups "up", which corresponds to the configuration of naturally occurring ß-D nucleosides), cis (with both groups "down", which is a nonnaturally occurring ß-L configuration), trans (with the C2' substituent "up" and the C4' substituent "down"), and trans (with the C2' substituent "down" and the C4' substituent "up"). The "D-nucleosides" are cis nucleosides in a natural configuration and the "L-nucleosides" are cis nucleosides in the nonnaturally occurring configuration.

Likewise, most amino acids are chiral (designated as L or D, wherein the L enantiomer is the naturally occurring configuration) and can exist as separate enantiomers.

Examples of methods to obtain optically active materials are known in the art, and include at least the following.
i) physical separation of crystals - a technique whereby macroscopic crystals of the individual enantiomers are manually separated. This technique can be used if crystals of the separate enantiomers exist, i.e., the material is a conglomerate, and the crystals are visually distinct;
ii) simultaneous crystallization - a technique whereby the individual enantiomers are separately crystallized from a solution of the racemate, possible only if the latter is a conglomerate in the solid state;
iii) enzymatic resolutions - a technique whereby partial or complete separation of a racemate by virtue of differing rates of reaction for the enantiomers with an enzyme;
iv) enzymatic asymmetric synthesis - a synthetic technique whereby at least one step of the synthesis uses an enzymatic reaction to obtain an enantiomerically pure or enriched synthetic precursor of the desired enantiomer;
v) chemical asymmetric synthesis - a synthetic technique whereby the desired enantiomer is synthesized from an achiral precursor under conditions that produce asymmetry (i.e., chirality) in the product, which may be achieved using chiral catalysts or chiral auxiliaries;
vi) diastereomer separations - a technique whereby a racemic compound is reacted with an enantiomerically pure reagent (the chiral auxiliary) that converts the individual enantiomers to diastereomers. The resulting diastereomers are then separated by chromatography or crystallization by virtue of their now more distinct structural differences and the chiral auxiliary later removed to obtain the desired enantiomer;
vii) first- and second-order asymmetric transformations - a technique whereby diastereomers from the racemate equilibrate to yield a preponderance in solution of the diastereomer from the desired enantiomer or where preferential crystallization of the diastereomer from the desired enantiomer perturbs the equilibrium such that eventually in principle all the material is converted to the crystalline diastereomer from the desired enantiomer. The desired enantiomer is then released from the diastereomer;
viii) kinetic resolutions - this technique refers to the achievement of partial or complete resolution of a racemate (or of a further resolution of a partially resolved compound) by virtue of unequal reaction rates of the enantiomers with a chiral, non-racemic reagent or catalyst under kinetic conditions;
ix) enantiospecific synthesis from non-racemic precursors - a synthetic technique whereby the desired enantiomer is obtained from non-chiral starting materials and where the stereochemical integrity is not or is only minimally compromised over the course of the synthesis;
x) chiral liquid chromatography - a technique whereby the enantiomers of a racemate are separated in a liquid mobile phase by virtue of their differing interactions with a stationary phase. The stationary phase can be made of chiral material or the mobile phase can contain an additional chiral material to provoke the differing interactions;
xi) chiral gas chromatography - a technique whereby the racemate is volatilized and enantiomers are separated by virtue of their differing interactions in the gaseous mobile phase with a column containing a fixed non-racemic chiral adsorbent phase;
xii) extraction with chiral solvents - a technique whereby the enantiomers are separated by virtue of preferential dissolution of one enantiomer into a particular chiral solvent;
xiii) transport across chiral membranes - a technique whereby a racemate is placed in contact with a thin membrane barrier. The barrier typically separates two miscible fluids, one containing the racemate, and a driving force such as concentration or pressure differential causes preferential transport across the membrane barrier. Separation occurs as a result of the non-racemic chiral nature of the membrane which allows only one enantiomer of the racemate to pass through.

### III. Definitions

The term "alkyl", as used herein, unless otherwise specified, refers to a saturated straight, branched, or cyclic, primary, secondary, or tertiary hydrocarbon of typically C₁ to C₁₀, and specifically includes methyl, CF₃, CCl₃, CFCl₂, CF₂Cl, ethyl, CH₂CF₃, CF₂CF₃, propyl, isopropyl, cyclopropyl, butyl, isobutyl, secbutyl, *t*-butyl, pentyl, cyclopentyl, isopentyl, neopentyl, hexyl, isohexyl, cyclohexyl, cyclohexylmethyl, 3-methylpentyl, 2,2-dimethylbutyl, and 2,3-dimethylbutyl. The term includes both substituted and unsubstituted alkyl groups, and particularly includes halogenated alkyl groups, and even more particularly fluorinated alkyl groups. Non-limiting examples of moieties with which the alkyl group can be substituted are selected from the group consisting of halogen (fluoro, chloro, bromo or iodo), hydroxyl, amino, alkylamino, arylamino, alkoxy, aryloxy, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991, hereby incorporated by reference.

The term "lower alkyl", as used herein, and unless otherwise specified, refers to a C₁ to C₄ saturated straight, branched, or if appropriate, a cyclic (for example, cyclopropyl) alkyl group, including both substituted and unsubstituted moieties.

The term "alkylamino" or "arylamino" refers to an amino group that has one or two alkyl or aryl substituents, respectively. Unless otherwise specifically stated in this application, when alkyl is a suitable moiety, lower alkyl is preferred. Similarly, when alkyl or lower alkyl is a suitable moiety, unsubstituted alkyl or lower alkyl is preferred.

The term "protected" as used herein and unless otherwise defined refers to a group that is added to an oxygen, nitrogen, or phosphorus atom to prevent its further reaction or for other purposes. A wide variety of oxygen and nitrogen protecting groups are known to those skilled in the art of organic synthesis.

The term "aryl", as used herein, and unless otherwise specified, refers to phenyl, biphenyl, or naphthyl, and preferably phenyl. The term includes both substituted and unsubstituted moieties. The aryl group can be substituted with any described moiety, including, but not limited to,one or more moieties selected from the group consisting of halogen (fluoro, chloro, bromo or iodo), hydroxyl, amino, alkylamino, arylamino, alkoxy, aryloxy, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991.

The term "alkaryl" or "alkylaryl" refers to an alkyl group with an aryl substituent. The term aralkyl or arylalkyl refers to an aryl group with an alkyl substituent.

The term "halo", as used herein, includes chloro, bromo, iodo, and fluoro.

The term "purine" or "pyrimidine" base includes, but is not limited to, adenine, N⁶-alkylpurines, N⁶-acylpurines (wherein acyl is C(O)(alkyl, aryl, alkylaryl, or arylalkyl), N⁶-benzylpurine, N⁶-halopurine, N⁶-vinylpurine, N⁶-acetylenic purine, N⁶-acyl purine, N⁶-hydroxyalkyl purine, N⁶-alkylaminopurine, N⁶-thioalkyl purine, N²-alkylpurines, N²-alkyl-6-thiopurines, thymine, cytosine, 5-fluorocytosine, 5-methylcytosine, 6-azapyrimidine, including 6-azacytosine, 2- and/or 4-mercaptopyrmidine, uracil, 5-halouracil, including 5-fluorouracil, C⁵-alkylpyrimidines, C⁵-benzylpyrimidines, C⁵-halopyrimidines, C⁵-vinylpyrimidine, C⁵-acetylenic pyrimidine, C⁵-acyl pyrimidine, C⁵-hydroxyalkyl purine, C⁵-amidopyrimidine, C⁵-cyanopyrimidine, C⁵-iodopyrimidine, C⁶-iodo-pyrimidine, C⁵-Br-vinyl pyrimidine, C⁶-Br-vinyl pyrimidine, C⁵-nitropyrimidine, C⁵-amino-pyrimidine, N²-alkylpurines, N²-alkyl-6-thiopurines, 5-azacytidinyl, 5-azauracilyl, triazolopyridinyl, imidazolopyridinyl, pyrrolopyrimidinyl, and pyrazolopyrimidinyl. Purine bases include, but are not limited to, guanine, adenine, hypoxanthine, 2,6-diaminopurine, and 6-chloropurine. Functional oxygen and nitrogen groups on the base can be protected as necessary or desired. Suitable protecting groups are well known to those skilled in the art, and include trimethylsilyl, dimethylhexylsilyl, *t-*butyldimethylsilyl, and *t*-butyldiphenylsilyl, trityl, alkyl groups, and acyl groups such as acetyl and propionyl, methanesulfonyl, and p-toluenesulfonyl.

The term "acyl" or "O-linked ester" refers to a group of the formula C(O)R', wherein R' is an straight, branched, or cyclic alkyl (including lower alkyl), amino acid, aryl including phenyl, alkaryl, aralkyl including benzyl, alkoxyalkyl including methoxymethyl, aryloxyalkyl such as phenoxymethyl; or substituted alkyl (including lower alkyl), aryl including phenyl optionally substituted with chloro, bromo, fluoro, iodo, C₁ to C₄ alkyl or C₁ to C₄ alkoxy, sulfonate esters such as alkyl or aralkyl sulphonyl including methanesulfonyl, the mono, di or triphosphate ester, trityl or monomethoxy-trityl, substituted benzyl, alkaryl, aralkyl including benzyl, alkoxyalkyl including methoxymethyl, aryloxyalkyl such as phenoxymethyl. Aryl groups in the esters optimally comprise a phenyl group. In particular, acyl groups include acetyl, trifluoroacetyl, methylacetyl, cyclopropylacetyl, cyclopropyl carboxy, propionyl, butyryl, hexanoyl, heptanoyl, octanoyl, neo-heptanoyl, phenylacetyl, 2-acetoxy-2-phenylacetyl, diphenylacetyl, α-methoxy-α-trifluoromethyl-phenylacetyl, bromoacetyl, 2-nitro-benzeneacetyl, 4-chloro-benzeneacetyl, 2-chloro-2,2-diphenylacetyl, 2-chloro-2-phenylacetyl, trimethylacetyl, chlorodifluoroacetyl, perfluoroacetyl, fluoroacetyl, bromodifluoroacetyl, methoxyacetyl, 2-thiopheneacetyl, chlorosulfonylacetyl, 3-methoxyphenylacetyl, phenoxyacetyl, tert-butylacetyl, trichloroacetyl, monochloro-acetyl, dichloroacetyl, 7H-dodecafluoro-heptanoyl, perfluoroheptanoyl, 7H-dodeca-fluoroheptanoyl, 7-chlorododecafluoro-heptanoyl, 7-chlorododecafluoro-heptanoyl, 7H-dodecafluoroheptanoyl, 7H-dodeca-fluoroheptanoyl, nonafluoro-3,6-dioxa-heptanoyl, nonafluoro-3,6-dioxaheptanoyl, perfluoroheptanoyl, methoxybenzoyl, methyl 3-amino-5-phenylthiophene-2-carboxyl, 3,6-dichloro-2-methoxybenzoyl, 4-(1,1,2,2-tetrafluoro-ethoxy)-benzoyl, 2-bromo-propionyl, omega-aminocapryl, decanoyl, n-pentadecanoyl, stearyl, 3-cyclopentyl-propionyl, 1-benzene-carboxyl, O-acetylmandelyl, pivaloyl acetyl, 1-adamantane-carboxyl, cyclohexane-carboxyl, 2,6-pyridinedicarboxyl, cyclopropane-carboxyl, cyclobutane-carboxyl, perfluorocyclohexyl carboxyl, 4-methylbenzoyl, chloromethyl isoxazolyl carbonyl, perfluorocyclohexyl carboxyl, crotonyl, 1-methyl-1H-indazole-3-carbonyl, 2-propenyl, isovaleryl, 1-pyrrolidinecarbonyl, 4-phenylbenzoyl. When the term acyl is used, it is meant to be a specific and independent disclosure of acetyl, trifluoroacetyl, methylacetyl, cyclopropylacetyl, propionyl, butyryl, hexanoyl, heptanoyl, octanoyl, neo-heptanoyl, phenylacetyl, diphenylacetyl, α-trifluoromethyl-phenylacetyl, bromoacetyl, 4-chloro-benzeneacetyl, 2-chloro-2,2-diphenylacetyl, 2-chloro-2-phenylacetyl, trimethylacetyl, chlorodifluoroacetyl, perfluoroacetyl, fluoroacetyl, bromodifluoroacetyl, 2-thiopheneacetyl, tert-butylacetyl, trichloroacetyl, monochloro-acetyl, dichloroacetyl, methoxybenzoyl, 2-bromo-propionyl, decanoyl, n-pentadecanoyl, stearyl, 3-cyclopentyl-propionyl, 1-benzene-carboxyl, pivaloyl acetyl, 1-adamantane-carboxyl, cyclohexane-carboxyl, 2,6-pyridinedicarboxyl, cyclopropane-carboxyl, cyclobutane-carboxyl, 4-methylbenzoyl, crotonyl, 1-methyl-1H-indazole-3-carbonyl, 2-propenyl, isovaleryl, 4-phenylbenzoyl.

The term "amino acid" includes naturally occurring and synthetic α, β γ or δ amino acids, and includes but is not limited to, amino acids found in proteins, *i.e.* glycine, alanine, valine, leucine, isoleucine, methionine, phenylalanine, tryptophan, proline, serine, threonine, cysteine, tyrosine, asparagine, glutamine, aspartate, glutamate, lysine, arginine and histidine. In a preferred embodiment, the amino acid is in the L-configuration. Alternatively, the amino acid can be a derivative of alanyl, valinyl, leucinyl, isoleuccinyl, prolinyl, phenylalaninyl, tryptophanyl, methioninyl, glycinyl, serinyl, threoninyl, cysteinyl, tyrosinyl, asparaginyl, glutaminyl, aspartoyl, glutaroyl, lysinyl, argininyl, histidinyl, (3-alanyl, β-valinyl, β-leucinyl, β-isoleuccinyl, β-prolinyl, β-phenylalaninyl, β-tryptophanyl, β-methioninyl, β-glycinyl, β-serinyl, β-threoninyl, β-cysteinyl, β-tyrosinyl, β-asparaginyl, β-glutaminyl, β-aspartoyl, β-glutaroyl, β-lysinyl, β-argininyl or β-histidinyl. Tables 1-24 set out examples of species within the present invention. When the term amino acid is used, it is considered to be a specific and independent disclosure of each of the esters of α, β γ or δ glycine, alanine, valine, leucine, isoleucine, methionine, phenylalanine, tryptophan, proline, serine, threonine, cysteine, tyrosine, asparagine, glutamine, aspartate, glutamate, lysine, arginine and histidine in the D and L-configurations.

As used herein, the term "substantially free of" or "substantially in the absence of" refers to a nucleoside composition that includes at least 85 or 90% by weight, preferably 95%, 98 %, 99% or 100% by weight, of the designated enantiomer of that nucleoside. In a preferred embodiment, in the methods and compounds of this invention, the compounds are substantially free of enantiomers.

Similarly, the term "isolated" refers to a nucleoside composition that includes at least 85%, 90%, 95%, 98%, 99%, or 100% by weight, of the nucleoside, the remainder comprising other chemical species or enantiomers.

The term "host", as used herein, refers to an unicellular or multicellular organism in which the virus can replicate, including cell lines and animals, and preferably a human. Alternatively, the host can be carrying a part of the *Flaviviridae,* viral genome, whose replication or function can be altered by the compounds of the present invention. The term host specifically refers to infected cells, cells transfected with all or part of the *Flaviviridae* genome and animals, in particular, primates (including chimpanzees) and humans. In most animal applications of the present invention, the host is a human patient. Veterinary applications, in certain indications, however, are clearly anticipated by the present invention (such as chimpanzees).

The term "pharmaceutically acceptable salt or prodrug" is used throughout the specification to describe any pharmaceutically acceptable form (such as an ester, phosphate ester, salt of an ester or a related group) of a nucleoside compound which, upon administration to a patient, provides the nucleoside compound. Pharmaceutically acceptable salts include those derived from pharmaceutically acceptable inorganic or organic bases and acids. Suitable salts include those derived from alkali metals such as potassium and sodium, alkaline earth metals such as calcium and magnesium, among numerous other acids well known in the pharmaceutical art. Pharmaceutically acceptable prodrugs refer to a compound that is metabolized, for example hydrolyzed or oxidized, in the host to form the compound of the present invention. Typical examples of prodrugs include compounds that have biologically labile protecting groups on a functional moiety of the active compound. Prodrugs include compounds that can be oxidized, reduced, aminated, deaminated, hydroxylated, dehydroxylated, hydrolyzed, dehydrolyzed, alkylated, dealkylated, acylated, deacylated, phosphorylated, dephosphorylated to produce the active compound. The compounds of this invention possess antiviral activity against a *Flaviviridae,* or are metabolized to a compound that exhibits such activity.

### IV. Prodrugs and Derivatives

The active compound can be administered as any salt or prodrug that upon administration to the recipient is capable of providing directly or indirectly the parent compound, or that exhibits activity itself. Nonlimiting examples are the pharmaceutically acceptable salts (alternatively referred to as "physiologically acceptable salts"), and a compound, which has been alkylated, acylated, or otherwise modified at the 5'-position, or on the purine or pyrimidine base (a type of "pharmaceutically acceptable prodrug"). Further, the modifications can affect the biological activity of the compound, in some cases increasing the activity over the parent compound. This can easily be assessed by preparing the salt or prodrug and testing its antiviral activity according to the methods described herein, or other methods known to those skilled in the art.

### A. Pharmaceutically Acceptable Salts

In cases where compounds are sufficiently basic or acidic to form stable nontoxic acid or base salts, administration of the compound as a pharmaceutically acceptable salt may be appropriate. Examples of pharmaceutically acceptable salts are organic acid addition salts formed by addition of acids, which form a physiological acceptable anion, for example, tosylate, methanesulfonate, acetate, citrate, malonate, tartarate, succinate, benzoate, ascorate, α-ketoglutarate, α-glycerophosphate, formate, fumarate, propionate, glycolate, lactate, pyruvate, oxalate, maleate, and salicylate. Suitable inorganic salts may also be formed, including, sulfate, nitrate, bicarbonate, carbonate salts, hydrobromate and phosphoric acid. In a preferred embodiment, the salt is a mono- or dihydrochloride salt.

Pharmaceutically acceptable salts may be obtained using standard procedures well known in the art, for example by reacting a sufficiently basic compound such as an amine with a suitable acid affording a physiologically acceptable anion. Alkali metal (for example, sodium, potassium or lithium) or alkaline earth metal (for example calcium) salts of carboxylic acids can also be made. In one embodiment, the salt is a hydrochloride salt of the compound. In another embodiment, the pharmaceutically acceptable salt is a dihydrochloride salt.

### B. Nucleotide Prodrug Formulations

The nucleosides described herein can be administered as a nucleotide prodrug to increase the activity, bioavailability, stability or otherwise alter the properties of the nucleoside. A number of nucleotide prodrug ligands are known. In general, alkylation, acylation or other lipophilic modification of the mono-, di- or triphosphate of the nucleoside reduces polarity and allows passage into cells. Examples of substituent groups that can replace one or more hydrogens on the phosphate moiety are alkyl, aryl, steroids, carbohydrates, including sugars, 1,2-diacylglycerol and alcohols. Many are described in R. Jones and N. Bischoferger, Antiviral Research, 1995, 27:1-17. Any of these can be used in combination with the disclosed nucleosides to achieve a desired effect.

In an alternative embodiment, the nucleoside is delivered as a phosphonate or a SATE derivative.

The active nucleoside can also be provided as a 3' and/or 5'-phosphoether lipid or a 3' and/or 5'-ether lipid. Non-limiting examples are described include the following references: Kucera, L.S., N. Iyer, E. Leake, A. Raben, Modest E.K., D.L.W., and C. Piantadosi. 1990. "Novel membrane-interactive ether lipid analogs that inhibit infectious HIV-1 production and induce defective virus formation." AIDS Res. Hum. Retro Viruses. 6:491-501; Piantadosi, C., J. Marasco C.J., S.L. Morris-Natschke, K.L. Meyer, F. Gumus, J.R. Surles, K.S. Ishaq, L.S. Kucera, N. Iyer, C.A. Wallen, S. Piantadosi, and E.J. Modest. 1991. "Synthesis and evaluation of novel ether lipid nucleoside conjugates for anti-HIV activity." J. Med. Chem. 34:1408.1414; Hosteller, K.Y., D.D. Richman, D.A. Carson, L.M. Stuhmiller, G.M. T. van Wijk, and H. van den Bosch. 1992. "Greatly enhanced inhibition of human immunodeficiency virus type 1 replication in CEM and HT4-6C cells by 3'-deoxythymine diphosphate dimyristoylglycerol, a lipid prodrug of 3,-deoxythymine." Antimicrob. Agents Chemother. 36:2025.2029; Hosetler, K.Y., L.M. Stuhmiller, H.B. Lenting, H. van den Bosch, and D.D. Richman, 1990. "Synthesis and antiretroviral activity of phospholipid analogs of azidothymine and other antiviral nucleosides." J. Biol. Chem. 265:6112.6117.

Nonlimiting examples of U.S. patents that disclose suitable lipophilic substituents that can be covalently incorporated into the nucleoside, preferably at the 3' and/or 5'-OH position of the nucleoside or lipophilic preparations, include U.S. Patent Nos. 5,149,794 (Sep. 22, 1992, Yatvin et al.); 5,194,654 (Mar. 16, 1993, Hostetler et al.); 5,223,263 (June 29, 1993, Hostetler et al.*);* 5,256,641 (Oct. 26, 1993, Yatvin et al.); 5,411,947 (May 2, 1995, Hostetler et al.); 5,463,092 (Oct. 31, 1995, Hostetler et al.); 5,543,389 (Aug. 6, 1996, Yatvin et al.); 5,543,390 (Aug. 6, 1996, Yatvin et al.); 5,543,391 (Aug. 6, 1996, Yatvin et al.); and 5,554,728 (Sep. 10, 1996; Basava et al.). Foreign patent applications that disclose lipophilic substituents that can be attached to the nucleosides of the present invention, or lipophilic preparations, include WO 89/02733, W0 90/00555, W0 91/16920, W0 91/18914, W0 93/00910, W0 94/26273, W0 96/15132, EP 0 350 287, EP 93917054.4, and W0 91/19721.

Aryl esters, especially phenyl esters, are also provided. Nonlimiting examples are disclosed in DeLambert et al., J. Med. Chem. 37: 498 (1994). Phenyl esters containing a carboxylic ester ortho to the phosphate are also provided. Khamnei and Torrence, J. Med. Chem.; 39:4109-4115 (1996). In particular, benzyl esters, which generate the parent compound, in some cases using substituents at the ortho- or para-position to accelerate hydrolysis, are provided. Examples of this class of prodrugs are described by Mitchell et al., J. Chem. Soc. Perkin Trans. I 2345 (1992); and Glazier et al. WO 91/19721.

Cyclic and noncyclic phosphonate esters are also provided. Nonlimiting examples are disclosed in Hunston et al., J. Med. Chem. 27: 440-444 (1984) and Starrett et al. J. Med. Chem. 37: 1857-1864 (1994). Additionally, cyclic 3',5'-phosphate esters are provided. Nonlimiting examples are disclosed in Meier et al. J. Med. Chem. 22: 811-815 (1979). Cyclic 1',3'-propanyl phosphonate and phosphate esters, such as ones containing a fused aryl ring, i.e. the cyclosaligenyl ester, are also provided (Meier et al., Bioorg. Med. Chem. Lett. 7: 99-104 (1997)). Unsubstituted cyclic 1',3'-propanyl esters of the monophosphates are also provided (Farquhar et al., J. Med. Chem. 26: 1153 (1983); Farquhar et al., J. Med. Chem. 28: 1358 (1985)) were prepared. In addition, cyclic 1',3'-propanyl esters substituted with a pivaloyloxy methyloxy group at C-1' are provided (Freed et al., Biochem. Pharmac. 38: 3193 (1989); Biller et al., U.S. Pat. No. 5,157,027).

Cyclic phosphoramidates are known to cleave in vivo by an oxidative mechanism. A variety of substituted 1',3' propanyl cyclic phosphoramidates are provided. Non-limiting examples are disclosed by Zon, Progress in Med. Chem. 19, 205 (1982). Additionally, a number of 2'- and 3'-substituted proesters are provided. 2'-Substituents include methyl, dimethyl, bromo, trifluoromethyl, chloro, hydroxy, and methoxy; 3'-substituents including phenyl, methyl, trifluoromethyl, ethyl, propyl, i-propyl, and cyclohexyl. A variety of 1'-substituted analogs are also provided.

Cyclic esters of phosphorus-containing compounds are also provided. Non-limiting examples are described in the following:
- [1] di and tri esters of phosphoric acids as reported in Nifantyev et al., Phosphorus, Sulfur Silicon and Related Elements, 113: 1 (1996); Wijnberg et al., EP-180276 A1;
- [2] phosphorus (III) acid esters. Kryuchkov et al., Izv. Akad. Nauk SSSR, Ser. Khim. 6: 1244 (1987). Some of the compounds were claimed to be useful for the asymmetric synthesis of L-Dopa precursors. Sylvain et al., DE3512781 A1;
- [3] phosphoramidates. Shih et al., Bull. Inst. Chem. Acad. Sin, 41: 9 (1994); Edmundson et al., J. Chem. Res. Synop. 5: 122 (1989); and
- [4] phosphonates. Neidlein et al., Heterocycles 35: 1185 (1993).

Further, nonlimiting examples of U.S. and International Patent Applications that disclose suitable cyclic phosphoramidate prodrugs include U.S. Patent No. 6,312,662; WO 99/45016; WO 00/52015; WO 01/47935; and WO 01/18013 to Erion, et al. from Metabasis Therapeutics, Inc. Specifically, prodrugs of the formula below are provided:
Examples include: 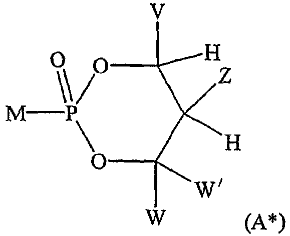 wherein:
   - together V and Z are connected via an additional 3-5 atoms to form a cyclic group containing 5-7 atoms, optionally 1 heteroatom, substituted with hydroxy, acyloxy, alkoxycarbonyloxy, or aryloxycarbonyloxy attached to a carbon atom that is three atoms from both O groups attached to the phosphorus; or
   - together V and Z are connected via an additional 3-5 atoms to form a cyclic group, optionally containing 1 heteroatom, that is fused to an aryl group at the beta and gamma position to the O attached to the phosphorus;
   - together V and W are connected via an additional 3 carbon atoms to form an optionally substituted cyclic group containing 6 carbon atoms and substituted with one substituent selected from the group consisting of hydroxy, acyloxy, alkoxycarbonyloxy, alkylthiocarbonyloxy, and aryloxycarbonyloxy, attached to one of said carbon atoms that is three atoms from an O attached to the phosphorus;
   - together Z and W are connected via an additional 3-5 atoms to form a cyclic group, optionally containing one heteroatom, and V must be aryl, substituted aryl, heteroaryl, or substituted heteroaryl;
   - together W and W' are connected via an additional 2-5 atoms to form a cyclic group, optionally containing 0-2 heteroatoms, and V must be aryl, substituted aryl, heteroaryl, or substituted heteroaryl;
   - Z is selected from the group consisting of -CHR² OH, -CHR² OC(O)R³, -CHR² OC(S)R³, -CHR² OC(S)OR³, -CHR² OC(O)SR³, -CHR² OCO₂ R³, -OR², -SR², - CHR² N₃, -CH² aryl, -CH(aryl)OH, -CH(CH=CR²₂)OH, -CH(C.ident.CR²)OH, -R², -NR²₂, -OCOR³, --OCO₂ R³, -SCOR³, -SCO₂ R³, -NHCOR², -NHCO₂ R³, -CH₂ NHaryl, -(CH₂)ₚ -OR¹², and -(CH₂)ₚ -SR¹² ;
   - p is an integer 2 or 3;
   - with the provisos that:
      - a) V, Z, W, W' are not all -H; and
      - b) when Z is --R², then at least one of V, W, and W' is not -H, alkyl, aralkyl, or alicyclic;
   - R² is selected from the group consisting of R³ and -H;
   - R³ is selected from the group consisting of alkyl, aryl, alicyclic, and aralkyl;
   - R¹² is selected from the group consisting of -H, and lower acyl;
   - M is the biologically active agent, and that is attached to the phosphorus in formula I via the 3' and/or 5'-hydroxyl.

### V. Combination or Alternation Therapy

The active compounds of the present invention can be administered in combination or alternation with another anti-flavivirus or pestivirus agent, or in particular an anti-HCV agent to treat any of the conditions described herein. In combination therapy, effective dosages of two or more agents are administered together, whereas in alternation or sequential-step therapy, an effective dosage of each agent is administered serially or sequentially. The dosages given will depend on absorption, inactivation and excretion rates of the drug as well as other factors known to those of skill in the art. It is to be noted that dosage values will also vary with the severity of the condition to be alleviated. It is to be further understood that for any particular subject, specific dosage regimens and schedules should be adjusted over time according to the individual need and the professional judgment of the person administering or supervising the administration of the compositions. In preferred embodiments, an anti-HCV (or anti-pestivirus or anti-flavivirus) compound that exhibits an EC₅₀ of 10-15 µM, or preferably less than 1-5 µM, is desirable.

It has been recognized that drug-resistant variants of flaviviruses, pestiviruses or HCV can emerge after prolonged treatment with an antiviral agent. Drug resistance most typically occurs by mutation of a gene that encodes for an enzyme used in viral replication. The efficacy of a drug against the viral infection can be prolonged, augmented, or restored by administering the compound in combination or alternation with a second, and perhaps third, antiviral compound that induces a different mutation from that caused by the principle drug. Alternatively, the pharmacokinetics, biodistribution or other parameter of the drug can be altered by such combination or alternation therapy. In general, combination therapy is typically preferred over alternation therapy because it induces multiple simultaneous stresses on the virus.

Any of the viral treatments described in the Background of the Invention can be used in combination or alternation with the compounds described in this specification. Nonlimiting examples include:
1) Protease inhibitors
   Examples include substrate-based NS3 protease inhibitors (Attwood et al., Antiviral peptide derivatives, PCT WO 98/22496, 1998; Attwood et al., Antiviral Chemistry and Chemotherapy 1999, 10, 259-273; Attwood et al., Preparation and use of amino acid derivatives as anti-viral agents, German Patent Pub. DE 19914474; Tung et al. Inhibitors of serine proteases, particularly hepatitis C virus NS3 protease, PCT WO 98/17679), including alphaketoamides and hydrazinoureas, and inhibitors that terminate in an electrophile such as a boronic acid or phosphonate (Llinas-Brunet et al, Hepatitis C inhibitor peptide analogues, PCT WO 99/07734); Non-substrate-based NS3 protease inhibitors such as 2,4,6-trihydroxy-3-nitro-benzamide derivatives (Sudo K. et al., Biochemical and Biophysical Research Communications, 1997, 238, 643-647; Sudo K. et al. Antiviral Chemistry and Chemotherapy, 1998, 9, 186), including RD3-4082 and RD3-4078, the former substituted on the amide with a 14 carbon chain and the latter processing a para-phenoxyphenyl group; and Sch 68631, a phenanthrenequinone, an HCV protease inhibitor (Chu M. et al., Tetrahedron Letters 37:7229-7232, 1996).
   Sch 351633, isolated from the fungus *Penicillium griseofulvum,* was identified as a protease inhibitor (Chu M. et al., Bioorganic and Medicinal Chemistry Letters 9:1949-1952). Eglin c, isolated from leech, is a potent inhibitor of several serine proteases such as S. griseus proteases A and B, α-chymotrypsin, chymase and subtilisin. Qasim M.A. et al., Biochemistry 36:1598-1607, 1997.
   U.S. patents disclosing protease inhibitors for the treatment of HCV include, for example, U.S. Patent No. 6,004,933 to Spruce et al. which discloses a class of cysteine protease inhibitors for inhibiting HCV endopeptidase 2; U.S. Patent No. 5,990,276 to Zhang et al. which discloses synthetic inhibitors of hepatitis C virus NS3 protease; U.S. Patent No. 5,538,865 to Reyes et a; WO 02/008251 to Corvas International, Inc, and WO 02/08187 and WO 02/008256 to Schering Corporation. HCV inhibitor tripeptides are disclosed in US Patent Nos. 6,534,523, 6,410,531, and 6,420,380 to Boehringer Ingelheim and WO 02/060926 to Bristol Myers Squibb. Diaryl peptides as NS3 serine protease inhibitors of HCV are disclosed in WO 02/48172 to Schering Corporation. Imidazoleidinones as NS3 serine protease inhibitors of HCV are disclosed in WO 02/08198 to Schering Corporation and WO 02/48157 to Bristol Myers Squibb. WO 98/17679 to Vertex Pharmaceuticals and WO 02/48116 to Bristol Myers Squibb also disclose HCV protease inhibitors.
2) Thiazolidine derivatives which show relevant inhibition in a reverse-phase HPLC assay with an NS3/4A fusion protein and NS5A/5B substrate (Sudo K. et al., Antiviral Research, 1996, 32, 9-18), especially compound RD-1-6250, possessing a fused cinnamoyl moiety substituted with a long alkyl chain, RD4 6205 and RD4 6193;
3) Thiazolidines and benzanilides identified in Kakiuchi N. et al. J. EBS Letters 421, 217-220; Takeshita N. et al. Analytical Biochemistry, 1997, 247, 242-246;
4) A phenan-threnequinone possessing activity against protease in a SDS-PAGE and autoradiography assay isolated from the fermentation culture broth of Streptomyces sp., Sch 68631 (Chu M. et al., Tetrahedron Letters, 1996, 37, 7229-7232), and Sch 351633, isolated from the fungus *Penicillium griseofulvum,* which demonstrates activity in a scintillation proximity assay (Chu M. et al., Bioorganic and Medicinal Chemistry Letters 9, 1949-1952);
5) Helicase inhibitors (Diana G.D. et al., Compounds, compositions and methods for treatment of hepatitis C, U.S. Pat. No. 5,633,358; Diana G.D. et al., Piperidine derivatives, pharmaceutical compositions thereof and their use in the treatment of hepatitis C, PCT WO 97/36554);
6) Nucleotide polymerase inhibitors and gliotoxin (Ferrari R. et al. Journal of Virology, 1999, 73, 1649-1654), and the natural product cerulenin (Lohmann V. et al., Virology, 1998, 249, 108-118);
7) Antisense phosphorothioate oligodeoxynucleotides (S-ODN) complementary to sequence stretches in the 5' non-coding region (NCR) of the virus (Alt M. et al., Hepatology, 1995, 22, 707-717), or nucleotides 326-348 comprising the 3' end of the NCR and nucleotides 371-388 located in the core coding region of the HCV RNA (Alt M. et al., Archives of Virology, 1997, 142, 589-599; Galderisi U. et al., Journal of Cellular Physiology, 1999, 181, 251-257);
8) Inhibitors of IRES-dependent translation (Ikeda N et al., Agent for the prevention and treatment of hepatitis C, Japanese Patent Pub. JP-08268890; Kai Y. et al. Prevention and treatment of viral diseases, Japanese Patent Pub. JP-10101591);
9) Ribozymes, such as nuclease-resistant ribozymes (Maccjak, D. J. et al., Hepatology 1999, 30, abstract 995) and those disclosed in U.S. Patent No. 6,043,077 to Barber et al., and U.S. Patent Nos. 5,869,253 and 5,610,054 to Draper et al.; and
10) Nucleoside analogs have also been developed for the treatment of Flaviviridae infections.
11) any of the compounds described by Idenix Pharmaceuticals in International Publication Nos. WO 01/90121 and WO 01/92282; .
12) Compound in other patent applications disclosing the use of certain nucleoside analogs to treat hepatitis C virus include: PCT/CA00/01316 (WO 01/32153; filed November 3, 2000) and PCT/CA01/00197 (WO 01/60315; filed February 19, 2001) filed by BioChem Pharma, Inc. (now Shire Biochem, Inc.); PCT/US02/01531 (WO 02/057425; filed January 18, 2002) and PCT/US02/03086 (WO 02/057287; filed January 18, 2002) filed by Merck & Co., Inc., PCT/EP01/09633 (WO 02/18404; published August 21, 2001) filed by Roche, and PCT Publication Nos. WO 01/79246 (filed April 13, 2001), WO 02/32920 (filed October 18, 2001) and WO 02/48165 by Pharmasset, Ltd.
13) PCT Publication No. WO 99/43691 to Emory University, entitled "2'-Fluoronucleosides" discloses the use of certain 2'-fluoronucleosides to treat HCV.
14) Other miscellaneous compounds including 1-amino-alkylcyclohexanes (U.S. Patent No. 6,034,134 to Gold et al.), alkyl lipids (U.S. Pat. No. 5,922,757 to Chojkier et al.), vitamin E and other antioxidants (U.S. Pat. No. 5,922,757 to Chojkier et al.), squalene, amantadine, bile acids (U.S. Pat. No. 5,846,964 to Ozeki et al.), N-(phosphonoacetyl)-L-aspartic acid, (U.S. Pat. No. 5,830,905 to Diana et al.), benzenedicarboxamides (U.S. Pat. No. 5,633,388 to Diana et al.), polyadenylic acid derivatives (U.S. Pat. No. 5,496,546 to Wang et al.), 2',3'-dideoxyinosine (U.S. Pat. No. 5,026,687 to Yarchoan et al.), benzimidazoles (U.S. Pat. No. 5,891,874 to Colacino et al.), plant extracts (U.S. Patent No. 5,837,257 to Tsai et al., U.S. Patent No. 5,725,859 to Omer et al., and U.S. Patent No. 6,056,961), and piperidenes (U.S. Patent No. 5,830,905 to Diana et al.).
15) Other compounds currently in preclinical or clinical development for treatment of hepatitis c virus include: Interleukin-10 by Schering-Plough, IP-501 by Interneuron, Merimebodib (VX-497) by Vertex, AMANTADINE® (Symmetrel) by Endo Labs Solvay, HEPTAZYME® by RPI, IDN-6556 by Idun Pharma., XTL-002 by XTL., HCV/MF59 by Chiron, CIVACIR® (Hepatitis C Immune Globulin) by NABI, LEVOVIRIN® by ICN/Ribapharm, VIRAMIDINE® by ICN/Ribapharm, ZADAXIN® (thymosin alpha-1) by Sci Clone, thymosin plus pegylated interferon by Sci Clone, CEPLENE® (histamine dihydrochloride) by Maxim, VX 950 / LY 570310 by Vertex/Eli Lilly, ISIS 14803 by Isis Pharmaceutical/Elan, IDN-6556 by Idun Pharmaceuticals, Inc., JTK 003 by AKROS Pharma, BILN-2061 by Boehringer Ingelheim, CellCept (mycophenolate mofetil) by Roche, T67, a β-tubulin inhibitor, by Tularik, a therapeutic vaccine directed to E2 by Innogenetics, FK788 by Fujisawa Healthcare, Inc., IdB 1016 (Siliphos, oral silybin-phosphatdylcholine phytosome), RNA replication inhibitors (VP50406) by ViroPharma/Wyeth, therapeutic vaccine by Intercell, therapeutic vaccine by Epimmune/Genencor, IRES inhibitor by Anadys, ANA 245 and ANA 246 by Anadys, immunotherapy (Therapore) by Avant, protease inhibitor by Corvas/SChering, helicase inhibitor by Vertex, fusion inhibitor by Trimeris, T cell therapy by CellExSys, polymerase inhibitor by Biocryst, targeted RNA chemistry by PTC Therapeutics, Dication by Immtech, Int., protease inhibitor by Agouron, protease inhibitor by Chiron/Medivir, antisense therapy by AVI BioPharma, antisense therapy by Hybridon, hemopurifier by Aethlon Medical, therapeutic vaccine by Merix, protease inhibitor by Bristol-Myers Squibb/Axys, Chron-VacC, a therapeutic vaccine, by Tripep, UT 231B by United Therapeutics, protease, helicase and polymerase inhibitors by Genelabs Technologies, IRES inhibitors by Immusol, R803 by Rigel Pharmaceuticals, INFERGEN® (interferon alphacon-1) by InterMune, OMNIFERON® (natural interferon) by Viragen, ALBUFERON® by Human Genome Sciences, REBIF® (interferon beta-1a) by Ares-Serono, Omega Interferon by BioMedicine, Oral Interferon Alpha by Amarillo Biosciences, interferon gamma, interferon tau, and Interferon gamma- 1b by InterMune.

### VI Pharmaceutical Compositions

Hosts, including humans, infected with pestivirus, flavivirus, HCV infection, or any other condition described herein, or another organism replicating through a RNA-dependent RNA viral polymerase, or for treating any other disorder described herein, can be treated by administering to the patient an effective amount of the active compound or a pharmaceutically acceptable prodrug or salt thereof in the presence of a pharmaceutically acceptable carrier or dilutent. The active materials can be administered by any appropriate route, for example, orally, parenterally, intravenously, intradermally, subcutaneously, or topically, in liquid or solid form.

A preferred dose of the compound for pestivirus, flavivirus or HCV will be in the range from about 1 to 50 mg/kg, preferably 1 to 20 mg/kg, of body weight per day, more generally 0.1 to about 100 mg per kilogram body weight of the recipient per day. Lower doses may be preferable, for example doses of 0.5-100 mg, 0.5-50 mg, 0.5-10 mg, or 0.5-5 mg per kilogram body weight per day. Even lower doses may be useful, and thus ranges can include from 0.1-0.5 mg per kilogram body weight per day. The effective dosage range of the pharmaceutically acceptable salts and prodrugs can be calculated based on the weight of the parent nucleoside to be delivered. If the salt or prodrug exhibits activity in itself, the effective dosage can be estimated as above using the weight of the salt or prodrug, or by other means known to those skilled in the art.

The compound is conveniently administered in unit any suitable dosage form, including but not limited to one containing 7 to 3000 mg, preferably 70 to 1400 mg of active ingredient per unit dosage form. An oral dosage of 50-1000 mg is usually convenient, including in one or multiple dosage forms of 50, 100, 200, 250, 300, 400, 500, 600, 700, 800, 900 or 1000 mgs. Lower doses may be preferable, for example from 10-100 or 1-50 mg. Also contemplated are doses of 0.1-50 mg, or 0.1-20 mg or 0.1-10.0 mg. Furthermore, lower doses may be utilized in the case of administration by a non-oral route, as, for example, by injection or inhalation.

Ideally the active ingredient should be administered to achieve peak plasma concentrations of the active compound of from about 0.2 to 70 µM, preferably about 1.0 to 10 µM. This may be achieved, for example, by the intravenous injection of a 0.1 to 5% solution of the active ingredient, optionally in saline, or administered as a bolus of the active ingredient.

The concentration of active compound in the drug composition will depend on absorption, inactivation and excretion rates of the drug as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed composition. The active ingredient may be administered at once, or may be divided into a number of smaller doses to be administered at varying intervals of time.

A preferred mode of administration of the active compound is oral. Oral compositions will generally include an inert diluent or an edible carrier. They may be enclosed in gelatin capsules or compressed into tablets. For the purpose of oral therapeutic administration, the active compound can be incorporated with excipients and used in the form of tablets, troches, or capsules. Pharmaceutically compatible binding agents, and/or adjuvant materials can e included as part of the composition.

The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. When the dosage unit form is a capsule, it can contain, in addition to material of the above type, a liquid carrier such as a fatty oil. In addition, dosage unit forms can contain various other materials which modify the physical form of the dosage unit, for example, coatings of sugar, shellac, or other enteric agents.

The compound can be administered as a component of an elixir, suspension, syrup, wafer, chewing gum or the like. A syrup may contain, in addition to the active compounds, sucrose as a sweetening agent and certain preservatives, dyes and colorings and flavors.

The compound or a pharmaceutically acceptable prodrug or salts thereof can also be mixed with other active materials that do not impair the desired action, or with materials that supplement the desired action, such as antibiotics, antifungals, anti-inflammatories, or other antivirals, including other nucleoside compounds. Solutions or suspensions used for parenteral, intradermal, sucutaneous, or topical application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parental preparation can be enclosed in ampoules, disposable syringes or multiple dose vials made of glass or plastic.

If administered intravenously, preferred carriers are physiological saline or phosphate buffered saline (PBS).

In a preferred embodiment, the active compounds are prepared with carriers that will protect the compound against rapid elimination from the body, such as a controlled release formulation, including implants and microencapsulated delivery systems. biodegradable, biocompatible polymers can be used, such as ethylene vinyl acetate, polyanhydrides, polyglycolic acid, collagen, polyorthoesters and polylactic acid. Methods for preparation of such formulations will be apparent to those skilled in the art. The materials can also be obtained commercially from Alza Corporation.

Liposomal suspensions (including liposomes targeted to infected cells with monoclonal antibodies to viral antigens) are also preferred as pharmaceutically acceptable carriers. These may be prepared according to methods known to those skilled in the art, for example, as described in U.S. Patent No. 4,522,811. For example, liposome formulations may be prepared by dissolving appropriate lipid(s) (such as stearoyl phosphatidyl ethanolamine, stearoyl phosphatidyl choline, arachadoyl phosphatidyl choline, and cholesterol) in an inorganic solvent that is then evaporated, leaving behind a thin film of dried lipid on the surface of the container. An aqueous solution of the active compound or its monophosphate, diphosphate, and/or triphosphate derivatives is then introduced into the container. The container is then swirled by hand to free lipid material from the sides of the container and to disperse lipid aggregates, thereby forming the liposomal suspension.

### VII. Processes for the Preparation of Active Compounds

The nucleosides of the present invention can be synthesized by any means known in the art. In particular, the synthesis of the present nucleosides can be achieved by either alkylating the appropriately modified sugar, followed by glycosylation or glycosylation followed by alkylation of the nucleoside. The following non-limiting embodiments illustrate some general methodology to obtain the nucleosides of the present invention.

### A. General Synthesis of 1'-C-Branched Nucleosides (not according to the invention)

### 1) Modification from the lactone

The key starting material for this process is an appropriately substituted lactone. The lactone can be purchased or can be prepared by any known means including standard epimerization, substitution and cyclization techniques. The lactone can be optionally protected with a suitable protecting group, preferably with an acyl or silyl group, by methods well known to those skilled in the art, as taught by Greene et al. Protective Groups in Organic Synthesis, John Wiley and Sons, Second Edition, 1991. The protected lactone can then be coupled with a suitable coupling agent, such as an organometallic carbon nucleophile, such as a Grignard reagent, an organolithium, lithium dialkylcopper or R⁶-SiMe₃ in TBAF with the appropriate non-protic solvent at a suitable temperature, to give the 1'-alkylated sugar.

The optionally activated sugar can then be coupled to the BASE by methods well known to those skilled in the art, as taught by Townsend Chemistry of Nucleosides and Nucleotides, Plenum Press, 1994. For example, an acylated sugar can be coupled to a silylated base with a Lewis acid, such as tin tetrachloride, titanium tetrachloride or trimethylsilyltriflate in the appropriate solvent at a suitable temperature.

Subsequently, the nucleoside can be deprotected by methods well known to those skilled in the art, as taught by Greene et al. Protective Groups in Organic Synthesis, John Wiley and Sons, Second Edition, 1991.

In a particular embodiment, the 1'-C-branched ribonucleoside is desired. The synthesis of a ribonucleoside is shown in **Scheme 1.** Alternatively, deoxyribo-nucleoside is desired. To obtain these nucleosides, the formed ribonucleoside can optionally be protected by methods well known to those skilled in the art, as taught by Greene et al. Protective Groups in Organic Synthesis, John Wiley and Sons, Second Edition, 1991, and then the 2'-OH can be reduced with a suitable reducing agent. Optionally, the 2'-hydroxyl can be activated to facilitate reduction; i.e. via the Barton reduction.

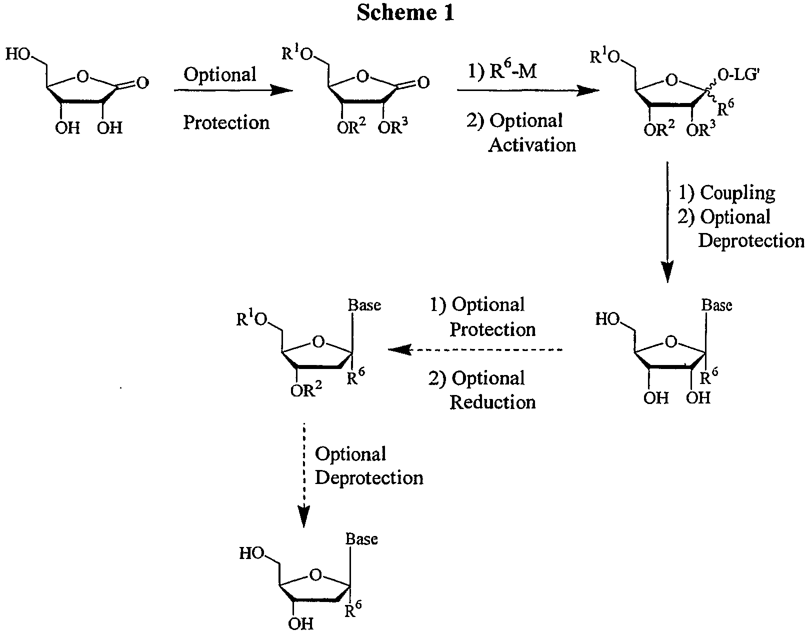

### 2. Alternative method for the preparation of 1'-C-branched nucleosides

The key starting material for this process is an appropriately substituted hexose. The hexose can be purchased or can be prepared by any known means including standard epimerization (e.g. via alkaline treatment), substitution and coupling techniques. The hexose can be selectively protected to give the appropriate hexa-furanose, as taught by Townsend Chemistry of Nucleosides and Nucleotides, Plenum Press, 1994.

The 1'-hydroxyl can be optionally activated to a suitable leaving group such as an acyl group or a halogen via acylation or halogenation, respectively. The optionally activated sugar can then be coupled to the BASE by methods well known to those skilled in the art, as taught by Townsend Chemistry of Nucleosides and Nucleotides, Plenum Press, 1994. For example, an acylated sugar can be coupled to a silylated base with a Lewis acid, such as tin tetrachloride, titanium tetrachloride or trimethylsilyltriflate in the appropriate solvent at a suitable temperature. Alternatively, a halo-sugar can be coupled to a silylated base with the presence of trimethylsilyltriflate.

The 1'-CH₂-OH, if protected, can be selectively deprotected by methods well known in the art. The resultant primary hydroxyl can be functionalized to yield various C-branched nucleosides. For example, the primary hydroxyl can be reduced to give the methyl, using a suitable reducing agent. Alternatively, the hydroxyl can be activated prior to reduction to facilitate the reaction; i.e. via the Barton reduction. In an alternate embodiment, the primary hydroxyl can be oxidized to the aldehyde, then coupled with a carbon nucleophile, such as a Grignard reagent, an organolithium, lithium dialkylcopper or R⁶-SiMe₃ in TBAF with the appropriate non-protic solvent at a suitable temperature.

In a particular embodiment, the 1'-C-branched ribonucleoside is desired. The synthesis of a ribonucleoside is shown in Scheme 2. Alternatively, deoxyribo-nucleoside is desired. To obtain these nucleosides, the formed ribonucleoside can optionally be protected by methods well known to those skilled in the art, as taught by Greene et al. Protective Groups in Organic Synthesis, John Wiley and Sons, Second Edition, 1991, and then the 2'-OH can be reduced with a suitable reducing agent. Optionally, the 2'-hydroxyl can be activated to facilitate reduction; i.e. via the Barton reduction.

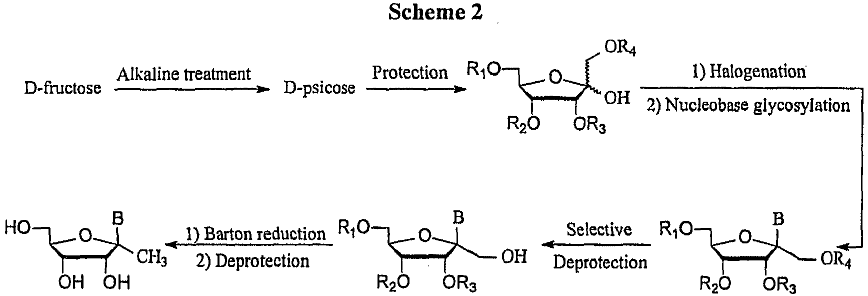

In addition, the L-enantiomers corresponding to the compounds of the invention can be prepared following the same general methods (1 or 2), beginning with the corresponding L-sugar or nucleoside L-enantiomer as starting material.

### B. General Synthesis of 2'-C-Branched Nucleosides

### 1. Glycosylation of the nucleobase with an appropriately modified sugar

The key starting material for this process is an appropriately substituted sugar with a 2'-OH and 2'-H, with the appropriate leaving group (LG), for example an acyl group or a halogen. The sugar can be purchased or can be prepared by any known means including standard epimerization, substitution, oxidation and reduction techniques. The substituted sugar can then be oxidized with the appropriate oxidizing agent in a compatible solvent at a suitable temperature to yield the 2'-modified sugar. Possible oxidizing agents are Jones reagent (a mixture of chromic acid and sulfuric acid), Collins's reagent (dipyridine Cr(VI) oxide, Corey's reagent (pyridinium chlorochromate), pyridinium dichromate, acid dichromate, potassium permanganate, MnO₂, ruthenium tetroxide, phase transfer catalysts such as chromic acid or permanganate supported on a polymer, Cl₂-pyridine, H₂O₂-ammonium molybdate, NaBrO₂-CAN NaOCl in HOAc, copper chromite, copper oxide, Raney nickel, palladium acetate, Meerwin-Pondorf-Verley reagent (aluminum t-butoxide with another ketone) and *N*-bromosuccinimide.

Then coupling of an organometallic carbon nucleophile, such as a Grignard reagent, an organolithium, lithium dialkylcopper or R⁶-SiMe₃ in TBAF with the ketone with the appropriate non-protic solvent at a suitable temperature, yields the 2'-alkylated sugar. The alkylated sugar can be optionally protected with a suitable protecting group, preferably with an acyl or silyl group, by methods well known to those skilled in the art, as taught by Greene et al. Protective Groups in Organic Synthesis, John Wiley and Sons, Second Edition, 1991.

The optionally protected sugar can then be coupled to the BASE by methods well known to those skilled in the art, as taught by Townsend Chemistry of Nucleosides and Nucleotides, Plenum Press, 1994. For example, an acylated sugar can be coupled to a silylated base with a Lewis acid, such as tin tetrachloride, titanium tetrachloride or trimethylsilyltriflate in the appropriate solvent at a suitable temperature. Alternatively, a halo-sugar can be coupled to a silylated base with the presence of trimethylsilyltriflate.

Subsequently, the nucleoside can be deprotected by methods well known to those skilled in the art, as taught by Greene et al. Protective Groups in Organic Synthesis, John Wiley and Sons, Second Edition, 1991.

In a particular embodiment, the 2'-C-branched ribonucleoside is desired. The synthesis of a ribonucleoside is shown in **Scheme 3.** Alternatively, deoxyribo-nucleoside is desired. To obtain these nucleosides, the formed ribonucleoside can optionally be protected by methods well known to those skilled in the art, as taught by Greene et al. Protective Groups in Organic Synthesis, John Wiley and Sons, Second Edition, 1991, and then the 2'-OH can be reduced with a suitable reducing agent. Optionally, the 2'-hydroxyl can be activated to facilitate reduction; i.e. via the Barton reduction.

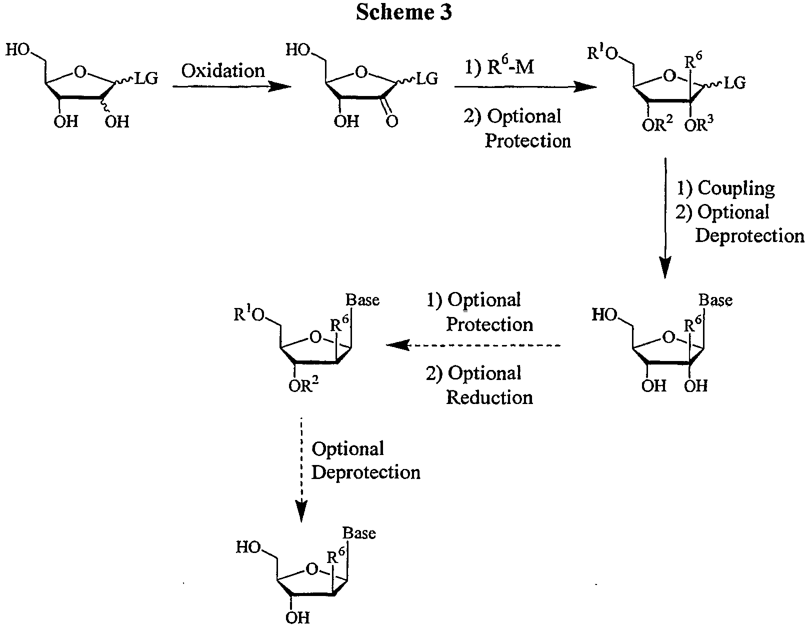

### 2. Modification of a pre-formed nucleoside

The key starting material for this process is an appropriately substituted nucleoside with a 2'-OH and 2'-H. The nucleoside can be purchased or can be prepared by any known means including standard coupling techniques. The nucleoside can be optionally protected with suitable protecting groups, preferably with acyl or silyl groups, by methods well known to those skilled in the art, as taught by Greene et al. Protective Groups in Organic Synthesis, John Wiley and Sons, Second Edition, 1991.

The appropriately protected nucleoside can then be oxidized with the appropriate oxidizing agent in a compatible solvent at a suitable temperature to yield the 2'-modified sugar. Possible oxidizing agents are Jones reagent (a mixture of chromic acid and sulfuric acid), Collins's reagent (dipyridine Cr(VI) oxide, Corey's reagent (pyridinium chlorochromate), pyridinium dichromate, acid dichromate, potassium permanganate, MnO₂, ruthenium tetroxide, phase transfer catalysts such as chromic acid or permanganate supported on a polymer, Cl₂-pyridine, H₂O₂-ammonium molybdate, NaBrO₂-CAN NaOCl in HOAc, copper chromite, copper oxide, Raney nickel, palladium acetate, Meerwin-Pondorf-Verley reagent (aluminum t-butoxide with another ketone) and N-bromosuccinimide.

Subsequently, the nucleoside can be deprotected by methods well known to those skilled in the art, as taught by GreeneGreene et al. Protective Groups in Organic Synthesis, John Wiley and Sons, Second Edition, 1991.

In a particular embodiment, the 2'-C-branched ribonucleoside is desired. The synthesis of a ribonucleoside is shown in **Scheme 4.** Alternatively, deoxyribo-nucleoside is desired. To obtain these nucleosides, the formed ribonucleoside can optionally be protected by methods well known to those skilled in the art, as taught by Greene et al. Protective Groups in Organic Synthesis, John Wiley and Sons, Second Edition, 1991, and then the 2'-OH can be reduced with a suitable reducing agent. Optionally, the 2'-hydroxyl can be activated to facilitate reduction; i.e. via the Barton reduction.

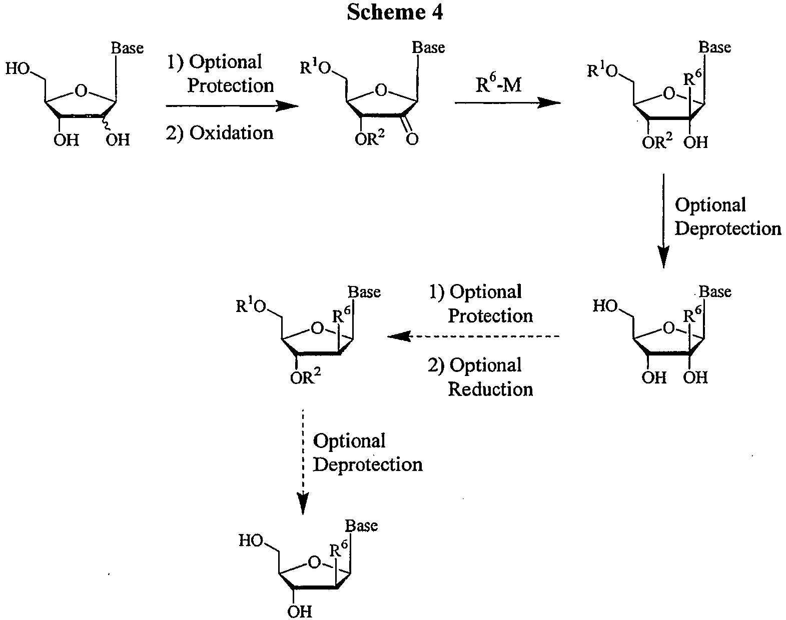

In another embodiment of the invention, the L-enantiomers are desired. Therefore, the L-enantiomers can be corresponding to the compounds of the invention can be prepared following the same foregoing general methods, beginning with the corresponding L-sugar or nucleoside L-enantiomer as starting material.

### C. General Synthesis of 3'-C-Branched Nucleosides (not according to the invention)

### 1 Glycosylation of the nucleobase with an appropriately modified sugar

The key starting material for this process is an appropriately substituted sugar with a 3'-OH and 3'-H, with the appropriate leaving group (LG), for example an acyl group or a halogen. The sugar can be purchased or can be prepared by any known means including standard epimerization, substitution, oxidation and reduction techniques. The substituted sugar can then be oxidized with the appropriate oxidizing agent in a compatible solvent at a suitable temperature to yield the 3'-modified sugar. Possible oxidizing agents are Jones reagent (a mixture of chromic acid and sulfuric acid), Collins's reagent (dipyridine Cr(VI) oxide, Corey's reagent (pyridinium chlorochromate), pyridinium dichromate, acid dichromate, potassium permanganate, MnO₂, ruthenium tetroxide, phase transfer catalysts such as chromic acid or permanganate supported on a polymer, Cl₂-pyridine, H₂O₂-ammonium molybdate, NaBrO₂-CAN, NaOCl in HOAc, copper chromite, copper oxide, Raney nickel, palladium acetate, Meerwin-Pondorf-Verley reagent (aluminum t-butoxide with another ketone) and *N*-bromosuccinimide.

Then coupling of an organometallic carbon nucleophile, such as a Grignard reagent, an organolithium, lithium dialkylcopper or R⁶-SiMe₃ in TBAF with the ketone with the appropriate non-protic solvent at a suitable temperature, yields the 3'-C-branched sugar. The 3'-C-branched sugar can be optionally protected with a suitable protecting group, preferably with an acyl or silyl group, by methods well known to those skilled in the art, as taught by Greene et al. Protective Groups in Organic Synthesis, John Wiley and Sons, Second Edition, 1991.

The optionally protected sugar can then be coupled to the BASE by methods well known to those skilled in the art, as taught by Townsend Chemistry of Nucleosides and Nucleotides, Plenum Press, 1994. For example, an acylated sugar can be coupled to a silylated base with a Lewis acid, such as tin tetrachloride, titanium tetrachloride or trimethylsilyltriflate in the appropriate solvent at a suitable temperature. Alternatively, a halo-sugar can be coupled to a silylated base with the presence of trimethylsilyltriflate.

Subsequently, the nucleoside can be deprotected by methods well known to those skilled in the art, as taught by Greene et al. Protective Groups in Organic Synthesis, John Wiley and Sons, Second Edition, 1991.

In a particular embodiment, the 3'-C-branched ribonucleoside is desired. The synthesis of a ribonucleoside is shown in **Scheme 5.** Alternatively, deoxyribo-nucleoside is desired. To obtain these nucleosides, the formed ribonucleoside can optionally be protected by methods well known to those skilled in the art, as taught by Greene et al. Protective Groups in Organic Synthesis, John Wiley and Sons, Second Edition, 1991, and then the 2'-OH can be reduced with a suitable reducing agent. Optionally, the 2'-hydroxyl can be activated to facilitate reduction; i.e. via the Barton reduction.

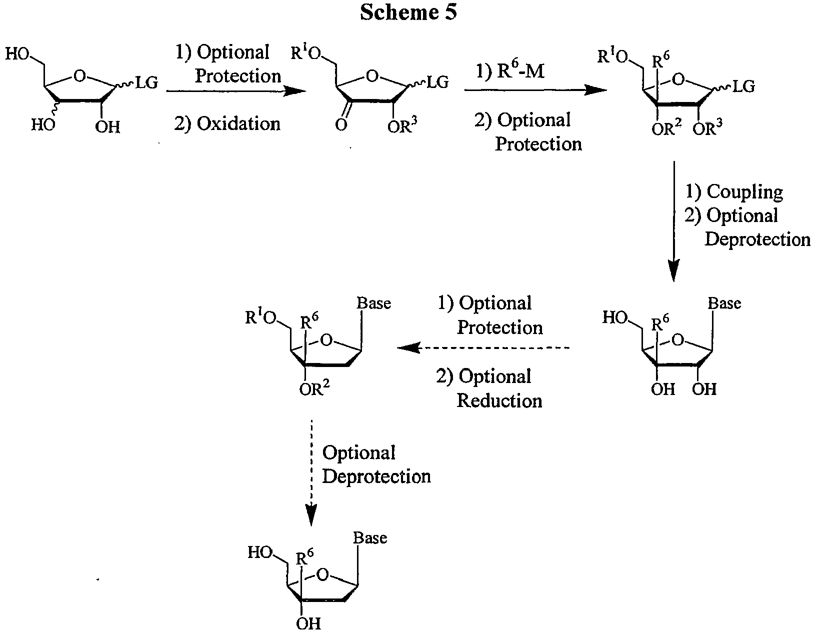

### 2. Modification of a pre-formed nucleoside

The key starting material for this process is an appropriately substituted nucleoside with a 3'-OH and 3'-H. The nucleoside can be purchased or can be prepared by any known means including standard coupling techniques. The nucleoside can be optionally protected with suitable protecting groups, preferably with acyl or silyl groups, by methods well known to those skilled in the art, as taught by Greene et al. Protective Groups in Organic Synthesis, John Wiley and Sons, Second Edition, 1991.

The appropriately protected nucleoside can then be oxidized with the appropriate oxidizing agent in a compatible solvent at a suitable temperature to yield the 2'-modified sugar. Possible oxidizing agents are Jones reagent (a mixture of chromic acid and sulfuric acid), Collins's reagent (dipyridine Cr(VI) oxide, Corey's reagent (pyridinium chlorochromate), pyridinium dichromate, acid dichromate, potassium permanganate, MnO₂, ruthenium tetroxide, phase transfer catalysts such as chromic acid or permanganate supported on a polymer, Cl₂-pyridine, H₂O₂-ammonium molybdate, NaBrO₂-CAN NaOCl in HOAc, copper chromite, copper oxide, Raney nickel, palladium acetate, Meerwin-Pondorf-Verley reagent (aluminum t-butoxide with another ketone) and N-bromosuccinimide.

Subsequently, the nucleoside can be deprotected by methods well known to those skilled in the art, as taught by Greene et al. Protective Groups in Organic Synthesis, John Wiley and Sons, Second Edition, 1991.

In a particular embodiment, the 3'-C-branched ribonucleoside is desired. The synthesis of a ribonucleoside is shown in **Scheme 6.** Alternatively, deoxyribo-nucleoside is desired. To obtain these nucleosides, the formed ribonucleoside can optionally be protected by methods well known to those skilled in the art, as taught by Greene et al. Protective Groups in Organic Synthesis, John Wiley and Sons, Second Edition, 1991, and then the 2'-OH can be reduced with a suitable reducing agent. Optionally, the 2'-hydroxyl can be activated to facilitate reduction; i.e. via the Barton reduction.

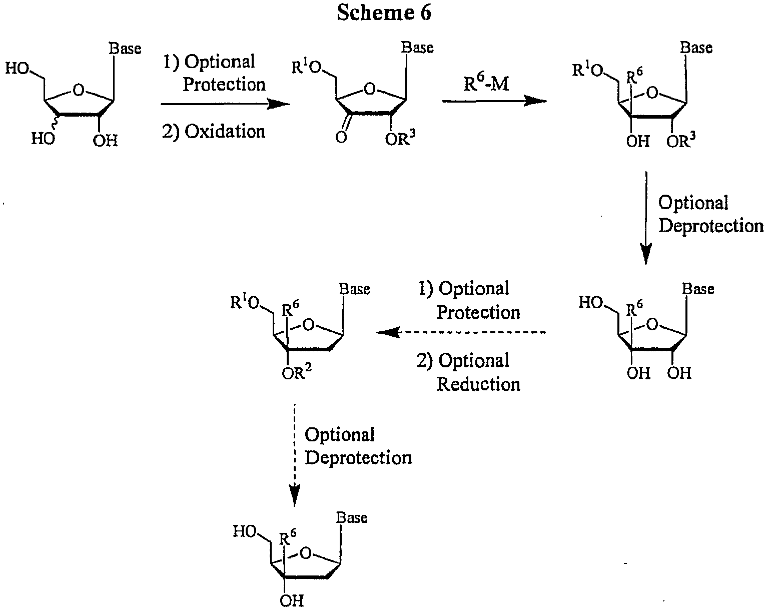

In another embodiment of the invention, the L-enantiomers are desired. Therefore, the L-enantiomers can be corresponding to the compounds of the invention can be prepared following the same foregoing general methods, beginning with the corresponding L-sugar or nucleoside L-enantiomer as starting material.

### D. General Synthesis of 4'-C-Branched Nucleosides (not according to the invention)

### 1. Modification from the pentodialdo-furanose

The key starting material for this process is an appropriately substituted pentodialdo-furanose. The pentodialdo-furanose can be purchased or can be prepared by any known means including standard epimerization, substitution and cyclization techniques.

In a preferred embodiment, the pentodialdo-furanose is prepared from the appropriately substituted hexose. The hexose can be purchased or can be prepared by any known means including standard epimerization (e.g. via alkaline treatment), substitution and coupling techniques. The hexose can be either in the furanose form, or cyclized via any means known in the art, such as methodology taught by Townsend Chemistry of Nucleosides and Nucleotides, Plenum Press, 1994, preferably by selectively protecting the hexose, to give the appropriate hexafuranose.

The 4'-hydroxymethylene of the hexafuranose then can be oxidized with the appropriate oxidizing agent in a compatible solvent at a suitable temperature to yield the 4'-aldo-modified sugar. Possible oxidizing agents are Swern reagents, Jones reagent (a mixture of chromic acid and sulfuric acid), Collins's reagent (dipyridine Cr(VI) oxide, Corey's reagent (pyridinium chlorochromate), pyridinium dichromate, acid dichromate, potassium permanganate, MnO₂, ruthenium tetroxide, phase transfer catalysts such as chromic acid or permanganate supported on a polymer, Cl₂-pyridine, H₂O₂-ammonium molybdate, NaBrO₂-CAN, NaOCl in HOAc, copper chromite, copper oxide, Raney nickel, palladium acetate, Meerwin-Pondorf-Verley reagent (aluminum *t*-butoxide with another ketone) and *N*-bromosuccinimide, though preferably using H₃PO₄, DMSO and DCC in a mixture of benzene/pyridine at room temperature.

Then, the pentodialdo-furanose can be optionally protected with a suitable protecting group, preferably with an acyl or silyl group, by methods well known to those skilled in the art, as taught by Greene et al. Protective Groups in Organic Synthesis, John Wiley and Sons, Second Edition, 1991. In the presence of a base, such as sodium hydroxide, the protected pentodialdo-furanose can then be coupled with a suitable electrophilic alkyl, halogeno-alkyl (i.e. CF₃), alkenyl or alkynyl (i.e. allyl), to obtain the 4'-alkylated sugar. Alternatively, the protected pentodialdo-furanose can be coupled with the corresponding carbonyl, such as formaldehyde, in the presence of a base, such as sodium hydroxide, with the appropriate polar solvent, such as dioxane, at a suitable temperature, which can then be reduced with an appropriate reducing agent to give the 4'-alkylated sugar. In one embodiment, the reduction is carried out using PhOC(S)Cl, DMAP, preferably in acetonitrile at room temperature, followed by treatment of ACCN and TMSS refluxed in toluene.

The optionally activated sugar can then be coupled to the BASE by methods well known to those skilled in the art, as taught by Townsend Chemistry of Nucleosides and Nucleotides, Plenum Press, 1994. For example, an acylated sugar can be coupled to a silylated base with a Lewis acid, such as tin tetrachloride, titanium tetrachloride or trimethylsilyltriflate in the appropriate solvent at a suitable temperature.

Subsequently, the nucleoside can be deprotected by methods well known to those skilled in the art, as taught by Greene et al. Protective Groups in Organic Synthesis, John Wiley and Sons, Second Edition, 1991.

In a particular embodiment, the 4'-C-branched ribonucleoside is desired. Alternatively, deoxyribonucleoside is desired. To obtain these deoxyribo-nucleosides, a formed ribo-nucleoside can optionally be protected by methods well known to those skilled in the art, as taught by Greene et al. Protective Groups in Organic Synthesis, John Wiley and Sons, Second Edition, 1991, and then the 2'-OH can be reduced with a suitable reducing agent. Optionally, the 2'-hydroxyl can be activated to facilitate reduction; i.e. via the Barton reduction.

In another embodiment of the invention, the L-enantiomers are desired. Therefore, the L-enantiomers can be corresponding to the compounds of the invention can be prepared following the same foregoing general methods, beginning with the corresponding L-pentodialdo-furanose as starting material.

### E. General Synthesis of 2' and/or 3'-Prodrugs

The key starting material for this process is an appropriately substituted 1', 2', 3' or 4'-branched β-D or β-L nucleosides. The branched nucleoside can be purchased or can be prepared by any known means including the techniques disclosed herein. The branched nucleoside can be optionally protected with a suitable protecting group, preferably with a silyl group, by methods well known to those skilled in the art, as taught by Greene et al. Protective Groups in Organic Synthesis, John Wiley and Sons, Second Edition, 1991. The protected branched nucleoside can then be coupled with a suitable acyl doner, such as an acyl chloride and/or an acyl anhydride with the appropriate protic or aprotic solvent at a suitable temperature, to give the 2' and/or 3' prodrug of 1', 2', 3' or 4'-branched β-D or β-L nucleoside. Alternatively, the protected branched nucleoside can then be coupled with a suitable acyl, such as a carboxylic acid, such as alkanoic acid and/or amino acid residue, optionally with a suitable coupling agent, with the appropriate aprotic solvent at a suitable temperature, to give the 2' and/or 3' prodrug of 1', 2', 3' or 4'-branched β-D or β-L nucleoside. Possible coupling reagents are any reagents that promote coupling, including but are not limiting to, Mitsunobu reagents (e.g. diisopropyl azodicarboxylate and diethyl azodicarboxylate) with triphenylphosphine or various carbodiimides.

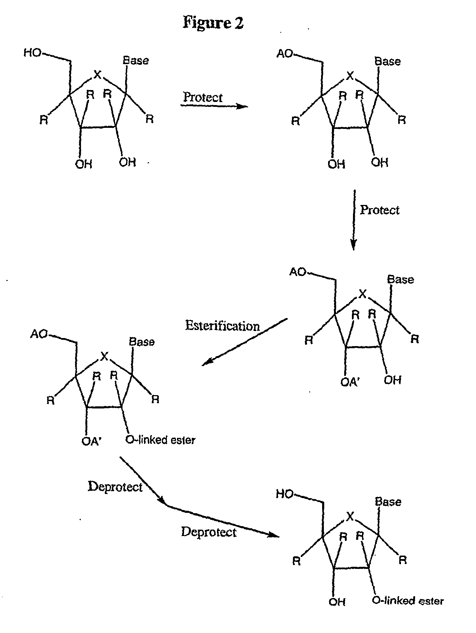

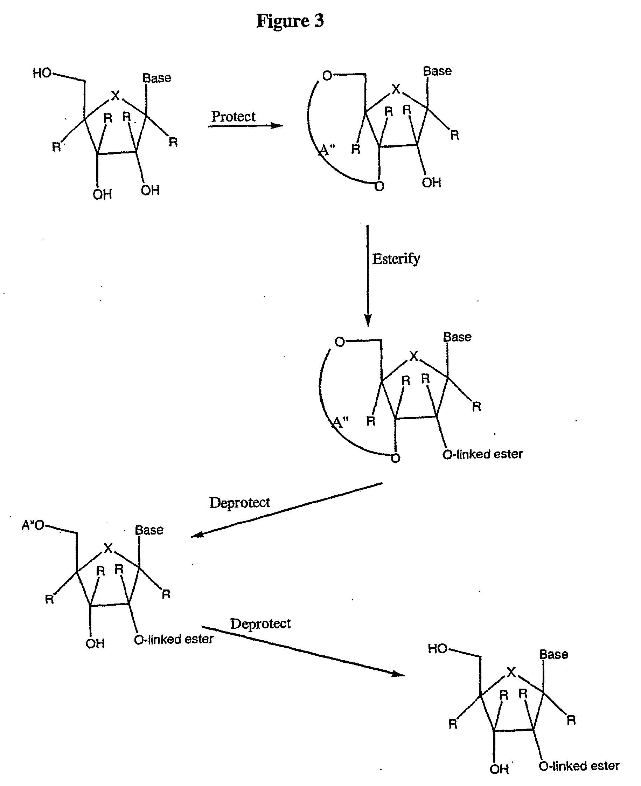

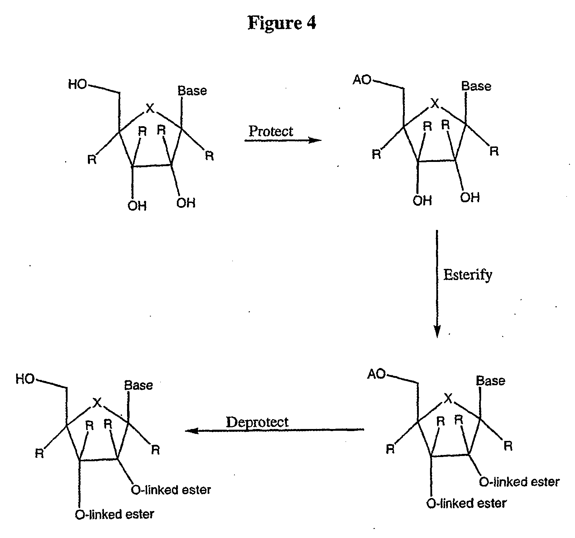

For example, simple amino-alcohols can be esterified using acid chlorides in refluxing acetonitrile-benzene mixture (See **Scheme 7** below: *Synthetic Communications,* **1978,** *8*(5), 327-333; hereby incorporated by reference). Alternatively, esterification can be achieved using an anhydride, as described in J. Am. Chem. Soc., 1999, 121(24), 5661-5664, which is hereby incorportated by reference. See Figures 2, 3 and 4. 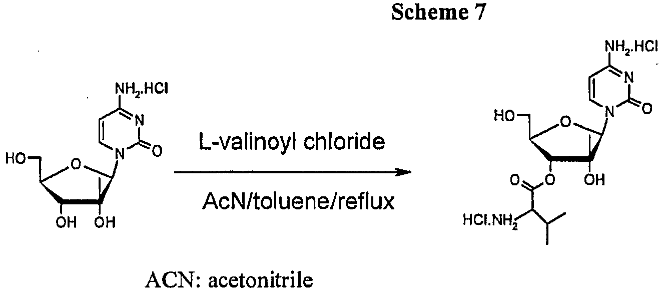 The present invention is described by way of illustration, in the following examples.

### REFERENCE EXAMPLE 1: PREPARATION OF 1'-C-METHYLRIBOADENINE VIA 6-AMINO-9-(1-DEOXY-β-D-PSICOFURANOSYL)PURINE

Melting points were determined on a Mel-temp II apparatus and are uncorrected. NMR spectra were recorded on a Bruker 400 AMX spectrometer at 400 MHz for ¹H NMR and 100 MHz for ¹³C NMR with TMS as internal standard. Chemical shifts (δ) are reported in parts per million (ppm), and signals are reported as s (singlet), d (doublet), t (triplet), q (quartet), m (multiplet), or bs (broad singlet). IR spectra were measured on a Nicolet 510P FT-IR spectrometer. Mass spectra were recorded on a Micromass Autospec high-resolution mass spectrometer. TLC were performed on Uniplates (silica gel) purchased from Analtech Co. Column chromatography was performed using either silica gel-60 (220-440 mesh) for flash chromatography or silica gel G (TLC grade, > 440 mesh) for vacuum flash column chromatography. UV spectra were obtained on a Beckman DU 650 spectrophotometer. Elemental analysis was performed by Atlantic Microlab, Inc., Norcross, GA, or Galbraith Laboratories, Inc., Knoxville, TN. HPLC was performed with a Waters HPLC system (Millipore Corporation, Milford, MA) equipped with a Model 600 controller, a Model 996 photodiode array detector and a Model 717 plus autosampler. Millennium 2010 software was used for system control, data acquisition and processing. A chiralyser polarimetric detector, Perkin-Elmer Model 241MC polarimeter (Wilton, CT), was used for the determination of optical rotations.

### Synthesis of 1'-C-methylribo-8-methyladenine

The title compound could also be prepared according to a published procedure (J. Farkas, and F. Sorm, "Nucleic acid components and their analogues. XCIV. Synthesis of 6-amino-9-(1-deoxy-β-D-psicofuranosyl)purine" Collect. Czech. Chem. Commits. 1967, 32, 2663-2667; J. Farkas", Collect. Czech. Chem. Commun. 1966, 31, 1535) **(Scheme 8).**

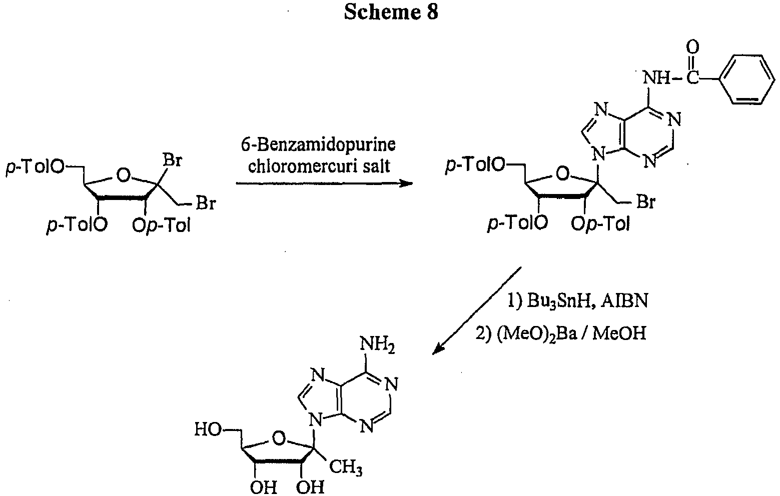

### EXAMPLE 2: PREPARATION OF 2'-C-METHYLRIBO-8-METHYLADENINE

The title compound was prepared according to a published procedure (R.E. Harry-O'kuru, J.M. Smith, and M.S. Wolfe, "A short, flexible route toward 2'-C-branched ribonucleosides", J. Org. Chem. 1997, 62, 1754-1759) (**Scheme 9**). (Reference Example)

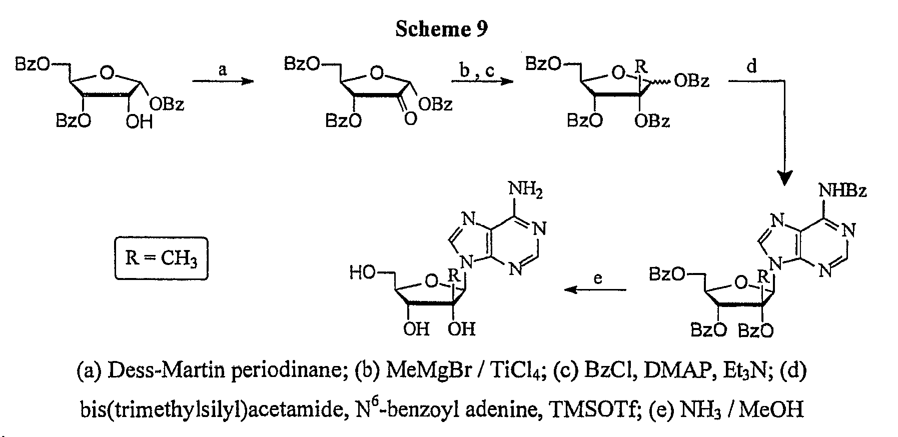

The 3'-prodrug of the 2'-branched nucleoside was prepared according to published procedure, *(*Synthetic Communications, 1978, 8(5), 327-333; J. Am. Chem. Soc., 1999, 121(24), 5661-5664). Alternatively, the 2'-branched nucleoside can be esterified without protection **(Scheme 9b).** Carbonyldiimidazole (377 mg, 2.33 mmol) was added to a solution of N-(tert-butoxycarbonyl)-L-valine (507 mg, 2.33 mmol) in 15 mL of anhydrous tetrahydrofuran. The mixture was stirred at 20 °C for one hour and at 50 °C for 10 minutes and then added to a solution of 4-Amino-1-(3,4-dihydroxy-5-hydroxymethyl-3-methyl-tetrahydro-furan-2-yl)-*1H*-pyrimidine-2-one (500 mg, 1.95 mmol), 4-(dimethylamino)pyridine (25 mg, 0.195 mmol), triethylamine (5 mL) in anhydrous N,N-dimethylformamide (10 mL), which is also stirring at 50 °C. The reaction mixture was stirred at 50 °C for one hour and then examined by HPLC. HPLC analysis indicated the formation of 52% of the desired ester, 17% of starting material in addition to undesired byproducts. The 3'-OH of 4-amino-1-(3,4-dihydroxy-5-hydroxymethyl-3-methyl-tetrahydrofuran-2-yl)-*1H*-pyrimidine-2-one tends to react selectively when coupled with BOC-Val.

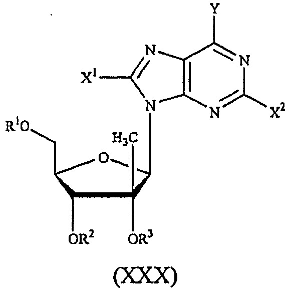

### REFERENCE EXAMPLE 3: PREPARATION OF 3'-C-METHYLRIBO-8-METHYLADENINE

The title compound can be prepared according to a published procedure (R.F. Nutt, M.J. Dickinson, F.W. Holly, and E. Walton, "Branched-chain sugar nucleosides. III. 3'-C-methyladenine ", J.Org. Chem. 1968, 33, 1789-1795) **(Scheme 10).**

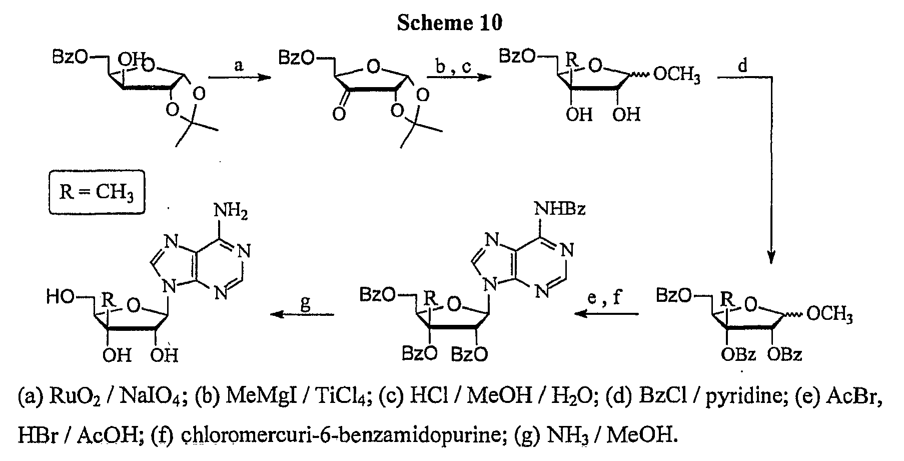

### REFERENCE EXAMPLE 4: PREPARATION OF 1-O-METHYL-2,3-O-ISOPROPYLIDENE-β-D-RIBOFURANOSE - (1)

The title compound can be prepared according to a published procedure (Leonard, N. J.; Carraway, K. L. "5-Amino-5-deoxyribose derivatives. Synthesis and use in the preparation of "reversed" nucleosides" J. Heterocycl. Chem. 1966, 3, 485-489).

A solution of 50.0 g (0.34 mole) of dry D-ribose in 1.0 L of acetone, 100 mL of 2,2-dimethoxypropane, 200 mL of methanol containing 20 mL of methanol saturated with hydrogen chloride at 0°C was stirred overnight at room temperature. The resulting solution was neutralized with pyridine and evaporated under reduced pressure. The resulting oil was partitioned between 400 mL of water and 400 mL of methylene chloride. The water layer was extracted twice with methylene chloride (400 mL). The combined organic extracts were dried over sodium sulfate and evaporated under reduced pressure. The residue was purified by silica gel column chromatography [eluent: stepwise gradient of methanol (1-2%) in methylene chloride] to give pure 1 (52.1 g, 75%) as a yellow syrup. ¹H-NMR (CDCl₃): δ 5.00 (s, 1H, H-1), 4.86 (d, 1H, H-2, J₂₋₃ = 5.9 Hz), 4.61 (d, 1H, H-3, J₃₋₂ = 5.9 Hz), 4.46 (t, 1H, H-4, J₄₋₅ = 2.7 Hz), 3.77-3.61 (m, 2H, H-5 and H-5'), 3.46 (s, 1H, OCH₃), 3.0-2.4 (br s, 1H, OH-5), 1.51 (s, 3H CH₃), 1.34 (s, 3H CH₃); MS (matrix GT): FAB>0 *m*/*z* 173 (M-OCH3)⁺.

### REFERENCE EXAMPLE 5: PREPARATION OF 1-O-METHYL-2,3-O-ISOPROPYLIDENE-β-D-PENTODIALDO-RIBOFURANOSE - (2)

The title compound can be prepared according to a published procedure (Jones, G. H.; Moffatt, J. G. Oxidation of carbohydrates by the sulfoxide-carbodiimide and related methods. Oxidation with dicyclohexylcarbodiimide-DMSO, diisopropylcarbodiimide-DMSO, acetic anhydride-DMSO, and phosphorus pentoxide-DMSO: in Methods in Carbohydrate Chemistry; Whisler, R. L. and Moffatt, J. L. Eds; Academic Press: New York, 1972; 315-322).

Compound **1** was co-evaporated twice with anhydrous pyridine. Dicyclohexylcarbodi-imide (DCC, 137.8 g, 0.67 mol) was added to a solution of **1** (68.2 g, 0.33 mole) in anhydrous benzene (670 mL), DMSO (500 mL) and pyridine (13.4 mL). To the resulting solution, cooled to 0°C, was added a solution of anhydrous crystalline orthophosphoric acid (16.4 g, 0.167 mmol) in anhydrous DMSO (30 mL). The mixture was stirred for 1.5 hours at 0°C and 18 hours at room temperature under argon atmosphere, diluted with ethyl acetate (1000 mL). A solution of oxalic acid dihydrate (63.1 g, 038 mol) in DMSO (30 mL) was added and the reaction mixture was stirred at room temperature during 1 hour and then filtered to eliminate precipitated dicyclohexylurea (DCU). The filtrate was concentrated to a volume of about 600 mL under reduced pressure and neutralized with a saturated aqueous sodium hydrogen carbonate solution (400 mL). Brine (200 mL) was added and the organic layer was extracted with ethyl acetate (4x 1000 mL). The combined organic layers were concentrated to a volume of about 2000 mL, washed with a saturated aqueous sodium hydrogen carbonate solution (2x 700 mL), and with brine (2x 700 mL) before being dried over sodium sulfate and evaporated under reduced pressure. A small fraction of the crude residue was purified on silica gel chromatography [eluent: chloroform/ethyl ether, 8:2] in order to confirm the structure of 2 which was obtained as a pale yellow solid. ¹H-NMR (CDCl₃): δ 9.61 (s, 1H, H-5), 5.12 (s, 1H, H-1), 5.08 (d, 1H, H-2, J₂₋₃ = 5.9 Hz), 4.53 (d, 1H, H-3, J₃₋₂ = 6.0 Hz), 4.51 (s, 1H, H-4), 3.48 (s, 1H, OCH₃), 1.56 (s, 3H CH₃), 1.36 (s, 3H CH₃); MS (matrix GT): FAB>0 *m*/*z* 203 (M+H)⁺, 171 (M-OCH₃)⁺.

### REFERENCE EXAMPLE 6: PREPARATION OF 4-C-HYDROXYMETHYL-1-O-METHYL-2,3-O-ISOPROPYLIDENE-β-D-RIBOFURANOSE - (3)

The title compound can be prepared according to a published procedure (Leland, D. L.; Kotick, M. P. "Studies on 4-C-(hydroxymethyl)pentofuranoses. Synthesis of 9-[4-C-(hydroxymethyl)-a-L-threo-pentofuranosyl]adenine" Carbohydr. Res. 1974, 38, C9-C11; Jones, G. H.; Taniguchi, M.; Tegg, D.; Moffatt, J. G. "4'-substituted nucleosides. 5. Hydroxylation of nucleoside 5'-aldehydes" J. Org. Chem. 1979, 44, 1309-1317; Gunic, E.; Girardet, J.-L.; Pietrzkowski, Z.; Esler, C.; Wang, G. "Synthesis and cytotoxicity of 4'-C- and 5'-C-substituted Toyocamycins" Bioorg. Med. Chem. 2001, 9, 163-170).

To a solution of the crude material **(2)** obtained above and 37% aqueous formaldehyde (167 mL) in dioxane (830 mL) was added aqueous sodium hydroxyde (2N, 300 mL). The mixture was stirred at room temperature for 4 hours and neutralized by addition of Dowex 50 W X 2 (H⁺ form). The resin was filtered, washed with methanol, and the combined filtrates were concentrated to dryness and coevaporated several times with absolute ethanol. Sodium formate which was precipitated from absolute ethanol was removed by filtration, the filtrate was concentrated to dryness and the residue was purified by silica gel column chromatography [eluent: stepwise gradient of methanol (0-4%) in chloroform] to give pure 3 (42.2 g, 54% from 1), which was recrystallized from cyclohexane. Mp = 94-95 (dec.) (lit.94-96.5; 97-98 : Refs :3,4), ¹H-NMR (DMSO-d₆): δ 4.65 (s, 1H, H-1), 4.44-4.37 (m, 3H, H-2, H-3 and OH-6), 4.27 (t, 1H, OH-5, J = 5.6 Hz, J = 6.0 Hz), 3.42-3.34 (m, 2H, H-5 and H-6) 3.29 (dd, 1H, H-5', J_{5'-OH} = 5.4 Hz, J5-5' = 11.4 Hz), 3.11 (dd, 1H, H-6', J_{6'-OH} = 5.7 Hz, J6-6' = 10.9 Hz), 3.03 (s, 3H, OCH₃), 1.48 (s, 3H CH₃), 1.05 (s, 3H CH₃); MS (matrix GT): FAB>0 *m*/*z* 469 (2M+H)⁺, 235 (M+H)⁺, 203 (M-OCH₃)+ FAB<0 *m*/*z* 233 (M-H)⁻.

### REFERENCE EXAMPLE 7: PREPARATION OF 6-O-MONOMETHOXYTRITYL-4-C-HYDROXYMETHYL-1-O-METHYL-2,3-O-ISOPROPYLIDENE-β-D-RIBOFURANOSE - (4)

The title compound can be prepared according to a published procedure (Gunic, E.; Girardet, J.-L.; Pietrzkowski, Z.; Esler, C.; Wang, G. "Synthesis and cytotoxicity of 4'-C-and 5'-C-substituted Toyocamycins" Bioorg. Med. Chem. 2001, 9, 163-170).

To a solution of 3 (41.0 g, 175 mmol) in pyridine (700 ml) was added by portions dimethoxytrityl chloride (60.5 g, 178 mmol) at +4°C. The reaction mixture was stirred for 3 hours at room temperature. After addition of methanol, the reaction mixture was concentrated (200 ml) and then dissolved with ethyl acetate (2 L). The organic layer was washed with a 5% aqueous sodium hydrogen carbonate solution, with water and dried over sodium sulfate and then evaporated to dryness. Purification by silica gel column chromatography [eluent: ethyl acetate / hexane 15/85] afforded pure **4** (63.0 g, 68%) as a syrup. ¹H-NMR (CDCl₃): δ 7.5-6.9 (m, 13H, MMTr), 4.89 (s, 1H, H-1), 4.72-4.62 (m, 3H, H-2, H-3 and OH-5), 3.82 (dd, 1H, H-5, J_{5-OH} = 5.5 Hz, J5-5' = 10.5 Hz), 3.79 (s, 6H, OCH3), 3.54 (dd, 1H, H-5', J_{5'-OH} = 4.9 Hz, J_{5'-5} = 10.5 Hz), 3.31 (s, 3H, OCH₃), 3.24 (d, 1H, H-6, J_{6-6'} = 9.2 Hz), 3.13 (d, 1H, H-6', J_{6'-6} = 9.2 Hz), 1.24 (s, 3H CH₃), 1.15 (s, 3H CH₃); MS (matrix GT): FAB>0 m/z 303 (DMTr)⁺.

### REFERENCE EXAMPLE 8: PREPARATION OF 5-O-BENZOYL-4-C-HYDROXYMETHYL-1-O-METHYL-2,3-O-ISOPROPYLIDENE-β-D-RIBO-FURANOSE - (5)

The title compound can be prepared according to a published procedure (Gunic, E.; Girardet, J.-L.; Pietrzkowski, Z.; Esler, C.; Wang, G. "Synthesis and cytotoxicity of 4'-C- and 5'-C-substituted Toyocamycins" Bioorg. Med. Chem. 2001, 9, 163-170).

To a solution of **4** (2.51 g, 4.68 mmol) in anhydrous pyridine (37 mL) was added under argon benzoyl chloride (1.09 mL, 9.36 mmol) and the reaction mixture was stirred for 13 hours at to room temperature. Then the reaction was cooled to 0°C and stopped with ice-cold water (100 mL). The water layer was extracted with methylene chloride (3□ 200 mL). The combined organic layers were washed with a saturated aqueous sodium hydrogen carbonate solution (2x 150 mL), with water (1x 150 mL) and then dried over sodium sulfate and evaporated under reduced pressure. The residue was dissolved in 80% acetic acid (70.2 mL) and the mixture was stirred at room temperature for 3hr and concentrated to dryness. Purification by silica gel column chromatography [eluent: chloroform] afforded pure 5 (1.40 g, 88%) as a syrup. ¹H-NMR (CDCl₃): δ 8.1-7.4 (m, 5H, C₆H₅CO), 5.08 (s, 1H, H-1), 4.77 (dd, 2H, H-2 and H-3, J = 6.1 Hz, J = 8.2 Hz), 4.51 (q, 2H, H-5 and H-5', J =11.5 Hz, J_{5-5'} = 23.8 Hz), 3.91 (t, 2H, H-6 and H-6', J = 12.3 Hz), 4.38 (s, 1H, OCH₃), 2.2-1.8 (brs, 1H, OH-6), 1.57 (s, 3H CH₃), 1.38 (s, 3H CH₃); MS (matrix GT): FAB>0 *m*/*z* 677 (2M+H)⁺, 339 (M+H)⁺, 307 (M-OCH₃)⁺, 105 (C₆H₅CO)⁺ FAB<0 *m*/*z* 121 (C₆H₅CO₂)⁻.

### REFERENCE EXAMPLE 9: PREPARATION OF 5-O-BENZOYL-4-C-METHYL-1-O-METHYL-2,3-O-ISOPROPYLIDENE-β-D-RIBOFURANOSE - (6)

The title compound can be prepared according to a published procedure (Gunic, E.; Girardet, J.-L.; Pietrzkowski, Z.; Esler, C.; Wang, G. "Synthesis and cytotoxicity of 4'-C- and 5'-C-substituted Toyocamycins" Bioorg. Med. Chem. 2001, 9, 163-170).

A solution of **5** (37.6 g, 0.111 mol), 4-dimethylaminopyridine (DMAP, 40.7 g, 0.333 mol) and phenoxythiocarbonyle chloride in anhydrous acetonitrile (1000 mL) was stirred at room temperature for 1 hour and concentrated to dryness. The residue was dissolved in methylene chloride (500 mL) and successively washed with 0.2 M hydrochloric acid (2x 500 mL) and water (500 mL) before being dried over sodium sulfate, evaporated under reduced pressure and coevaporated several times with anhydrous toluene. The crude material was dissolved in anhydrous toluene (880 mL) and tris(trimethylsilyl)silane (TMSS, 42.9 mL, 0.139 mol), and 1,1'-azobis(cyclohexanecarbonitrile) (ACCN, 6.8 g, 27.8 mmol) were added. The reaction mixture was stirred under reflux for 45 minutes, cooled to room temperature and concentrated under reduced pressure. The resulting residue was purified by silica gel column chromatography [eluent: stepwise gradient of diethyl ether (5-20%) in petroleum ether] to give pure **6** (26.4 g, 74%) as a pale yellow syrup. ¹H-NMR (DMSO-d₆): δ 8.0-7.5 (m, 5H, C₆H₅CO), 4.85 (s, 1H, H-1), 4.63 (dd, 2H, H-2 and H-3, J = 6.1 Hz, J = 11.6 Hz), 4.24 (d, 1H, H-5, J_{5-5'} = 11.1 Hz), 4.10 (d, 1H, H-5', J_{5'-5} = 11.1 Hz), 3.17 (s, 1H, OCH₃), 1.38 (s, 3H CH₃), 1.30 (s, 3H CH₃), 1.25 (s, 3H CH₃); MS (matrix GT): FAB>0 *m*/*z* 291 (M-OCH₃)⁺, 105 (C₆H₅CO)⁺ FAB<0 *m*/*z* 121 (C₆H₅CO₂)⁻.

### REFERENCE EXAMPLE 10: PREPARATION OF 5-O-BENZOYL-4-C-METAYL-1,2,3-O-ACETYL-α,β-D-RIBOFURANOSE - (7)

Compound **6** (22.5 g, 70 mmol) was suspended in a 80% aqueous acetic acid solution (250 mL). The solution was heated at 100°C for 3 hours. The volume was then reduced by half and coevaporated with absolute ethanol and pyridine. The oily residue was dissolved in pyridine (280 mL) and then cooled at 0°C. Acetic anhydride (80 mL) and 4-dimethylamino-pyridine (500 mg) were added. The reaction mixture was stirred at room temperature for 3 hours and then concentrated under reduced pressure. The residue was dissolved with ethyl acetate (1 L) and successively washed with a saturated aqueous sodium hydrogen carbonate solution, a 1 M hydrochloric acid and water. The organic layer was dried over sodium sulfate and evaporated under reduced pressure. The resulting residue was purified by silica gel column chromatography [eluent: stepwise gradient of diethyl ether (30-40%) in petroleum ether] to give pure 7 (16.2 g, 60%) as a pale yellow syrup. A small fraction of the material was re-purified on silica gel chromatography [same eluent: system] in order separate the α and the β anomers.
α anomer: ¹H-NMR (DMSO-d₆): δ 8.1-7.5 (m, 5H, C₆H₅CO), 6.34 (pt, 1H, H-1, J = 2.4 Hz, J = 2,1 Hz), 5.49 (m, 2H, H-2 and H-3), 4.33 (q, 2H, H-5 and H-5', J = 11.6 Hz, J = 18.7 Hz), 2.15 (s, 3H, CH₃CO₂), 2.11 (s, 3H, CH₃CO₂), 2.07 (s, 3H, CH₃CO₂), 1.37 (s, 3H, CH₃); MS (matrix GT): FAB>0 *m*/*z* 335 (M-CH₃CO₂⁻)⁺, 275 (M-CH₃CO₂⁻+H)⁺,105 (C₆H₅CO)⁺, 43 (CH₃CO)⁺ FAB<0 *m*/*z* 121 (C₆H₅CO₂)⁻, 59 (CH₃CO₂)⁻.
β anomer: ¹H-NMR (DMSO-d₆): δ 8.1-7.5 (m, 5H, C₆H₅CO), 5.99 (s, 1H, H-1), 5.46 (d, 1H, H-2, J₂₋₃ = 5.3 HZ), 5.30 (d, 1H, H-2, J₂₋₃ = 5.3 Hz), 4.39 (d, 1H, H-5, J_{5-5'} = 11.7 Hz), 4.19 (d, 1H, H-5', J_{5'-5} = 11.7 Hz), 2.10 (s, 3H, CH₃CO₂), 2.06 (s, 3H, CH₃CO₂), 2.02 (s, 3H, CH₃CO₂), 1.30 (s, 3H, CH₃); MS (matrix GT): FAB>0 *m*/*z* 335 (M-CH₃CO₂)⁺, 275 (M-CH₃CO2⁻+H)⁺, 105 (C₆H₅CO)⁺, 43 (CH₃CO)⁺ FAB<0 *m*/*z* 121 (C₆H₅CO₂)⁻, 59 (CH₃CO₂)⁻.

### REFERENCE EXAMPLE 11: PREPARATION OF O-6-DIPHENYLCARBAMOYL-N²-ISOBUTYRYL-9-(2,3-DI-O-ACETYL-5-O-BENZOYL-4-C-METHYL-β-D-RIBOFURANOSYL)-8-METHYLGUANINE - (18)

To a suspension of O-6-diphenylcarbamoyl-8-methyl-N²-isobutyrylguanine in anhydrous toluene (20 mL) was added N,O-bis(trimethylsilyl)acetamide (1.92 mL, 7.9 mmol). The reaction mixture was allowed to warm under reflux for 1 hour. Compound 7 (1.55 g, 3.93 mmol) was dissolved in toluene (10 mL) and trimethylsilyltrifluoro-methanesulfonate (TMSTf) (915 mL, 4.72 mmol) was added. The mixture was heated under reflux for 30 minutes. The solution was then cooled to room temperature and neutralized with a 5% aqueous sodium hydrogen carbonate solution. The reaction mixture was diluted with ethyl acetate (200 mL). The organic phase was washed with a 5% aqueous sodium hydrogen carbonate solution (150 mL) and with water (2x 150 mL). The organic layer was dried over Na₂SO₄ and evaporated to dryness. The residue was purified by silica gel column chromatography [eluent: stepwise gradient of diethyl ether (70-90%) in petroleum ether] to afford 18.

### REFERENCE EXAMPLE 12: PREPARATION OF 9-(4-C-METHYL-β-D-RIBOFURANOSYL)-8-METHYLGUANINE - (19)

The title compound can be prepared according to a published procedure from **18** (Waga, T.; Nishizaki, T.; Miyakawa, I.; Orhui, H.; Meguro, H. "Synthesis of 4'-C-methylnucleosides" Biosci. Biotechnol. Biochem. 1993, 57, 1433-1438).

A solution of **18** in methanolic ammonia (previously saturated at -10°C) (20 mL) was stirred at room temperature overnight. The solvent was evaporated under reduced pressure and the residue was partitioned between methylene chloride (60 mL) and water (60 mL). The aqueous layer was washed with methylene chloride (2x 60 mL), concentrated under reduced pressure. The residue was purified by an RP18 column chromatography [eluent water/acetonitrile 95/5] to afford **19.**

### REFERENCE EXAMPLE 13: 9-(2,3-DI-O-ACETYL-5-O-BENZOYL-4-C-METHYL-β-D-RIBOFURANOSYL)-8-METHYLADEMNE - (20)

A solution of 7 (1.10 g, 2.79 mmol) in anhydrous acetonitrile (50 ml) was treated with 8-methyladenine and stannic chloride (SnCl₄, 660 µL, 5.58 mmol) and stirred at room temperature overnight. The solution was concentrated under reduced pressure, diluted with chloroform (100 mL) and treated with a cold saturated aqueous solution of NaHCO₃ (100 ml). The mixture was filtered on celite, and the precipitate was washed with hot chloroform. The filtrates were combined, washed with water (100 ml) and brine (100 ml), dried (Na₂SO₄), and evaporated under reduced pressure. The residue was purified by silica gel column chromatography [eluent: stepwise gradient of methanol (3-5%) in dichloromethane] to afford **20.**

### REFERENCE EXAMPLE 14: PREPARATION OF 9-(4-C-METHYL-β-D-RIBOFURANOSYL)-8-METHYLADENINE - (21)

The title compound can be prepared according to a published procedure from 20 (Waga, T.; Nishizaki, T.; Miyakawa, I.; Orhui, H.; Meguro, H. "Synthesis of 4'-C-methylnucleosides" Biosci. Biotechnol. Biochem. 1993, 57, 1433-1438).

A solution of 20 in methanolic ammonia (previously saturated at -10°C) (50 mL) was stirred at room temperature overnight. The solvent was evaporated under reduced pressure and the residue was partitioned between methylene chloride (100 ml) and water (100 ml). The aqueous layer was washed with methylene chloride (2x 100 mL), and concentrated under reduced pressure. The residue was purified by silica gel column chromatography [eluent: stepwise gradient of methanol (10-30%) in ethyl acetate] to afford 21.

### REFERENCE EXAMPLE 15: PREPARATION OF 1-(5-O-BENZOYL-4-C-METHYL-2,3-O-ACETYL-β-D-RIBOFURANOSYL)-6-METHYLURACIL- (8)

A suspension of 6-methyluracil was treated with hexamethyldisilazane (HMDS, 21 mL) and a catalytic amount of ammonium sulfate during 17 hours under reflux. After cooling to room temperature, the mixture was evaporated under reduced pressure, and the residue, obtained as a colorless oil, was diluted with anhydrous 1,2-dichloroethane (7.5 mL). To the resulting solution was added 7 (0.99 g, 2.51 mmol) in anhydrous 1,2-dichloroethane (14 mL), followed by addition of trimethylsilyl trifluoromethanesulfonate (TMSTf, 0.97 mL, 5.02 mmol). The solution was stirred for 2.5 hours at room temperature under argon atmosphere, then diluted with chloroform (150 mL), washed with the same volume of a saturated aqueous sodium hydrogen carbonate solution and finally with water (2x 100 mL). The organic phase was dried over sodium sulfate, then evaporated under reduced pressure. The resulting crude material was purified by silica gel column chromatography [eluent: stepwise gradient of methanol (0-2%) in chloroform] to afford pure **8**.

### REFERENCE EXAMPLE 16: PREPARATION OF 1-(4-C-METHYL-β-D-RIBOFURANOSYL)-6-METHYLURACIL - (9)

The title compound can be prepared according to a published procedure from **8** (Waga, T.; Nishizaki, T.; Miyakawa, I.; Orhui, H.; Meguro, H. "Synthesis of 4'-C-methylnucleosides" Biosci. Biotechnol. Biochem. 1993, 57, 1433-1438).

A solution of **8** in methanolic ammonia (previously saturated at -10°C) (27 mL) was stirred at room temperature overnight. The solvent was evaporated under reduced pressure and the residue was partitioned between methylene chloride (40 mL) and water (40 mL). The aqueous layer was washed with methylene chloride (2x 40 mL), concentrated under reduced pressure and coevaporated several times with absolute ethanol. Recrystallization from a mixture absolute ethanol/methanol gave **9.**

### REFERENCE EXAMPLE 17: PREPARATION OF 1-(5-O-BENZOYL-4-C-METHYL-2,3-O-ACETYL-β-D-RIBOFURANOSYL)-4-THIO-6-METHYL-URACIL - (10)

Lawesson's reagent (926 mg, 2.29 mmol) was added under argon to a solution of **8** in anhydrous 1,2-dichloroethane (65 mL) and the reaction mixture was stirred overnight under reflux. The solvent was evaporated under reduced pressure and the residue was purified by silica gel column chromatography [eluent: stepwise gradient of methanol (1-2%) in chloroform] to give pure **10.**

### REFERENCE EXAMPLE 18: PREPARATION OF 1-(4-C-METHYL-β-D-RIBOFURANOSYL)-4-THIO-6-METHYLURACIL - (11)

A solution of **10** in methanolic ammonia (previously saturated at -10°C) (27 mL) was stirred at room temperature overnight. The solvent was evaporated under reduced pressure and the residue was partitioned between methylene chloride (40 ml) and water (40 mL). The aqueous layer was washed with methylene chloride (2x 40 mL), concentrated under reduced pressure. The crude material was purified by silica gel column chromatography [eluent: stepwise gradient of methanol (5-7%) in methylene chloride] to give 11, which was lyophilized.

### REFERENCE EXAMPLE 19: PREPARATION OF 1-(4-C-METHYL-β-D-RIBOFURANOSYL)-6-METHYLCYTOSINE, HYDROCHLORIC FORM - (12)

Compound **11** was treated with methanolic ammonia (previously saturated at - 10°C), (12 mL) at 100°C in a stainless-steel bomb for 3 hours, then cooled to room temperature. The solvent was evaporated under reduced pressure and the residue was partitioned between methylene chloride (40 mL) and water (40 mL). The aqueous layer was washed with methylene chloride (2x 40 mL), concentrated under reduced pressure. The crude material was purified by silica gel column chromatography [eluent: methylene chloride/ methanol/ammonium hydroxide 65:30:5]. The collected fractions were evaporated under reduced pressure and in absolute ethanol (6.3 mL). To the solution was added a 2N hydrochloric acid solution (1.5 mL) and the mixture was stirred before being concentrated under reduced pressure. The procedure was repeated twice and **12** was precipitated from absolute ethanol.

### REFERENCE EXAMPLE 20: PREPARATION OF 1-(5-O-BENZOYL-4-C-METHYL-2,3-O-ACETYL-β-D-RIBOFURANOSYL)-6-METHYLTHYMINE - (13)

A suspension of 6-methylthymine was treated with hexamethyldisilazane (HMDS, 17 mL) and a catalytic amount of ammonium sulfate overnight under reflux. After cooling to room temperature, the mixture was evaporated under reduced pressure, and the residue, obtained as a colorless oil, was diluted with anhydrous 1,2-dichloroethane (6 mL). To the resulting solution was added 7 (1.0 g, 2.53 mmol) in anhydrous 1,2-dichloroethane (14 mL), followed by addition of trimethylsilyl trifluoromethanesulfonate (TMSTf, 0.98 mL, 5.06 mmol). The solution was stirred for 5 hours at room temperature under argon atmosphere, then diluted with chloroform (150 mL), washed with the same volume of a saturated aqueous sodium hydrogen carbonate solution and finally with water (2x 100 mL). The organic phase was dried over sodium sulfate, then evaporated under reduced pressure. The resulting crude material was purified by silica gel column chromatography [eluent: 2% of methanol in chloroform] to afford pure **13.**

### REFERENCE EXAMPLE 21: PREPARATION OF 1-(4-C-METHYL-β-D-RIBOFURANOSYL)-6-METHYLTHYMINE - (14)

The title compound can be prepared according to a published procedure from **13** (Waga, T.; Nishizaki, T.; Miyakawa, I.; Orhui, H.; Meguro, H. "Synthesis of 4'-C-methylnucleosides" Biosci. Biotechnol. Biochem. 1993, 57, 1433-1438).

A solution of 13 in methanolic ammonia (previously saturated at -10°C) (60 mL) was stirred at room temperature overnight. The solvent was evaporated under reduced pressure and the residue was partitioned between methylene chloride (60 mL) and water (60 mL). The aqueous layer was washed with methylene chloride (2x 60 mL), concentrated under reduced pressure and coevaporated several times with absolute ethanol. Recrystallization from methanol gave 14.

### REFERENCE EXAMPLE 22: PREPARATION OF 1-(5,2,3-TRI-O-ACETYL-4-C-METHYL-β-D-RIBOFURANOSYL)-6-METHYLTHYMINE - (15)

A solution of 14 in anhydrous pyridine (7.4 mL) was treated with acetic anhydride (1.2 mL) and stirred at room temperature for 3 hours. The solvent was evaporated under reduced pressure, and the residue was purified by silica gel column chromatography [eluent: stepwise gradient of methanol (0-5%) in methylene chloride] to afford 15.

### REFERENCE EXAMPLE 23: PREPARATION OF 1-(5,2,3-TRI-O-ACETYL-4-C-METHYL-β-D-RIBOFURANOSYL)-4-THIO-6-METHYLTHYMINE - (16)

Lawesson's reagent (119 mg, 0.29 mmol) was added under argon to a solution of **15** in anhydrous 1,2-dichloroethane (11 mL) and the reaction mixture was stirred overnight under reflux. The solvent was evaporated under reduced pressure and the residue was purified by silica gel column chromatography [eluent: stepwise gradient of methanol (1-2%) in chloroform] to give **16.**

### EXAMPLE 24: PREPARATION OF 1-(4-C-METHYL-β-D-RIBOFURANOSYL)-5-METHYL-6-METHYLCYTOSINE - (17), HYDROCHLORIDE FORM (Reference Example)

Compound **16** was treated with methanolic ammonia (previously saturated at - 10°C), (10 mL) at 100°C in a stainless-steel bomb for 3 hours, then cooled to room temperature. The solvent was evaporated under reduced pressure and the residue was partitioned between methylene chloride (30 mL) and water (30 mL). The aqueous layer was washed with methylene chloride (2x 30 mL), concentrated under reduced pressure. The crude material was purified by silica gel column chromatography [eluent: 20% methanol in methylene chloride] to afford **17.** This compound was dissolved in EtOH 100 (1.5 mL), treated with a 2N hydrochloric acid solution (0.3 mL), and the mixture was stirred before being concentrated under reduced pressure. The procedure was repeated twice and 17 was precipitated from absolute ethanol.

Alternatively, the following nucleosides of Formula XXXXVII are prepared, using the appropriate sugar and pyrimidine or purine bases, wherein the substituents are as defined in the claims.

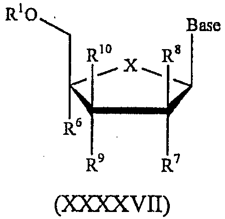

### VIII. Biological Assays

A number of assays are available to determine the potency of test compounds against viruses. Several of these biological assays are described in the examples below.

### EXAMPLE 25: ANTI-FLAVIVIRUS OR PESTIVIRUS ACTIVITY

Compounds can exhibit anti-flavivirus or pestivirus activity by inhibiting flavivirus or pestivirus polymerase, by inhibiting other enzymes needed in the replication cycle, or by other pathways.

### Phosphorylation Assay of Nucleoside to Active Triphosphate

To determine the cellular metabolism of the compounds, HepG2 cells are obtained from the American Type Culture Collection (Rockville, MD), and are grown in 225 cm² tissue culture flasks in minimal essential medium supplemented with non-essential amino acids, 1% penicillin-streptomycin. The medium is renewed every three days, and the cells are subcultured once a week. After detachment of the adherent monolayer with a 10 minute exposure to 30 mL of trypsin-EDTA and three consecutive washes with medium, confluent HepG2 cells are seeded at a density of 2.5 x 10⁶ cells per well in a 6-well plate and exposed to 10 µM of [³H] labeled active compound (500 dpm/pmol) for the specified time periods. The cells are maintained at 37°C under a 5% CO₂ atmosphere. At the selected time points, the cells are washed three times with ice-cold phosphate-buffered saline (PBS). Intracellular active compound and its respective metabolites are extracted by incubating the cell pellet overnight at -20°C with 60% methanol followed by extraction with an additional 20 µL of cold methanol for one hour in an ice bath. The extracts are then combined, dried under gentle filtered air flow and stored at -20°C until HPLC analysis.

### Bioavailability Assay in Cynomolgus Monkeys

Within 1 week prior to the study initiation, the cynomolgus monkey is surgically implanted with a chronic venous catheter and subcutaneous venous access port (VAP) to facilitate blood collection and underwent a physical examination including hematology and serum chemistry evaluations and the body weight was recorded. Each monkey (six total) receives approximately 250 µCi of ³H activity with each dose of active compound at a dose level of 10 mg/kg at a dose concentration of 5 mg/mL, either via an intravenous bolus (3 monkeys, IV), or via oral gavage (3 monkeys, PO). Each dosing syringe is weighed before dosing to gravimetrically determine the quantity of formulation administered. Urine samples are collected via pan catch at the designated intervals (approximately 18-0 hours pre-dose, 0-4, 4-8 and 8-12 hours post-dosage) and processed. Blood samples are collected as well (pre-dose, 0.25, 0.5, 1, 2, 3, 6, 8, 12 and 24 hours post-dosage) via the chronic venous catheter and VAP or from a peripheral vessel if the chronic venous catheter procedure should not be possible. The blood and urine samples are analyzed for the maximum concentration (Cₘₐₓ), time when the maximum concentration is achieved (Tₘₐₓ), area under the curve (AUC), half life of the dosage concentration (T_{½}), clearance (CL), steady state volume and distribution (Vₛₛ) and bioavailability (F).

### Bone Marrow Toxicity Assay

Human bone marrow cells are collected from normal healthy volunteers and the mononuclear population are separated by Ficoll-Hypaque gradient centrifugation as described previously by Sommadossi J-P, Carlisle R. "Toxicity of 3'-azido-3'-deoxythymidine and 9-(1,3-dihydroxy-2-propoxymethyl)guanine for normal human hematopoietic progenitor cells in vitro" Antimicrobial Agents and Chemotherapy 1987; 31:452-454; and Sommadossi J-P, Schinazi RF, Chu CK, Xie M-Y. "Comparison of cytotoxicity of the (-)- and (+)-enantiomer of 2',3'-dideoxy-3'-thiacytidine in normal human bone marrow progenitor cells" Biochemical Pharmacology 1992; 44:1921-1925. The culture assays for CFU-GM and BFU-E are performed using a bilayer soft agar or methylcellulose method. Drugs are diluted in tissue culture medium and filtered. After 14 to 18 days at 37°C in a humidified atmosphere of 5% CO₂ in air, colonies of greater than 50 cells are counted using an inverted microscope. The results are presented as the percent inhibition of colony formation in the presence of drug compared to solvent control cultures.

### Mitochondria Toxicity Assay

HepG2 cells are cultured in 12-well plates as described above and exposed to various concentrations of drugs as taught by Pan-Zhou X-R, Cui L, Zhou X-J, Sommadossi J-P, Darley-Usmer VM. "Differential effects of antiretroviral nucleoside analogs on mitochondrial function in HepG2 cells" Antimicrob. Agents Chemother. 2000; 44:496-503. Lactic acid levels in the culture medium after 4 day drug exposure are measured using a Boehringer lactic acid assay kit. Lactic acid levels are normalized by cell number as measured by hemocytometer count.

### Cytotoxicity Assay

Cells are seeded at a rate of between 5 x 10³ and 5 x 10⁴/well into 96-well plates in growth medium overnight at 37°C in a humidified CO₂ (5%) atmosphere. New growth medium containing serial dilutions of the drugs is then added. After incubation for 4 days, cultures are fixed in 50% TCA and stained with sulforhodamineB. The optical density was read at 550 nm. The cytotoxic concentration was expressed as the concentration required to reduce the cell number by 50% (CC₅₀).

### Cell Protection Assay (CPA)

The assay is performed essentially as described by Baginski, S. G.; Pevear, D. C.; Seipel, M.; Sun, S. C. C.; Benetatos, C. A.; Chunduru, S. K.; Rice, C. M. and M. S. Collett "Mechanism of action of a pestivirus antiviral compound" PNAS USA 2000, 97(14), 7981-7986. MDBK cells (ATCC) are seeded onto 96-well culture plates (4,000 cells per well) 24 hours before use. After infection with BVDV (strain NADL, ATCC) at a multiplicity of infection (MOI) of 0.02 plaque forming units (PFU) per cell, serial dilutions of test compounds are added to both infected and uninfected cells in a final concentration of 0.5% DMSO in growth medium. Each dilution is tested in quadruplicate. Cell densities and virus inocula are adjusted to ensure continuous cell growth throughout the experiment and to achieve more than 90% virus-induced cell destruction in the untreated controls after four days post-infection. After four days, plates are fixed with 50% TCA and stained with sulforhodamine B. The optical density of the wells is read in a microplate reader at 550 nm. The 50% effective concentration (EC₅₀) values are defined as the compound concentration that achieved 50% reduction of cytopathic effect of the virus.

### Plaque Reduction Assay

For each compound the effective concentration is determined in duplicate 24-well plates by plaque reduction assays. Cell monolayers are infected with 100 PFU/well of virus. Then, serial dilutions of test compounds in MEM supplemented with 2% inactivated serum and 0.75% of methyl cellulose are added to the monolayers. Cultures are further incubated at 37°C for 3 days, then fixed with 50% ethanol and 0.8% Crystal Violet, washed and air-dried. Then plaques are counted to determine the concentration to obtain 90% virus suppression.

### Yield Reduction Assay

For each compound the concentration to obtain a 6-log reduction in viral load is determined in duplicate 24-well plates by yield reduction assays. The assay is performed as described by Baginski, S. G.; Pevear, D. C.; Seipel, M.; Sun, S. C. C.; Benetatos, C. A.; Chunduru, S. K.; Rice, C. M. and M. S. Collett "Mechanism of action of a pestivirus antiviral compound" PNAS USA 2000, 97(14), 7981-7986, with minor modifications. Briefly, MDBK cells are seeded onto 24-well plates (2 x 105 cells per well) 24 hours before infection with BVDV (NADL strain) at a multiplicity of infection (MOI) of 0.1 PFU per cell. Serial dilutions of test compounds are added to cells in a final concentration of 0.5% DMSO in growth medium. Each dilution is tested in triplicate. After three days, cell cultures (cell monolayers and supernatants) are lysed by three freeze-thaw cycles, and virus yield is quantified by plaque assay. Briefly, MDBK cells are seeded onto 6-well plates (5 x 105 cells per well) 24 h before use. Cells are inoculated with 0.2 mL of test lysates for 1 hour, washed and overlaid with 0.5% agarose in growth medium. After 3 days, cell monolayers are fixed with 3.5% formaldehyde and stained with 1% crystal violet (w/v in 50% ethanol) to visualize plaques. The plaques are counted to determine the concentration to obtain a 6-log reduction in viral load.

### REFERENCE EXAMPLE 26: IN VITRO ANTI-VIRAL ACTIVITY

In vitro anti-viral activity was tested in the following cell lines: MT-4 for HIV; Vero 76, African green monkey kidney cells for SARS; BHK for Bovine Viral Diarrhea Virus; Sb-1 for poliovirus Sabin type-1; CVB-2, CVB-3, CVB-4, and CVA-9 for Coxsackieviruses B-2, B-3, B-4 and A-9; and REO-1 for double-stranded RNA viruses. Note: BVDV = bovine viral diarrhea virus; YFV = yellow fever virus; DENV = dengue virus; WNV = West Nile virus; CVB-2 = Coxsackie B-2 virus; Sb-1 = Sabin type 1 poliomyelitis virus; and REO = double-stranded RNA Reovirus.

### CC₅₀ and EC₅₀ Test Results for β-D-2'-C-methyl-7-methyl-6-phenyl-3,3a,5,8a-tetrahydro-1,3,4,5,7a-penta-aza-s-indacen-8-one (Compound F)

| | CC₅₀ | CC₅₀ | CC₅₀ | EC₅₀ | EC₅₀ | EC₅₀ | EC₅₀ | EC₅₀ | EC₅₀ |
|---|---|---|---|---|---|---|---|---|---|
| Compound | MT-4 | Vero 76 | BHK | Sb-1 | CVB-2 | CVB-3 | CVB-4 | CVA-9 | REO- 1 |
| F | >100 | >100 | >100 | 43 | 37 | 49 | 39 | 60 | 2 |

### CC₅₀ Test Results for β-D-2'-C-methyl-7-methyl-6-phenyl-3,3a,58a-tetrahydro-1,3,4,5,7a-penta-aza-s-indacen-8-one (Compound F)

| | CC₅₀ | | | | | | | |
|---|---|---|---|---|---|---|---|---|
| Compound | | BVDV | YFV | DENV 2 | WNV | CVB-2 | Sb-1 | REO |
| F | >100 | 10 | 2.5 | 1.3 | 1 | 37 | 43 | 2 |

## Claims

1. A compound of Formula (IX): 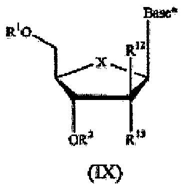 or a pharmaceutically acceptable salt thereof, wherein:
R¹ and R² are independently H; phosphate; straight chained, branched or cyclic alkyl; acyl; CO-alkyl; CO-aryl; CO-alkoxyalkyl; CO-aryloxyalkyl; CO-substituted aryl; sulfonate ester; benzyl, wherein the phenyl group is optionally substituted with one or more substituents; alkylsulfonyl; arylsulfonyl; aralkylsulfonyl; a lipid; an amino acid; a carbohydrate; a peptide; or a cholesterol;
X is O;
Base* is a purine or pyrimidine base;
R¹² is C(Y³)₃;
Y³ is H; and
R¹³ is fluoro.

2. The compound of claim 1, wherein R¹ and R² are H.

3. The compound of claim 1, wherein:
R¹ is H; phosphate; straight chained, branched or cyclic alkyl; acyl; CO-alkyl; CO-aryl; CO-alkoxyalkyl; CO-aryloxyalkyl; CO-substituted aryl; sulfonate ester; benzyl, wherein the phenyl group is optionally substituted with one or more substituents; alkylsulfonyl; arylsulfonyl; aralkylsulfonyl; a lipid; an amino acid; a carbohydrate; a peptide; or a cholesterol; and
R² is phosphate; straight chained, branched or cyclic alkyl; acyl; CO-alkyl; CO-aryl; CO-alkoxyalkyl; CO-aryloxyalkyl; CO-substituted aryl; sulfonate ester; benzyl, wherein the phenyl group is optionally substituted with one or more substituents; alkylsulfonyl; arylsulfonyl; aralkylsulfonyl; a lipid; an amino acid; a carbohydrate; a peptide; or a cholesterol.

4. The compound of claim 1, wherein:
R¹ is H; phosphate; straight chained, branched or cyclic alkyl; acyl; CO-alkyl; CO-aryl; CO-alkoxyalkyl; CO-aryloxyalkyl; CO-substituted aryl; sulfonate ester; benzyl, wherein the phenyl group is optionally substituted with one or more substituents; alkylsulfonyl; arylsulfonyl; aralkylsulfonyl; a lipid; an amino acid; a carbohydrate; a peptide; or a cholesterol;
R² is acyl; and
Base* is thymine, uracil, guanine, cytosine, adenine, hypoxanthine, 5-fluorouracil, or 5-fluorocytosine.

5. The compound of any one of claims 1-4, wherein Base* is cytosine, uracil, guanine, adenine, or thymine.

6. The compound of any one of claims 1-5, or a pharmaceutically acceptable salt thereof, for use in a method for the treatment of a host infected with a *Flaviviridae,* virus.

7. The compound for use of claim 6, wherein the virus is hepatitis C.

8. The compound for use of claim 6,
wherein the method comprises administration of the compound or pharmaceutically acceptable salt thereof in combination or alternation with a second anti-viral agent; or
wherein the method comprises administration of the compound or pharmaceutically acceptable salt thereof in combination or alternation with a second or more anti-viral agents.

9. The compound for use of claim 8, wherein the second anti-viral agent is selected from the group consisting of an interferon, a ribavirin, an interleukin, aNS3 protease inhibitor, a cysteine protease inhibitor, a phenan-threnequinone, a thiazolidine derivative, a thiazolidine, a benzanilide, a phenan-threnequinone, a helicase inhibitor, a polymerase inhibitor, a nucleotide analogue, a gliotoxin, a cerulenin, an antisense phosphorothioate oligodeoxynucleotide, an inhibitor of IRES-dependent translation, and a ribozyme.

10. The compound for use of claim 9, wherein the second anti-viral agent is an interferon.

11. The compound for use of claim 10, wherein the second anti-viral agent is selected from the group consisting of pegylated interferon alpha 2a, interferon alphacon-1, natural interferon, albuferon, interferon beta-1a, omega interferon, interferon alpha, interferon gamma, interferon tau, interferon delta and interferon gamma-1b.

12. The compound for use of claim 6, wherein the compound or pharmaceutically acceptable salt thereof is in the form of a dosage unit.

13. The compound for use of claim 12 wherein the dosage unit contains 50 to 1000 mg or 0.1 to 50 mg of the compound.

14. The compound for use of claim 12 wherein the dosage unit is a tablet or capsule.

15. The compound for use of claim 6, wherein the host is a human.

16. The compound for use of claim 6, wherein the compound or pharmaceutically acceptable salt thereof is in substantially pure form.

17. The compound for use of claim 6, wherein the compound or pharmaceutically acceptable salt thereof is at least 90% by weight of the β-D-isomer.

18. The compound for use of claim 6, wherein the compound or pharmaceutically acceptable salt thereof is at least 95% by weight of the β-D-isomer.

19. The compound for use of claim 6, wherein the compound is in the form of a pharmaceutically acceptable salt selected from the group consisting of a tosylate, methanesulfonate, acetate, citrate, malonate, tartrate, succinate, benzoate, ascorbate, α-ketoglutarate, α-glycerophosphate, formate, fumarate, propionate, glycolate, lactate, pyruvate, oxalate, maleate, salicylate, sulfate, nitrate, bicarbonate, carbonate salts, hydrobromate, hydrochloride, di-hydrochloride, and phosphoric acid salt.

20. The compound for use of claim 19, wherein the pharmaceutically acceptable salt is a hydrochloride salt.

21. A pharmaceutical composition comprising an effective amount to treat a *Flaviviridae* infection of a compound, or a pharmaceutically acceptable salt thereof, of any of claims 1 to 5 in a pharmaceutically acceptable carrier.

22. The pharmaceutical composition of claim 21 wherein the carrier is suitable for oral delivery.

23. The pharmaceutical composition of claim 21 comprising an effective amount of the compound or pharmaceutically acceptable salt thereof to treat a host infected with West Nile Virus, Yellow fever, Dengue Virus or BVDV.

24. The composition of claim 21, wherein the *Flaviviridae* virus is hepatitis C.

25. The pharmaceutical composition of claim 21, wherein the compound or pharmaceutically acceptable salt thereof, is in the form of a dosage unit.

26. The composition of claim 25, wherein the dosage unit contains 0.1 to 50 mg or 50 to 1000 mg of the compound or pharmaceutically acceptable salt thereof.

27. The composition of claim 25, wherein said dosage unit is a tablet or capsule.

28. The pharmaceutical composition of claim 21,
further comprising a second anti-viral agent; or
further comprising a second or more anti-viral agents.

29. The pharmaceutical composition of claim 28, wherein the second anti-viral agent is selected from the group consisting of an interferon, a ribavirin, an interleukin, a NS3 protease inhibitor, a cysteine protease inhibitor, a phenan-threnequinone, a thiazolidine derivative, a thiazolidine, a benzanilide, a phenan-threnequinone, a helicase inhibitor, a polymerase inhibitor, a nucleotide analogue, a gliotoxin, a cerulenin, an antisense phosphorothioate oligodeoxynucleotide, an inhibitor of IRES-dependent translation, and a ribozyme.

30. The pharmaceutical composition of claim 29, wherein the second anti-viral agent is an interferon.

31. The pharmaceutical composition of claim 30, wherein the second anti-viral agent is selected from the group consisting of pegylated interferon alpha 2a, interferon alphacon-1, natural interferon, albuferon, interferon beta-1a, omega interferon, interferon alpha, interferon gamma, interferon tau, interferon delta and interferon gamma-1b.

32. The pharmaceutical composition of claim 21, wherein the compound or pharmaceutically acceptable salt thereof, is in substantially pure form.

33. The pharmaceutical composition of claim 21, wherein the compound or pharmaceutically acceptable salt thereof, is at least 90% by weight of the β-D-isomer.

34. The pharmaceutical composition of claim 21, wherein the compound or pharmaceutically acceptable salt thereof, is at least 95% by weight of the β-D-isomer.

35. The pharmaceutical composition of claim 21 further comprising a pharmaceutically acceptable carrier suitable for oral, parenteral, inhalant or intravenous delivery.

36. The pharmaceutical composition of claim 21, wherein the pharmaceutically acceptable salt is selected from the group consisting of a tosylate, methanesulfonate, acetate, citrate, malonate, tartrate, succinate, benzoate, ascorbate, α-ketoglutarate, α-glycerophosphate, formate, fumarate, propionate, glycolate, lactate, pyruvate, oxalate, maleate, salicylate, sulfate, nitrate, bicarbonate, carbonate salts, hydrobromate, hydrochloride, di-hydrochloride, and phosphoric acid salt.

37. The pharmaceutical composition of claim 36, wherein the pharmaceutically acceptable salt is a hydrochloride salt.

## Patentansprüche

1. Verbindung der Formel (IX): 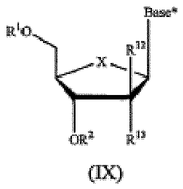 oder ein pharmazeutisch annehmbares Salz davon, in welcher:
R¹ und R² unabhängig voneinander H; Phosphat; geradkettiges, verzweigtes oder cyclisches Alkyl; Acyl; CO-Alkyl; CO-Aryl; CO-Alkoxyalkyl; CO-Aryloxyalkyl; CO-substituiertes Aryl; Sulfonatester; Benzyl, worin die Phenylgruppe gegebenenfalls mit einem oder mehreren Substituenten substituiert ist; Alkylsulfonyl; Arylsulfonyl; Arylalkylsulfonyl; ein Lipid; eine Aminosäure; ein Kohlenhydrat; ein Peptid oder ein Cholesterol sind;
X O ist;
Base* eine Purin- oder Pyrimidinbase ist;
R¹² C(Y³)₃ ist;
Y³ H ist; und
R¹³ Fluor ist.

2. Verbindung nach Anspruch 1, in welcher R¹ und R² H sind.

3. Verbindung nach Anspruch 1, in welcher:
R¹ H; Phosphat; geradkettiges, verzweigtes oder cyclisches Alkyl; Acyl; CO-Alkyl; CO-Aryl; CO-Alkoxyalkyl; CO-Aryloxyalkyl; CO-substituiertes Aryl; Sulfonatester; Benzyl, worin die Phenylgruppe gegebenenfalls mit einem oder mehreren Substituenten substituiert ist; Alkylsulfonyl; Arylsulfonyl; Arylalkylsulfonyl; ein Lipid; eine Aminosäure; ein Kohlenhydrat; ein Peptid oder ein Cholesterol ist; und
R² Phosphat; geradkettiges, verzweigtes oder cyclisches Alkyl; Acyl; CO-Alkyl; CO-Aryl; CO-Alkoxyalkyl; CO-Aryloxyalkyl; CO-substituiertes Aryl; Sulfonatester; Benzyl, worin die Phenylgruppe gegebenenfalls mit einem oder mehreren Substituenten substituiert ist; Alkylsulfonyl; Arylsulfonyl; Arylalkylsulfonyl; ein Lipid; eine Aminosäure; ein Kohlenhydrat; ein Peptid oder ein Cholesterol ist.

4. Verbindung nach Anspruch 1, in welcher:
R¹ H; Phosphat; geradkettiges, verzweigtes oder cyclisches Alkyl; Acyl; CO-Alkyl; CO-Aryl; CO-Alkoxyalkyl; CO-Aryloxyalkyl; CO-substituiertes Aryl; Sulfonatester; Benzyl, worin die Phenylgruppe gegebenenfalls mit einem oder mehreren Substituenten substituiert ist; Alkylsulfonyl; Arylsulfonyl; Arylalkylsulfonyl; ein Lipid; eine Aminosäure; ein Kohlenhydrat; ein Peptid oder ein Cholesterol ist;
R² Acyl ist; und
Base* Thymin, Uracil, Guanin, Cytosin, Adenin, Hypoxanthin, 5-Fluoruracil oder 5-Fluorcytosin ist.

5. Verbindung nach einem der Ansprüche 1-4, worin Base* Cytosin, Uracil, Guanin, Adenin oder Thymin ist.

6. Verbindung nach einem der Ansprüche 1-5 oder ein pharmazeutisch annehmbares Salz davon zur Verwendung in einem Verfahren für die Behandlung eines mit einem *Flaviviridae-*Virus infizierten Wirts.

7. Verbindung zur Verwendung nach Anspruch 6, wobei das Virus Hepatitis C ist.

8. Verbindung zur Verwendung nach Anspruch 6,
wobei das Verfahren eine Verabreichung der Verbindung oder eines pharmazeutisch annehmbaren Salzes davon in Kombination oder alternierend mit einem zweiten antiviralen Mittel umfasst; oder
wobei das Verfahren eine Verabreichung der Verbindung oder eines pharmazeutisch annehmbaren Salzes davon in Kombination oder alternierend mit einem zweiten oder mehreren antiviralen Mitteln umfasst.

9. Verbindung zur Verwendung nach Anspruch 8, wobei das zweite antivirale Mittel aus der Gruppe bestehend aus einem Interferon, einem Ribavirin, einem Interleukin, einem NS3-Proteaseinhibitor, einem Cysteinproteaseinhibitor, einem Phenanthrenchinon, einem Thiazolidin-Derivat, einem Thiazolidin, einem Benzanilid, einem Phenanthrenchinon, einem Helicaseinhibitor, einem Polymeraseinhibitor, einem Nukleotid-Analog, einem Gliotoxin, einem Cerulenin, einem Antisense-Phosphorothioat-oligodesoxynukleotid, einem Inhibitor der IRES-abhängigen Translation und einem Ribozym ausgewählt ist.

10. Verbindung zur Verwendung nach Anspruch 9, wobei das zweite antivirale Mittel ein Interferon ist.

11. Verbindung zur Verwendung nach Anspruch 10, wobei das zweite antivirale Mittel aus der Gruppe bestehend aus pegyliertem Interferon alpha 2a, Interferon alphacon-1, natürlichem Interferon, Albuferon, Interferon beta-1a, omega-Interferon, Interferon alpha, Interferon gamma, Interferon tau, Interferon delta und Interferon gamma-1b ausgewählt ist.

12. Verbindung zur Verwendung nach Anspruch 6, wobei die Verbindung oder das pharmazeutisch annehmbare Salz davon in Form einer Dosierungseinheit vorliegt.

13. Verbindung zur Verwendung nach Anspruch 12, wobei die Dosierungseinheit 50 bis 1000 mg oder 0,1 bis 50 mg der Verbindung enthält.

14. Verbindung zur Verwendung nach Anspruch 12, wobei die Dosierungseinheit eine Tablette oder Kapsel ist.

15. Verbindung zur Verwendung nach Anspruch 6, wobei der Wirt ein Mensch ist.

16. Verbindung zur Verwendung nach Anspruch 6, wobei die Verbindung oder das pharmazeutisch annehmbare Salz davon in im Wesentlichen reiner Form vorliegt.

17. Verbindung zur Verwendung nach Anspruch 6, wobei die Verbindung oder das pharmazeutisch annehmbare Salz davon zu mindestens 90 Gew.-% aus dem β-D-Isomer besteht.

18. Verbindung zur Verwendung nach Anspruch 6, wobei die Verbindung oder das pharmazeutisch annehmbare Salz davon zu mindestens 95 Gew.-% aus dem β-D-Isomer besteht.

19. Verbindung zur Verwendung nach Anspruch 6, wobei die Verbindung in Form eines pharmazeutisch annehmbaren Salzes, ausgewählt aus der Gruppe bestehend aus Tosylat-, Methansulfonat-, Acetat-, Citrat-, Malonat-, Tartrat-, Succinat-, Benzoat-, Ascorbat-, α-Ketoglutarat-, α-Glycerophosphat-, Formiat-, Fumarat-, Propionat-, Glycolat-, Lactat-, Pyruvat-, Oxalat-, Maleat-, Salicylat-, Sulfat-, Nitrat-, Bicarbonat-, Carbonat-Salzen, einem Hydrobromat-, Hydrochlorid-, Dihydrochlorid- und Phosphorsäuresalz, vorliegt.

20. Verbindung zur Verwendung nach Anspruch 19, wobei das pharmazeutisch annehmbare Salz ein Hydrochlorid-Salz ist.

21. Pharmazeutische Zusammensetzung, welche eine für eine Behandlung einer *Flaviviridae*-Infektion wirksame Menge einer Verbindung oder eines pharmazeutisch annehmbaren Salzes davon nach einem der Ansprüche 1 bis 5 in einem pharmazeutisch annehmbaren Träger umfasst.

22. Pharmazeutische Zusammensetzung nach Anspruch 21, wobei der Träger für eine orale Abgabe geeignet ist.

23. Pharmazeutische Zusammensetzung nach Anspruch 21, welche eine wirksame Menge der Verbindung oder eines pharmazeutisch annehmbaren Salzes davon, um einen mit dem West Nile-Virus, Gelbfieber, Dengue-Virus oder BVDV infizierten Wirt zu behandeln, umfasst.

24. Zusammensetzung nach Anspruch 21, wobei das *Flaviviridae*-Virus Hepatitis C ist.

25. Pharmazeutische Zusammensetzung nach Anspruch 21, wobei die Verbindung oder das pharmazeutisch annehmbare Salz davon in Form einer Dosierungseinheit vorliegt.

26. Zusammensetzung nach Anspruch 25, wobei die Dosierungseinheit 0,1 bis 50 mg oder 50 bis 1000 mg der Verbindung oder eines pharmazeutisch annehmbaren Salzes davon enthält.

27. Zusammensetzung nach Anspruch 25, wobei die Dosierungseinheit eine Tablette oder Kapsel ist.

28. Pharmazeutische Zusammensetzung nach Anspruch 21, welche des Weiteren ein zweites antivirales Mittel umfasst; oder
des Weiteren ein zweites oder mehrere antivirale Mittel umfasst.

29. Pharmazeutische Zusammensetzung nach Anspruch 28, wobei das zweite antivirale Mittel aus der Gruppe bestehend aus einem Interferon, einem Ribavirin, einem Interleukin, einem NS3-Proteaseinhibitor, einem Cysteinproteaseinhibitor, einem Phenanthrenchinon, einem Thiazolidin-Derivat, einem Thiazolidin, einem Benzanilid, einem Phenanthrenchinon, einem Helicaseinhibitor, einem Polymeraseinhibitor, einem Nukleotid-Analog, einem Gliotoxin, einem Cerulenin, einem Antisense-Phosphorothioat-oligodesoxynukleotid, einem Inhibitor der IRES-abhängigen Translation und einem Ribozym ausgewählt ist.

30. Pharmazeutische Zusammensetzung nach Anspruch 29, wobei das zweite antivirale Mittel ein Interferon ist.

31. Pharmazeutische Zusammensetzung nach Anspruch 30, wobei das zweite antivirale Mittel aus der Gruppe bestehend aus pegyliertem Interferon alpha 2a, Interferon alphacon-1, natürlichem Interferon, Albuferon, Interferon beta-1a, omega-Interferon, Interferon alpha, Interferon gamma, Interferon tau, Interferon delta und Interferon gamma-1b ausgewählt ist.

32. Pharmazeutische Zusammensetzung nach Anspruch 21, wobei die Verbindung oder das pharmazeutisch annehmbare Salz davon in im Wesentlichen reiner Form vorliegt.

33. Pharmazeutische Zusammensetzung nach Anspruch 21, wobei die Verbindung oder das pharmazeutisch annehmbare Salz davon zu mindestens 90 Gew.-% aus dem β-D-Isomer besteht.

34. Pharmazeutische Zusammensetzung nach Anspruch 21, wobei die Verbindung oder das pharmazeutisch annehmbare Salz davon zu mindestens 95 Gew.-% aus dem β-D-Isomer besteht.

35. Pharmazeutische Zusammensetzung nach Anspruch 21, welche des Weiteren einen pharmazeutisch annehmbaren Träger, der für eine orale, parenterale, durch Inhalation erfolgende oder intravenöse Abgabe geeignet ist, umfasst.

36. Pharmazeutische Zusammensetzung nach Anspruch 21, wobei das pharmazeutisch annehmbare Salz aus der Gruppe bestehend aus Tosylat-, Methansulfonat-, Acetat-, Citrat-, Malonat-, Tartrat-, Succinat-, Benzoat-, Ascorbat-, α-Ketoglutarat-, α-Glycerophosphat-, Formiat-, Fumarat-, Propionat-, Glycolat-, Lactat-, Pyruvat-, Oxalat-, Maleat-, Salicylat-, Sulfat-, Nitrat-, Bicarbonat-, Carbonat-Salzen, einem Hydrobromat-, Hydrochlorid-, Dihydrochlorid- und Phosphorsäuresalz ausgewählt ist.

37. Pharmazeutische Zusammensetzung nach Anspruch 36, wobei das pharmazeutisch annehmbare Salz ein Hydrochlorid-Salz ist.

## Revendications

1. Composé de formule (IX) 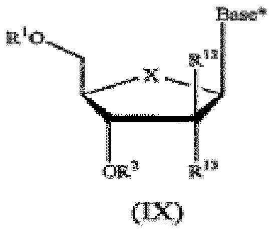 ou sel pharmaceutiquement acceptable de celui-ci, dans lequel :
R¹ et R² sont indépendamment H ; phosphate ; alkyle à chaîne droite, ramifié ou cyclique ; acyle ; CO-alkyle ; CO-aryle ; CO-alcoxyalkyle ; CO-aryloxyalkyle ; CO-aryle substitué ; ester de sulfonate ; benzyle, dans lequel le groupe phényle est éventuellement substitué avec un ou plusieurs substituants ; alkylsulfonyle ; arylsulfonyle ; aralkylsulfonyle ; un lipide ; un acide aminé ; un carbohydrate ; un peptide ou un cholestérol ;
X est O ;
Base* est une base de purine ou pyrimidine ;
R¹² est C(Y³)₃ ;
Y³ est H ; et
R¹³ est fluoro.

2. Composé selon la revendication 1, dans lequel R¹ et R² sont H.

3. Composé selon la revendication 1, dans lequel :
R¹ est H ; phosphate ; alkyle à chaîne droite, ramifié ou cyclique ; acyle ; CO-alkyle ; CO-aryle ; CO-alcoxyalkyle ; CO-aryloxyalkyle ; CO-aryle substitué ; ester de sulfonate ; benzyle, dans lequel le groupe phényle est éventuellement substitué avec un ou plusieurs substituants ; alkylsulfonyle ; arylsulfonyle ; aralkylsulfonyle ; un lipide ; un acide aminé ; un carbohydrate ; un peptide ou un cholestérol ; et
R² est phosphate ; alkyle à chaîne droite, ramifié ou cyclique ; acyle ; CO-alkyle ; CO-aryle ; CO-alcoxyalkyle ; CO-aryloxyalkyle ; CO-aryle substitué ; ester de sulfonate ; benzyle, dans lequel le groupe phényle est éventuellement substitué avec un ou plusieurs substituants ; alkylsulfonyle ; arylsulfonyle ; aralkylsulfonyle ; un lipide ; un acide aminé ; un carbohydrate ; un peptide ou un cholestérol.

4. Composé selon la revendication 1, dans lequel :
R¹ est H ; phosphate ; alkyle à chaîne droite, ramifié ou cyclique ; acyle ; CO-alkyle ; CO-aryle ; CO-alcoxyalkyle ; CO-aryloxyalkyle ; CO-aryle substitué ; ester de sulfonate ; benzyle, dans lequel le groupe phényle est éventuellement substitué avec un ou plusieurs substituants ; alkylsulfonyle ; arylsulfonyle ; aralkylsulfonyle ; un lipide ; un acide aminé ; un carbohydrate ; un peptide ou un cholestérol ;
R² est acyle ; et
Base* est thymine, uracile, guanine, cytosine, adénine, hypoxanthine, 5-fluorouracile ou 5-fluorocytosine.

5. Composé selon l'une quelconque des revendications 1 à 4, dans lequel Base* est cytosine, uracile, guanine, adénine ou thymine.

6. Composé selon l'une quelconque des revendications 1 à 5, ou sel pharmaceutiquement acceptable de celui-ci, pour utilisation dans une méthode pour le traitement d'un hôte infecté par un virus *Flaviviridae.*

7. Composé pour utilisation selon la revendication 6, dans lequel the virus est l'hépatite C.

8. Composé pour utilisation selon la revendication 6,
dans lequel la méthode comprend l'administration du composé ou d'un sel pharmaceutiquement acceptable de celui-ci en combinaison ou en alternance avec un second agent antiviral ; ou
dans lequel la méthode comprend l'administration du composé ou d'un sel pharmaceutiquement acceptable de celui-ci en combinaison ou en alternance avec un second agent antiviral ou plus.

9. Composé pour utilisation selon la revendication 8, dans lequel le second agent antiviral est choisi dans le groupe constitué par un interféron, une ribavirine, une interleucine, un inhibiteur de la protéase NS3, un inhibiteur de la cystéine protéase, une phénan-thrénéquinone, un dérivé de thiazolidine, une thiazolidine, un benzanilide, un inhibiteur d'hélicase, un inhibiteur de polymérase, un analogue de nucléotide, une gliotoxine, une cérulénine, une oligodéoxynucléotide de phosphorothioate antisens, un inhibiteur de la translation dépendante de l'IRES et un ribozyme.

10. Composé pour utilisation selon la revendication 9, dans lequel le second agent antiviral est un interféron.

11. Composé pour utilisation selon la revendication 10, dans lequel le second agent antiviral est choisi dans le groupe constitué par un interféron alpha pégylé 2a, un interféron alphacon-1, un interféron naturel, un albuféron, un interféron bêta-1a, un oméga interféron, un interféron alpha, un interféron gamma, un interféron tau, un interféron delta et un interféron gamma-1b.

12. Composé pour utilisation selon la revendication 6, dans lequel le composé ou sel pharmaceutiquement acceptable de celui-ci est sous la forme d'une unité de dosage.

13. Composé pour utilisation selon la revendication 12, dans lequel l'unité de dosage contient 50 à 1000 mg ou 0,1 à 50 mg du composé.

14. Composé pour utilisation selon la revendication 12, dans lequel l'unité de dosage est un comprimé ou une capsule.

15. Composé pour utilisation selon la revendication 6, dans lequel l'hôte est un humain.

16. Composé pour utilisation selon la revendication 6, dans lequel le composé ou sel pharmaceutiquement acceptable de celui-ci est sous une forme sensiblement pure.

17. Composé pour utilisation selon la revendication 6, dans lequel le composé ou sel pharmaceutiquement acceptable de celui-ci représente au moins 90 % en poids du 5-D-isomère.

18. Composé pour utilisation selon la revendication 6, dans lequel le composé ou sel pharmaceutiquement acceptable de celui-ci représente au moins 95 % en poids du 5-D-isomère.

19. Composé pour utilisation selon la revendication 6, dans lequel le composé est sous la forme d'un sel pharmaceutiquement acceptable choisi dans le groupe constitué par un tosylate, un méthanesulfonate, un acétate, un citrate, un malonate, un tartrate, un succinate, un benzoate, un ascorbate, un α-cétoglutarate, un α-glycérophosphate, un formate, un fumarate, un propionate, un glycolate, un lactate, un pyruvate, un oxalate, un maléate, un salicylate, un sulfate, un nitrate, un bicarbonate, des sels de carbonates, un hydrobromate, un hydrochlorure, un dihydrochlorure et un sel d'acide phosphorique..

20. Composé pour utilisation selon la revendication 19, dans lequel le sel pharmaceutiquement acceptable est un sel d'hydrochlorure.

21. Composition pharmaceutique comprenant une quantité efficace pour traiter une infection par *Flaviviridae* d'un composé, ou d'un sel pharmaceutiquement acceptable de celui-ci, selon l'une quelconque des revendications 1 à 5 dans un support pharmaceutiquement acceptable.

22. Composition pharmaceutique selon la revendication 21, dans laquelle le support est adapté à une administration orale.

23. Composition pharmaceutique selon la revendication 21 comprenant une quantité efficace du composé ou sel pharmaceutiquement acceptable de celui-ci pour traiter un hôte infecté par le virus du Nil occidental, la fièvre jaune, le virus de la dengue ou le VDVB.

24. Composition selon la revendication 21, dans laquelle le virus *Flaviviridae* est l'hépatite C.

25. Composition pharmaceutique selon la revendication 21, dans laquelle le composé ou sel pharmaceutiquement acceptable de celui-ci est sous la forme d'une unité de dosage.

26. Composition selon la revendication 25, dans laquelle l'unité de dosage contient 0,1 à 50 mg ou 50 à 1000 mg du composé ou sel pharmaceutiquement acceptable de celui-ci.

27. Composition selon la revendication 25, dans laquelle ladite unité de dosage est un comprimé ou une capsule.

28. Composition pharmaceutique selon la revendication 21,
comprenant en outre un second agent antiviral ; ou
comprenant en outre un second agent antiviral ou plus.

29. Composition pharmaceutique selon la revendication 28, dans laquelle le second agent antiviral est choisi dans le groupe constitué par un interféron, une ribavirine, une interleucine, un inhibiteur de la NS3 protéase, un inhibiteur de la cystéine protéase, une phénan-thrénéquinone, un dérivé de thiazolidine, une thiazolidine, un benzanilide, une phénan-thrénéquinone, un inhibiteur d'hélicase, un inhibiteur de polymérase, un analogue de nucléotide, une gliotoxine, une cérulénine, une oligodéoxynucléotide de phosphorothioate antisens, un inhibiteur de la translation dépendant de l'IRES et un ribozyme.

30. Composition pharmaceutique selon la revendication 29, dans laquelle le second agent antiviral est un interféron.

31. Composition pharmaceutique selon la revendication 30, dans laquelle le second agent antiviral est choisi dans le groupe constitué par un interféron alpha pégylé 2a, un interféron alphacon-1, un interféron naturel, un albuféron, un interféron bêta-1a, un oméga interféron, un interféron alpha, un interféron gamma, un interféron tau, un interféron delta et un interféron gamma-1b.

32. Composition pharmaceutique selon la revendication 21, dans laquelle le composé ou sel pharmaceutiquement acceptable de celui-ci est sous une forme sensiblement pure.

33. Composition pharmaceutique selon la revendication 21, dans laquelle le composé ou sel pharmaceutiquement acceptable de celui-ci représente au moins 90 % en poids du 5-D-isomère.

34. Composition pharmaceutique selon la revendication 21, dans laquelle le composé ou sel pharmaceutiquement acceptable de celui-ci représente au moins 95 % en poids du β-D-isomère.

35. Composition pharmaceutique selon la revendication 21, comprenant en outre un support pharmaceutiquement acceptable adapté à une administration orale, parentérale, par inhalation ou intraveineuse.

36. Composition pharmaceutique selon la revendication 21, dans laquelle le sel pharmaceutiquement acceptable est choisi dans le groupe constitué par un tosylate, un méthanesulfonate, un acétate, un citrate, un malonate, un tartrate, un succinate, un benzoate, un ascorbate, un α-cétoglutarate, un α-glycérophosphate, un formate, un fumarate, un propionate, un glycolate, un lactate, un pyruvate, un oxalate, un maléate, un salicylate, un sulfate, un nitrate, un bicarbonate, des sels de carbonates, un hydrobromate, un hydrochlorure, un dihydrochlorure et un sel d'acide phosphorique.

37. Composition pharmaceutique selon la revendication 36, dans laquelle le sel pharmaceutiquement acceptable est un sel d'hydrochlorure.
